# EUROPEAN PATENT SPECIFICATION

(11) **EP 1 773 383 B1**
(45) Date of publication and mention of the grant of the patent: **12.09.2012**
(21) Application number: 05780086.4
(22) Date of filing: 27.05.2005
(51) Int. Cl.: A61K 39/00, C07K 14/705, C07K 16/18, C07K 16/28, C07K 16/32

(54) **ANTIBODIES AS T CELL RECEPTOR MIMICS, METHODS OF PRODUCTION AND USES THEREOF**
ANTIKÖRPER ALS T-ZELLEN-REZEPTORMIMETIKA, HERSTELLUNGSVERFAHREN UND VERWENDUNG DAFÜR
ANTICORPS MIMETIQUES DE RECEPTEURS DE CELLULES T ET LEURS PROCEDES DE PRODUCTION ET D'UTILISATION

(30) Priority: 27.05.2004 US 574857 P; 28.12.2004 US 640020 P; 24.01.2005 US 646338 P; 20.04.2005 US 673296 P
(43) Date of publication of application: 18.04.2007
(73) Proprietor: Weidanz, Jon A., Amarillo TX 79124 (US); Wittman, Vaughan P., Amarillo TX 79124 (US)
(72) Inventor: Weidanz, Jon A., Amarillo TX 79124 (US); Wittman, Vaughan P., Amarillo TX 79124 (US)
(74) Representative: Richards, John
(86) International application number: PCT/US2005/018789
(87) International publication number: WO 2005/116072

(56) References cited:
- WO-A-02/14870
- WO-A-03/070752
- WO-A1-97/02342
- REAY P A ET AL: "Determination of the relationship between T cell responsiveness and the number of MHC-peptide complexes using specific monoclonal antibodies" JOURNAL OF IMMUNOLOGY, AMERICAN ASSOCIATION OF IMMUNOLOGISTS, US, vol. 164, no. 11, 1 June 2000 (2000-06-01), pages 5626-5634, XP002366691 ISSN: 0022-1767
- AHARONI R ET AL: "Immunomodulation of experimental allergic encephalomyelitis by antibodies to the antigen-Ia complex." NATURE 9 MAY 1991, vol. 351, no. 6322, 9 May 1991 (1991-05-09), pages 147-150, XP002514757 ISSN: 0028-0836
- PORGADOR A ET AL: "LOCALIZATION, QUANTITATION, AND IN SITU DETECTION OF SPECIFIC PEPTIDE-MHC CLASS I COMPLEXES USING A MONOCLONAL ANNTIBODY" IMMUNITY, CELL PRESS, US, vol. 6, no. 6, 1 June 1997 (1997-06-01), pages 715-726, XP000991374 ISSN: 1074-7613
- REITER Y ET AL: "peptide-specific killing of antigen-presenting cells by a recombinant antibody-toxin fusion protein targeted to major histocompatibility complex/peptide class I complexes with T cell receptor-like specificity" PROCEEDINGS OF THE NATIONAL ACADEMY OF SCIENCES OF USA, NATIONAL ACADEMY OF SCIENCE, WASHINGTON, DC.; US, vol. 94, 1 April 1997 (1997-04-01), pages 4631-4636, XP002967291 ISSN: 0027-8424
- NOY R ET AL: "T-CELL RECEPTOR-LIKE ANTIBODIES: NOVEL REAGENTS FOR CLINICAL CANCER IMMUNOLOGY AND IMMUNOTHERAPY" EXPERT REVIEW OF ANTICANCER THERAPY, FUTURE DRUGS, LONDON, GB, vol. 5, no. 3, 1 June 2005 (2005-06-01), pages 523-536, XP009067037 ISSN: 1473-7140
- WITTMAN VAUGHAN P ET AL: "Antibody targeting to a class I MHC-peptide epitope promotes tumor cell death" JOURNAL OF IMMUNOLOGY, vol. 177, no. 6, September 2006 (2006-09), pages 4187-4195, XP002514758 ISSN: 0022-1767
- POLAKOVA ET AL.: 'Antibodies Directed Against the MHC-I Molecule H-2Dd Complexed with an Antigenic Peptide: Similarities to a T Cell Receptor with the Same Specificity' THE JOURNAL OF IMMUNOLOGY vol. 165, 2000, pages 5703 - 5712, XP002986050
- CHUNG ET AL.: 'Competitive Inhibition In Vivo and Skewing of the T Cell Repertoire of Antigen-Specific CTL Priming by an Anti-Peptide-MHC Monoclonal Antibody' THE JOURNAL OF IMMUNOLOGY vol. 167, 2001, pages 699 - 707, XP002903695
- HARLOW AND LANE: 'Antibodies A LABORATORY MANUAL', 1988, COLD SPRING HARBOR LABORATORY page 173, XP008070536
- DATABASE GENESEQ [Online] 06 August 1998 XP003002328 Database accession no. (AAW70057)
- DATABASE GENESEQ [Online] 09 December 1993 XP003002329 Database accession no. (AAR44277)

## Description

### CROSS-REFERENCE TO RELATED APPLICATIONS

This application claims the benefit under 35 U.S.C. 119(e) of provisional applications US Serial No. 60/374,857, filed May 27, 2004; US Serial No. 60/640,020, filed December 28, 2004; US Serial No. 60/646,338, filed January 24, 2005; and US Serial No. 60/673,296, filed April 20, 2005.

### STATEMENT REGARDING FEDERALLY SPONSORED RESEARCH OR DEVELOPMENT

The government owns certain rights in the present invention pursuant to a grant from the Advanced Technology Program of the National Institute of Standards and Technology (Grant #70NANB4H3048).

### BACKGROUND OF THE INVENTION

### 1. Field of the Invention

The present invention relates generally to a methodology of producing antibodies that recognize peptides associated with a tumorigenic or disease state, wherein the peptides are displayed in the context of HLA molecules. These antibodies will mimic the specificity of a T cell receptor (TCR) such that the molecules may be used as therapeutic, diagnostic and research reagents.

### 2. Description of the Background Art

Class I major histocompatibility complex (MHC) molecules, designated HLA class I in humans, bind and display peptide antigen ligands upon the cell surface. The peptide antigen ligands presented by the class I MHC molecule are derived from either normal endogenous proteins ("self") or foreign proteins ("nonself") introduced into the cell. Nonself proteins may be products of malignant transformation or intracellular pathogens such as viruses. In this manner, class I MHC molecules convey information regarding the internal milieu of a cell to immune effector cells including but not limited to, CD8⁺ cytotoxic T lymphocytes (CTLs), which are activated upon interaction with "nonself" peptides, thereby lysing or killing the cell presenting such "nonself" peptides.

Class II MHC molecules, designated HLA class II in humans, also bind and display peptide antigen ligands upon the cell surface. Unlike class I MHC molecules which are expressed on virtually all nucleated cells, class II MHC molecules are normally confined to specialized cells, such as B lymphocytes, macrophages, dendritic cells, and other antigen presenting cells which take up foreign antigens from the extracellular fluid via an endocytic pathway. The peptides they bind and present are derived from extracellular foreign antigens, such as products of bacteria that multiply outside of cells, wherein such products include protein toxins secreted by the bacteria that often have deleterious and even lethal effects on the host (e.g., human). In this manner, class II molecules convey information regarding the fitness of the extracellular space in the vicinity of the cell displaying the class II molecule to immune effector cells, including but not limited to, CD4⁺ helper T cells, thereby helping to eliminate such pathogens. The extermination of such pathogens is accomplished by both helping B cells make antibodies against microbes, as well as toxins produced by such microbes, and by activating macrophages to destroy ingested microbes.

Class I and class II HLA molecules exhibit extensive polymorphism generated by systematic recombinatorial and point mutation events during cell differentiation and maturation resulting from allelic diversity of the parents; as such, hundreds of different HLA types exist throughout the world's population, resulting in a large immunological diversity. Such extensive HLA diversity throughout the population is the root cause of tissue or organ transplant rejection between individuals as well as of differing individual susceptibility and/or resistance to infectious diseases. HLA molecules also contribute significantly to autoimmunity and cancer. Because HLA molecules mediate most, if not all, adaptive immune responses, large quantities of pure isolated HLA proteins are required in order to effectively study transplantation, autoimmune disorders, and for vaccine development.

Class I HLA molecules alert the immune response to disorders within host cells. Peptides, which are derived from viral- and tumor-specific proteins within the cell, are loaded into the class I molecule's antigen binding groove in the endoplasmic reticulum of the cell and subsequently carried to the cell surface. Once the class I HLA molecule and its loaded peptide ligand are on the cell surface, the class I molecule and its peptide ligand are accessible to cytotoxic T lymphocytes (CTL). CTLs survey the peptides presented by the class I molecule and destroy those cells harboring ligands derived from infectious or neoplastic agents within that cell.

While specific CTL targets have been identified, little is known about the breadth and nature of ligands presented on the surface of a diseased cell. From a basic scientific perspective, many outstanding questions remain in the art regarding peptide presentation. For instance, it has been demonstrated that a virus can preferentially block expression of HLA class I molecules from a given locus while leaving expression at other loci intact. Similarly, there are numerous reports of cancerous cells that downregulate the expression of class I HLA at particular loci. However, there is no data describing how (or if) the classical HLA class I loci differ in the peptides they bind. It is therefore unclear how class I molecules from the different loci vary in their interaction with viral- and tumor-derived ligands and the number of peptides each will present.

Discerning virus- and tumor-specific ligands for CTL recognition is an important component of vaccine design. Ligands unique to tumorigenic or infected cells can be tested and incorporated into vaccines designed to evoke a protective CTL response. Several methodologies are currently employed to identify potentially protective peptide ligands. One approach uses T cell lines or clones to screen for biologically active ligands among chromatographic fractions of eluted peptides (Cox et al., 1994). This approach has been employed to identify peptide ligands specific to cancerous cells. A second technique utilizes predictive algorithms to identify peptides capable of binding to a particular class I molecule based upon previously determined motif and/or individual ligand sequences (De Groot et al., 2001; however, there have been reports describing discrepancies between these algorithms and empirical data). Peptides having high predicted probability of binding from a pathogen of interest can then be synthesized and tested for T cell reactivity in various assays, such as but not limited to, precursor, tetramer and ELISpot assays.

With the discovery of mAb technology, it was believed that "magic bullets" could be developed which specifically target malignant cells for destruction. Current strategies for the development of tumor specific antibodies rely on creating monoclonal antibodies (mAbs) to tumor-associated antigens (TAAs) displayed as intact proteins on the surface of malignant cells. Though targeting surface tumor antigens has resulted in the development of several successful anti-tumor antibodies (Herceptin and Rituxan), a significant number of patients (up to 70%) are refractory to treatment with these antibody molecules. This has raised several questions regarding the rationale for targeting whole molecules displayed on the tumor cell surface for developing cancer therapeutic reagents. First, antibody-based therapies directed at surface antigens are often associated with lower than expected killing efficiency of tumor cells. Free tumor antigens shed from the surface of the tumor occupy the binding sites of the anti-tumor specific antibody reducing the number of active molecules resulting in decreased tumor cell death. Second, current mAb molecules do not recognize many potential cancer antigens because these antigens are not expressed as an intact protein on the surface of tumor cells. The tumor suppressor protein p53 is a good example. p53 and similar intracellular tumor associated proteins are normally processed within the cell into peptides which are then presented in the context of either HLA class I or class II molecules on the surface of the tumor cell. Native antibodies are not generated against peptide-HLA complexes. Third, many of the antigens recognized by antibodies are heterogenic by nature, which limits the effectiveness of an antibody to a single tumor histology. For these reasons it is apparent that antibodies generated against surface expressed tumor antigens may not be optimal therapeutic targets for cancer immunotherapy.

In contrast, many T cell epitopes (specific peptide-HLA complexes) are common to a broad range of tumors which have originated from several distinct tissues. The primary goal of epitope discovery has been to identify peptide (tumor antigens) for use in the construction of vaccines that activate a clinically relevant cellular immune response against the tumor cells. The goal of vaccination in cancer immunotherapy is to elicit a cytotoxic T lymphocyte (CTL) response and activate T helper responses to eliminate the tumor. Although many of the epitopes discovered by current methods are immunogenic, shown by studies that generate peptide-specific CTL *in vitro* and *in vivo,* the application of vaccination protocols to cancer treatment has not been highly successful. This is especially true for cancer vaccines that target self-antigens ("normal" proteins that are overexpressed in the malignant cells). Although this class of antigens may not be ideal for vaccine formulation, due to an individual "tolerance" of self antigens, they still represent good targets for eliciting antibodies *ex vivo.*

The value of monoclonal antibodies which recognize peptide-MHC complexes has been recognized by others (see for example Reiter, US Publication No. US 2004/0191260 A1, filed March 26, 2003; Andersen et al., US Publication No. US 2002/0150914 A1, filed September 19, 2001; Hoogenboom et al., US Publication No. US 2003/0223994 A1, filed February 20, 2003; and Reiter et al., PCT Publication No. WO 03/068201 A2, filed February 11, 2003). However, these processes employ the use of phage display libraries that do not produce a whole, ready-to-use antibody product. These prior art methods also have not demonstrated production of antibodies capable of staining tumor cells in a robust manner, implying that they are of low affinity or specificity. The immunogen employed in the prior art methods uses MHC which has been "enriched" for one particular peptide, and therefore such immunogen contains a pool of peptide-MHC complexes and is not loaded solely with the peptide of interest. In addition, there has not been a concerted effort in these prior art methods to maintain the structure of the three dimensional epitope formed by the peptide/HLA complex, which is essential for generation of the appropriate antibody response. For these reasons, immunization protocols presented in these prior art references had to be carried out over long periods of time (i.e., approximately 5 months or longer).

Therefore, there exists a need in the art for diagnostic and therapeutic antibodies with novel recognition specificity for peptide-HLA domain in complexes present on the surface of tumor cells. The presently claims and disclosed invention provides innovative processes for creating antibody molecules endowed with unique antigen recognition specificities for peptide0HLA complexes, and the present invention recognizes that these peptide-HLA molecules are unique sources of tumor specific antigens available as therapeutic targets. In addition, the development of this technology will provide new tools to detect, visualize, quantify, and study antigen (peptide0HLA) presentation in tumors. Antibodies with T cell receptor-like specificity of the present invention enable the measurement of antigen presentation on tumors by direct visualization. Previous studios attempting to visualize peptide-HLA complexes using a soluble TCR found that the poor affinity of the TCR made it difficult to consistently detect low levels of target on tumor cells (Weidanz, 2000). Therefore, in addition to being used as targeting agents, TCRm of the present inventions serve as valuable tools to obtain information regarding the presence, expression pattern, and distribution of the target peptide-HLA complex antigens on the tumor surface and in tumor metastasis.

### SUMMARY OF THE INVENTION

As set out in the claims, the present invention relates to producing a T-cell receptor mimic which will mimic the specificity of a T-cell receptor (TCR) such that the molecules may be used as therapeutic, diagnostic and research reagents. In one embodiment, the T cell receptor mimics will have higher binding affinity than a T cell receptor. In a preferred embodiment, the T cell receptor mimic has a binding affinity of about 10 nanomolar or greater.

It is an object of the present invention to provide a method of producing a T-cell receptor mimic. The method of the presently disclosed and claimed invention includes identifying a peptide of interest, wherein the peptide of interest is capable of being presented by an MHC molecule. Then, an immunogen comprising at least one peptide/MHC complex is formed, wherein the peptide of the peptide-MHC complex is the peptide of interest. An effective amount of the immunogen is then administered to a host for eliciting an immune response, and the immunogen retains a three-dimensional form thereof for a period of time sufficient to elicit an immune response against the three-dimensional presentation of the peptide in the binding groove of the MHC molecule. Serum collected from the host is assayed to determine if desired antibodies that recognize a three-dimensional presentation of the peptide in the binding groove of the MHC molecule are being produced. The desired antibodies can differentiate the peptide/MHC complex from the MHC molecule alone, the peptide alone, and a complex of MHC and irrelevant peptide. Finally, the desired antibodies are isolated.

The peptide of interest may be associated with at least one of a tumorigenic state, an infections state and a disease state, or the peptide of interest may be specific to a particular organ or tissue. The presentation of the peptide in context of an MHC molecule may be novel to cancer cells, or it may be greatly increased in cancer cells when compared to normal cells.

In one embodiment, the step of forming an immunogen in the method of the presently disclosed and claimed invention may include recombinantly expressing the peptide class 1 MHC complex in the form of a single chain trimer. In another embodiment, the step of forming the immunogen of the present invention includes recombinantly expressing the class 1 MHC heavy chain and the class I MHC light chain separately in *E. coli,* and then refolding the MHC heavy and light chains with peptide in vitro.

In addition, the immunogen may be formed by multimerizing two or more peptide/MHC complexes, such as a dimer, a trimer, a tetramer, a pentamer, or a hexamer. The two or more peptide/MHC complexes may be covalently attached, and they may be modified to enable covalent attachment of the peptide/MHC complex to one another. Optionally, the two or more peptide/MHC complexes may be non-covalently attached. The two or more peptide/MHC complexes may be attached to a substrate When the peptide/MHC complexes are attached to a substrate, the desired antibodies should not recognize the substrate utilized in multimerization of the peptide/MHC complexes. A tail may be attached to the two or more peptide/MHC complexes may be attached to a substrate. When the peptide/MHC complexes are attached to a substrate, the desired antibodies should not recognize the substrate utilized in multimerization of the peptide/MHC complexes. A tail may be attached to the two or more peptide-MHC complexes to aid in multimerization, wherein the tail may be selected from the group including but not limited to, a biotinylation signal peptide tail, an immunoglobulin heavy chain tail, and TNF tail, an IgM tail, a Fos/Jun tail, and combinations thereof. In a further alternative, the peptide/MHC complexes may be multimerized through liposome encapsulation through the use of an artificial antigen presenting cell, or through the use of polymerized streptavidin.

In one embodiment, the immunogen may be further modified to aid in stabilization thereof. The modification may be selected from the group consisting of modifying an anchor in the peptide/MHC complex, modifying amino acids in the peptide/MHC complex, PEGalation, chemical cross-linking, change in pH or salt, addition of at least one chaperone protein, addition of at least one adjuvant, and combinations thereof.

The host immunized for eliciting an immune response in the presently disclosed and claimed method may be a rabbit, a rat, or a mouse, such as but not limited to, a Balb/c mouse or a transgenic mouse. The transgenic mouse may be transgenic for the MHC molecule of the immunogen, or the transgenic mouse may be capable of producing human antibodies.

The assaying step of the presently disclosed and claimed invention may further include preabsorbing the serum to remove antibodies than are not peptide specific.

The step of isolating the desired antibodies of the presently disclosed and claimed invention may further include a method for isolating at least one of B cells expressing surface immunoglobulin, B memory cells, hybridoma cells and plasma cells producing the desired antibodies. The steps of isolating the B memory cells may include sorting the B memory cells using at least one of FACS sorting, bead coated with peptide/MHC complex, magnetic beads, and intracellular staining. The method may further include the step of differentiating and expanding the B memory cells into plasma cells,

The method of the presently disclosed and claimed invention may further include the step of assaying the isolated desired antibodies to confirm the specificity and to determined if the isolated desired antibodies cross-react with other MHC molecules.

T cell receptor mimics of the present invention may include an antibody or antibody fragment reactive against a specific peptide class 1 MHC complex, wherein the antibody or antibody fragment can differentiate the specific peptide class 1 MHC complex from the MHC molecule along, the peptide alone, and a complex of class 1 MHC and an irrelevant peptide. The T cell receptor mimic is produced by immunizing a host with an effective amount of an immunogen comprising a multimer of two or more specific peptide class 1 MHC complexes. The immunogen may be in the form of a tetramer. The peptide of the specific peptide class 1 MHC complex may be associated with at least one or a tumorigenic state, an infections state and a disease state, or the peptide of the specific peptide/MHC complex may be specific to a particular organ or tissue. Alternatively, the presentation of the peptide of the specific peptide class 1 MHC complex in the context of an MHC molecule may be novel to cancer cells, or may be greatly increased in cancer cells when compared to normal cells. The peptide of the specific peptide/MHC complex may comprise SEQ ID NOS:1, 2 or 3.

In one embodiment, the T cell receptor mimic may have at least one functional moiety, such as a detectable moiety or a therapeutic moiety, bound thereto. The detectable moiety may be selected from the group consisting of a fluorophore, an enzyme, a radioisotope and combinations thereof, while the therapeutic moiety may be selected from the group consisting of cytotoxic moiety, a toxic moiety, cytokine moiety, a bi-specific moiety, and combinations thereof.

The present invention, while achieving the before-state objections, also permits provision of a hybridoma cell or a B cell producing a T cell receptor mimic comprising an antibody or antibody fragment reactive against a specific peptide class 1 MHC complex, wherein the antibody or antibody fragment can differentiate the specific peptide/MHC complex from the MHC molecule alone, the peptide alone, and a complex of MHC and an irrelevant peptide. The peptide of the specific peptide/MHC complex may be associated with at least one of a tumorigenic state, an infectious state and a disease state, or the peptide of the specific peptide/MHC complex may be specific to a particular organ or tissue. Alternatively, the presentation of the peptide of the specific peptide/MHC complex in the context of an MHC molecule may be novel to cancer cells, or may be greatly increased in cancer cells when compared to normal cells. The peptide of the specific peptide/MHC complex may comprise SEQ ID NOS: 1, 2 or 3.

The present invention further enables production of an isolated nucleic acid segment encoding a T cell receptor mimic comprising an antibody or antibody fragment reactive against a specific peptide/MHC complex, wherein the antibody or antibody fragment can differentiate the specific peptide/MHC complex from the MHC molecule alone, the peptide alone, and a complex of MHC and an irrelevant peptide. The peptide of the specific peptide/MHC complex may be associated with at least one of a tumorigenic state, an infectious state and a disease state, or the peptide of the specific peptide/MHC complex may be specific to a particular organ or tissue. Alternatively, the presentation of the peptide of the specific peptide/MHC complex in the context of an MHC molecule may be novel to cancer cells, or may be increased in cancer cells when compared to normal cells. The peptide of the specific peptide/MHC complex may comprise SEQ ID NOS:1,2 or 3.

The present invention also enables production of an immunogen used in production of a T cell receptor mimic. The immunogen includes a multimer of two or more identical peptide/MHC complexes, such as a tetramer, wherein the peptide/MHC complexes are capable of retaining their 3-dimensional form for a period of time sufficient to elicit an immune response in a host such that antibodies that recognize a three-dimensional presentation of the peptide in the binding groove of the MHC molecule are produced. The antibodies so produced are capable of differentiating the peptide/MHC complex from the MHC molecule alone, the peptide alone, and a complex of MHC and irrelevant peptide. The peptide of the specific peptide/MHC complex may be associated with at least one of a tumorigenic state, an infectious state and a disease state, or the peptide of the specific peptide/MHC complex may be specific to a particular organ or tissue. Alternatively, the presentation of the peptide of the specific peptide/MHC complex in the context of an MHC molecule may be novel to cancer cells, or may be greatly increased in cancer cells when compared to normal cells. The peptide of the specific peptide/MHC complex may comprise SEQ ID NOS:1, 2 or 3.

### BRIEF DESCRIPTION OF THE DRAWINGS

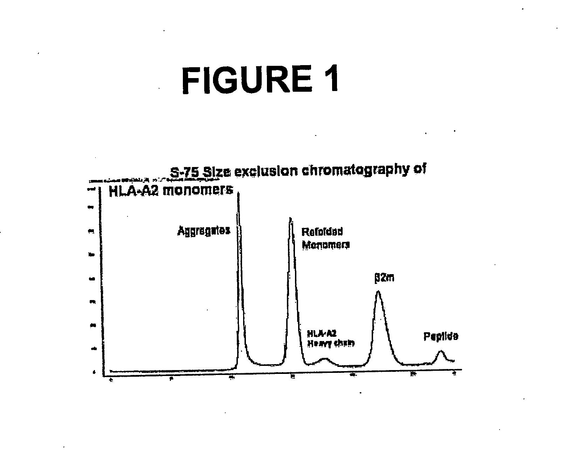

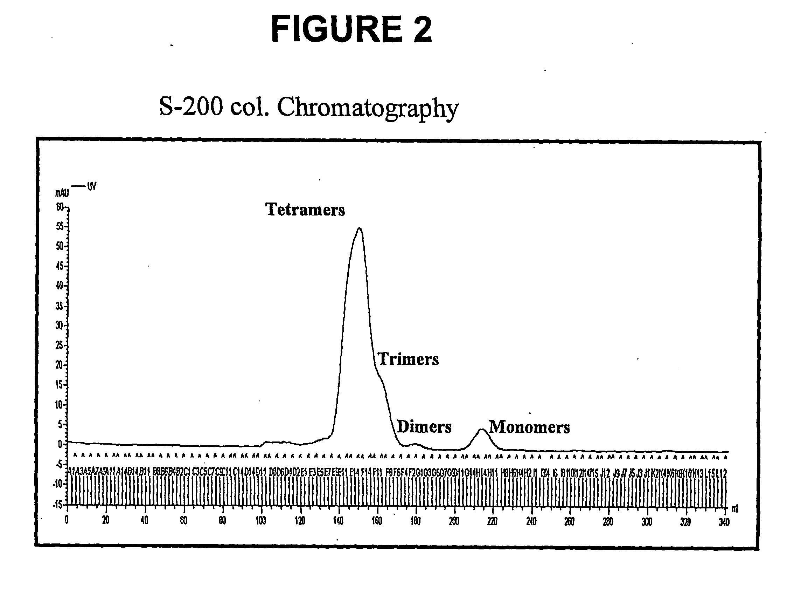

Figure 1 illustrates size exclusion chromatography on Sephadex S-75 column of mixture of refolded heavy and light (β2m) chains of HLA-A2 with synthetic peptide (LLGRNSFEV; SEQ ID NO:1). Peptide-HLA-A2 folded monomers were prepared and purified using S-75 size exclusion chromatography. Monomers consisting of peptide-HLA-A2 were prepared by mixing heavy chain (1 µM) together with beta-2 microglobulin (2µM) and 10 mg of the desired peptide in buffer (1 L) optimized to facilitate folding of conformationally correct peptide loaded HLA complexes. After 3 days of folding, the sample is concentrated 100-fold to 10 mL using an Amicon concentrator. The concentrated sample was filtered through a 0.2 µm filter (Millipore) and purified by FPLC (Pharmacia) chromatography using an S-75 size exclusion column (Pharmacia). Sample was applied to column and washed at 2 mL/min with buffer (PBS pH 7.4). Figure 1 shows the typical chromatogram profile for the purification of refolded peptide-HLA-A2 monomer. In this Figure, 5 peaks are seen, which are marked as aggregates, refolded monomer, HLA-A2 heavy chain, beta2- microglobulin, and peptide alone. A typical purification will yield 8 to 12 mg of peptide-HLA-A2 monomer. After collecting the desired fractions (generally in 50 mL) the sample is concentrated to approximately 5 mL using an Amicon concentrator and biotinylated with biotin ligase following standard procedures (Avidity, CO). The biotin labeled monomer was isolated using the same approach as described above (data not shown). The biotin labeled material is then used for making tetramers as described in Figure 2.

Figure 2 illustrates size exclusion chromatography on Sephadex S-200 column of multimerized refolded monomer peak of Figure 1. Preparation and purification of peptide-HLA tetramer using S-200 size exclusion chromatography. To form tetramers of peptide-HLA-A2, biotin labeled monomer is mixed with streptavidin at either 4:1 or 8:1 molar ratios. The precise ratio is determined for each peptide-HLA preparation and is based on the ratio of the two proteins which generates the largest amount of tetramer band as determined by gel shift assays by SDS-PAGE. Generally, 8 mg of biotin labeled monomer is used, and after mixing with the appropriate amount of streptavidin, the sample (usually in 5 to 10 mL) is applied to the S-200 column for purification by FPLC. Figure 2 shows the chromatogram profile for a typical tetramer purification run on an S-200 column and as shown 4 peaks are present which represent tetramer, trimer, dimmer and monomer forms of the peptide-HLA-A2 complex. 3 and 4 mg of purified tetramer is routinely produced.

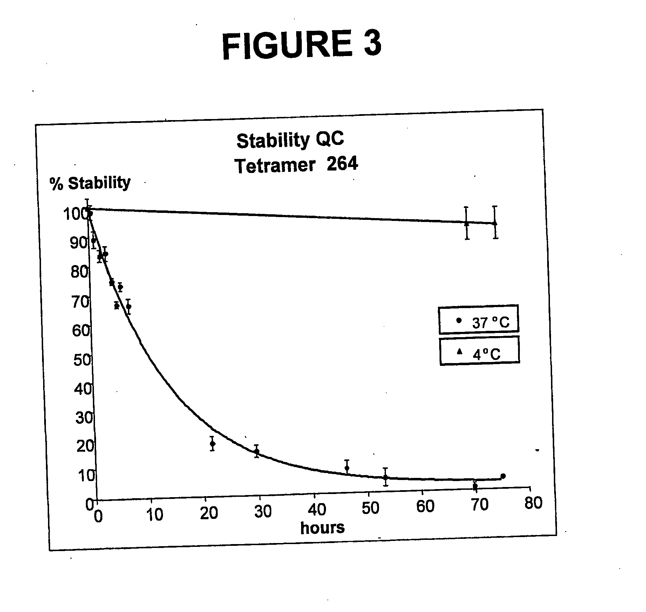

Figure 3 illustrates the stability of the 264 peptide-HLA-A2 tetramers. Tetramer stability was assessed in mouse serum at 4°C and 37°C. 25 µg of 264 peptide-tetramer complex was added to 5 mL of 100% mouse serum and incubated at 4°C and 37°C for 75hr. At designated times, 50 µL aliquots of sample were removed and stored at -20°C and remained frozen until completion of the experiment. To determine the integrity of the peptide-HLA tetramer, samples were evaluated using a sandwich ELISA and two antibodies, BB7.2 and W6/32 that bind only conformationally intact peptide-HLA tetramers. An ELISA protocol was developed using 96-well plates (Nunc maxisorb plates) that were coated O/N at 4°C with 0.5 µg of BB7.2,washed with buffer (PBS/0.05% Tween-20) and then blocked with 200 µl of 5% milk for 1 hr at room temperature. Sample (50 µL) from each time point was assayed in duplicate wells, incubated for 1 hr at room temperature, washed, and then 50 µL of at 1:1000 dilution of biotin conjugated W6/32 antibody was added to each well and incubated for 1 hr at room temperature. To detect bound antibody the streptavidin-HRP (horseradish peroxidase) conjugate was added to wells at 1:500 dilution, incubated for 15 minutes and washed, and then the assay was developed using ABTS substrate. All sample signals were plotted as % of control. Control tetramer was added to serum, mixed, and immediately removed for assaying by ELISA. The stability half-life for the 264-peptide-HLA-A2 tetramer at 4°C was greater than 72 hrs while at 37°C was approximately 10 hrs.

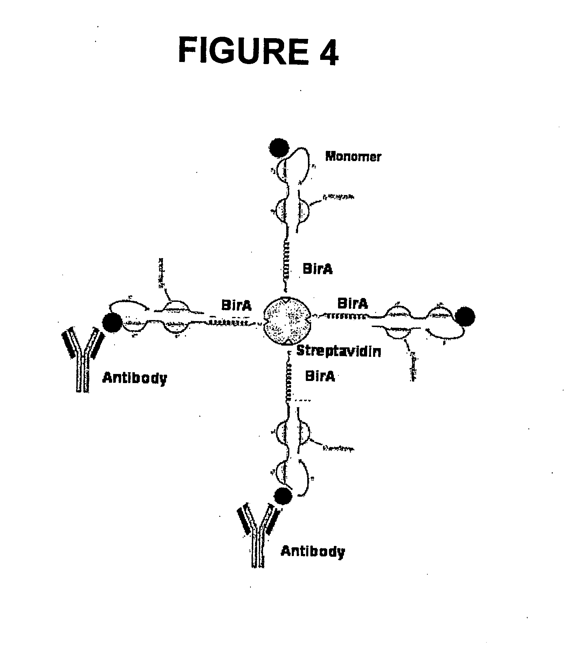

Figure 4 illustrates the complete structure of the peptide-HLA-A2 tetramer immunogen, as obtained from the tetramer peak of Figure 2, and recognition of the peptide-HLA epitope by a TCR mimic.

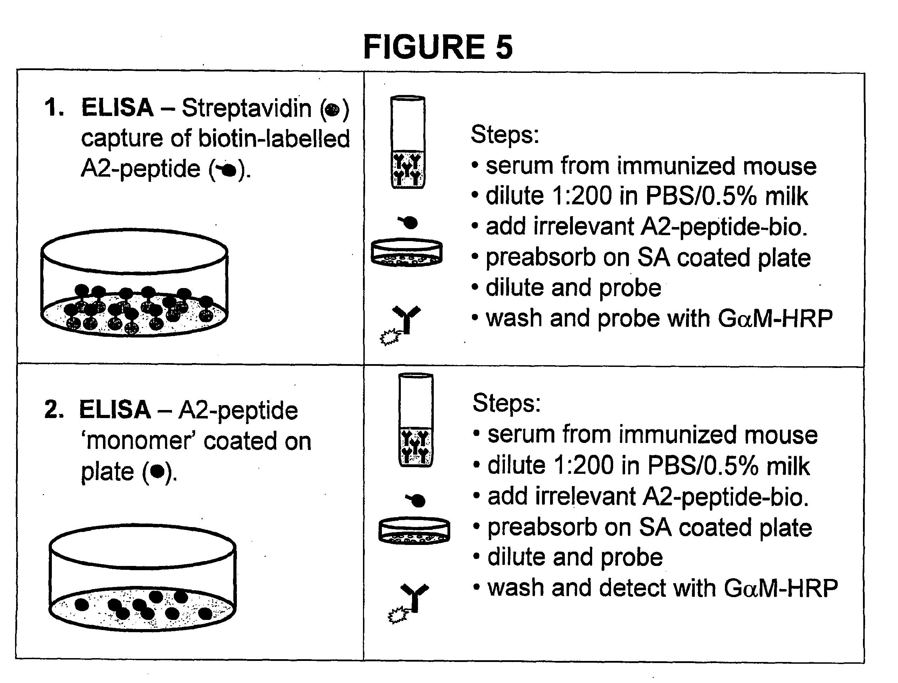

Figure 5 illustrates the development of an ELISA assay to screen mouse bleeds to determine if there are antibodies specific to the peptide-of-interest-HLA-molecule complex present. The schematic illustrates two newly developed screening assays for detection of anti-peptide-HLA specific antibodies from immunized mouse serum. Assay #2 evolved from Assay #1.

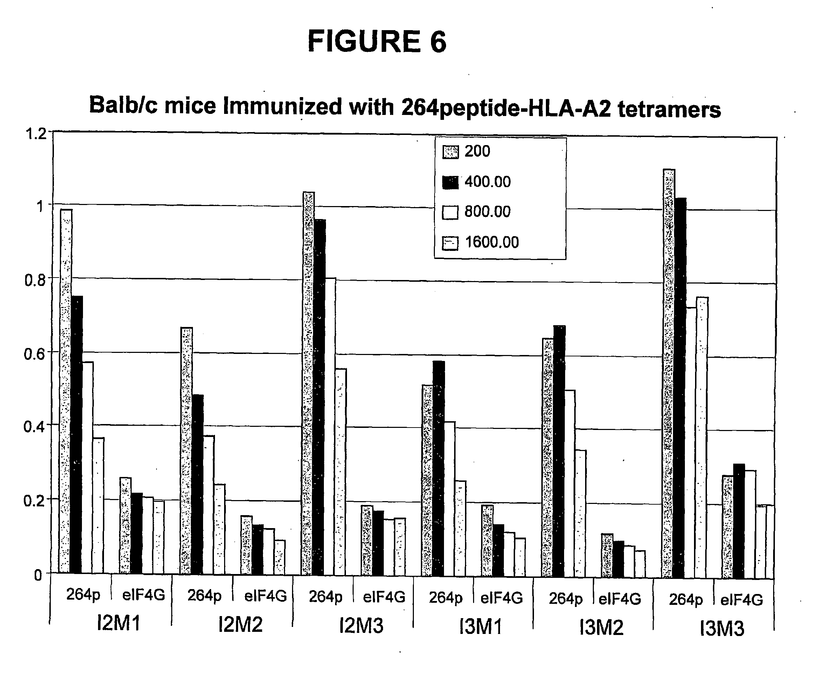

Figure 6 illustrates the results from an ELISA of 6 individual bleeds from Balb/c mice immunized with tetramers of 264 peptide-HLA-A2, using assay format #2 as described in Figure 5. Mice (male and female Balb/c; I3 and I2 groups, respectively) were immunized 4 times every 2 weeks by subcutaneous injection in the region behind the head or in the side flanks with 100 µl containing 50 µg of 264 peptide-HLA-A2 tetramer and 25 µg of QuilA (adjuvant). Bleeds were taken at 3 weeks, 5 weeks and just prior to sacrificing the mice. Figure 6 shows screening results from mice sera after 3 immunizations (week 5). Detection of polyclonal antibodies reactive for 264peptide-HLA-A2 tetramer was carried out by ELISA (assay #2 described in Figure 5). The ELISA results demonstrate that a 264 peptide-HLA-A2 antibody response can be elicited in both male (I3M1-M3) and female (I2M1-M3) mice using the immunization protocol and screening assay of the presently disclosed and claimed invention.

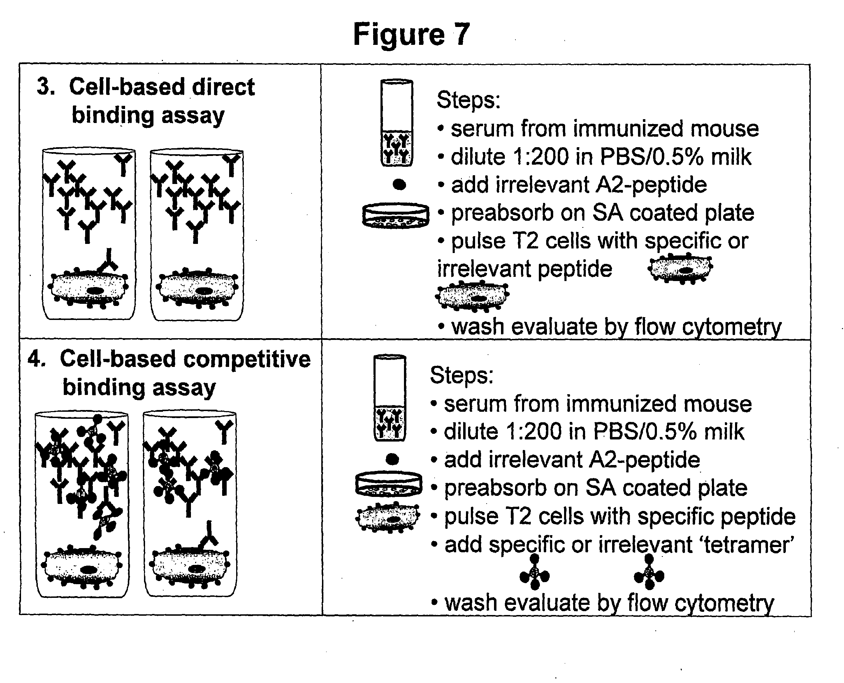

Figure 7 illustrates development of cell-based direct and competitive binding assays for screening mouse bleeds for antibodies specific to the peptide-of-interest-HLA-molecule complex. The schematic illustrates two newly developed cell-based screening assays for detection of anti-peptide-HLA specific antibodies from immunized mouse serum. Two cell based assays were developed: Assay #3 is a Cell-based direct binding approach and Assay #4 is a Cell-based competitive binding approach which uses soluble monomer or tetramer peptide-HLA-A2 complexes as competitors and non-competitors. The sensitivity of Assay # 4 is much greater than Assay #3.

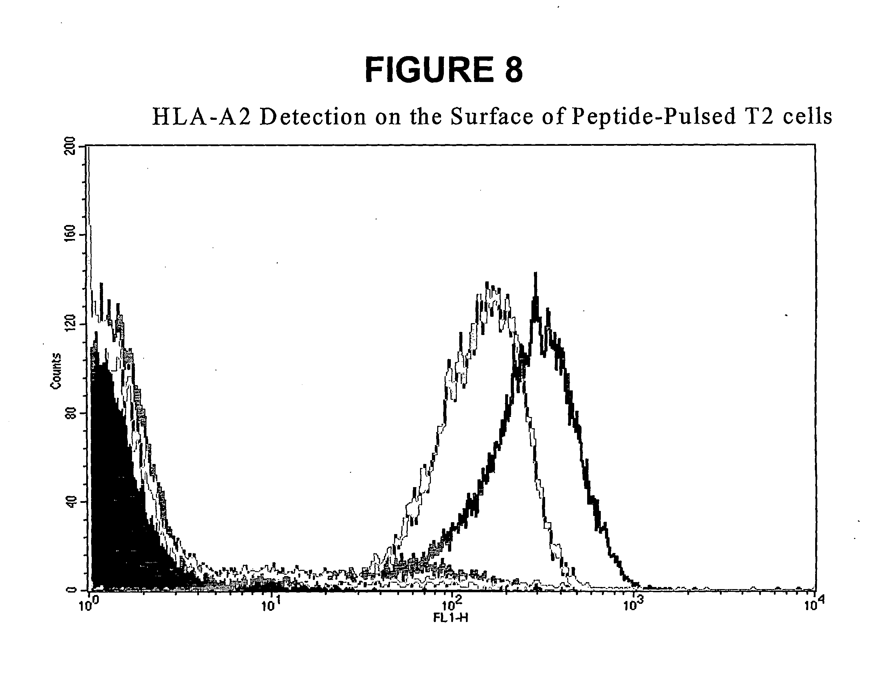

Figure 8 illustrates peptide loading of T2 cells. T2 cells (HLA-A2⁺, TAP deficient) were stained with BB7 antibody (specific for properly folded HLA-A2, ATCC #HB-82) to demonstrate that addition of exogenous peptide. increased the surface expression of the HLA-A2 molecule. 5x10⁵ T2 cells were incubated in 100µl of buffer containing 100µg of either 264 or elF4G peptide for 6 hours at 37°C, washed and stained with 0.5 µg BB7.2 for 20 min. Negative control cells were not pulsed with peptide. After staining, the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). Peptide pulsed T2 cells (open traces) shifted significantly to the right when stained, indicating the presence of HLA-A2 molecules on the surface, while unpulsed cells did not.

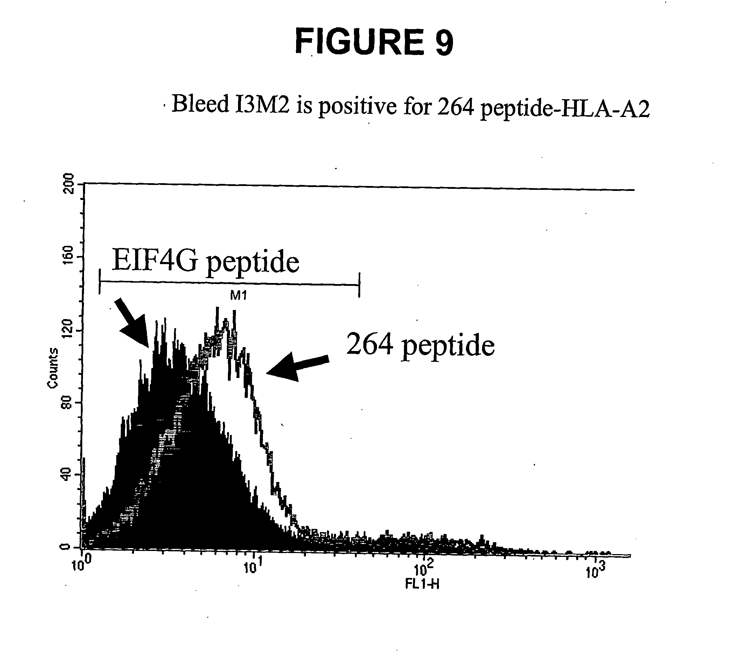

Figure 9 illustrates an example of the cell-based direct binding assay of Figure 7, which contains the results of staining of 264 peptide-loaded T2 cells with the I3M2 mouse bleed. T2 cells (HLA-A2⁺, TAP deficient) were stained with preabsorbed, diluted serum from mouse I3M2 (immunized with 264 tetramers) to demonstrate that antibodies exist in the serum which are specific for the 264p-HLA-A2 complex. 5x10⁵ T2 cells were incubated in 100 µl of buffer containing 100 µg of either 264 or elF4G peptide for 6 hours at 37°C, washed and stained with 100 µl of a 1:200 dilution of preabsorbed sera for 20 min. After staining the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). 264 peptide-pulsed T2 cells (open trace) shifted significantly to the right of the eIF4G peptide pulsed T2s when stained, indicating the presence of 264p-HLA-A2 specific antibodies from immunized mice.

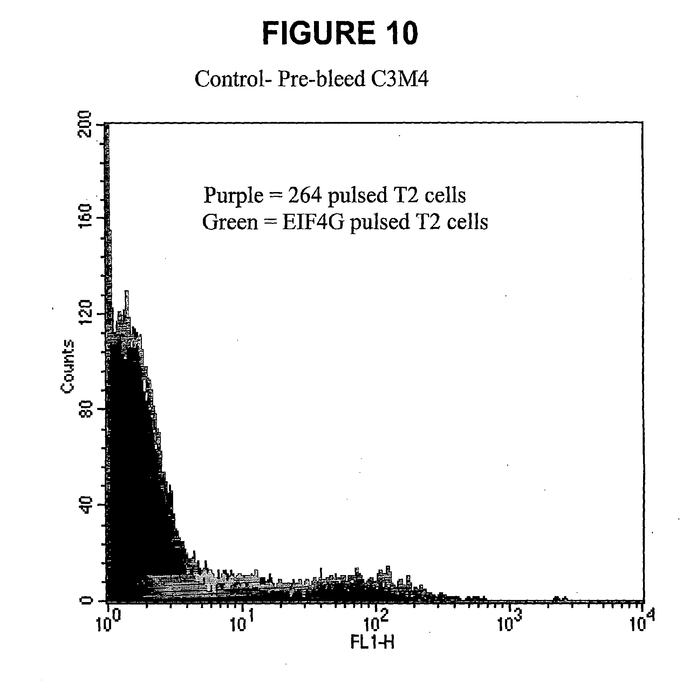

Figure 10 illustrates that pre-bleed samples (mice bleeds taken prior to immunization) show no sign of reactivity to T2 cells pulsed with either the 264- or elF4G peptides. T2 cells (HLA-A2⁺, TAP deficient) were stained with diluted serum from mouse C3M4 (unimmunized) to demonstrate that antibodies do not preexist in the serum which are specific for the 264p-HLA-A2 complex. 5x10⁵ T2 cells were incubated in 100 µl of buffer containing 100 µg of either 264 or elF4G peptide for 6 hours at 37°C, washed and stained with 100 µl of a 1:200 dilution of sera for 20 min. After staining the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). 264 peptide-pulsed T2 cells (filled trace) and elF4G peptide pulsed T2s (open trace) did not shift significantly from the origin when stained, indicating the absence of any HLA-A2 specific antibodies in the mouse's serum.

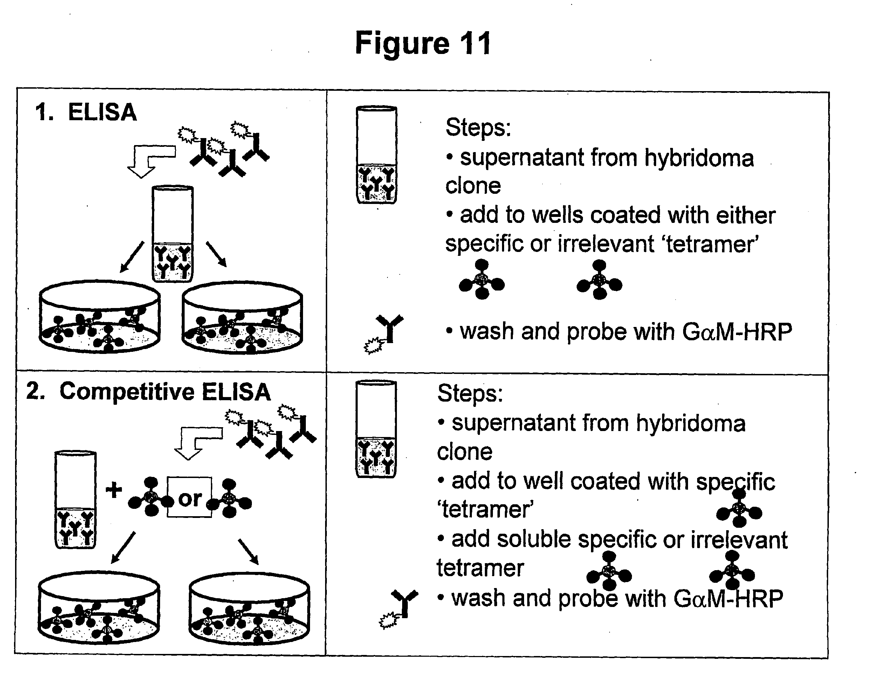

Figure 11 depicts development of assays to screen hybridomas to determine if they are producing anti-HLA-peptide specific antibodies. The schematic illustrates two ELISA-based screening assays for detection of anti-peptide-HLA specific monoclonal antibodies from culture supernatant. Assay #1 is an ELISA-based direct binding approach that coats wells of a 96-well plate with 0.5 µg of either specific or irrelevant tetramer. Hybridoma cell culture supernatant (50 µL) was assayed in duplicate by addition to an antibody coated plate blocked with 5% milk for 1 hr at room temperature. Plates were incubated for 1 hr at room temperature, washed, and probed with goat anti-mouse-HRP for 30 minutes. The assay was developed by adding 50 µL of either TMB or ABTS and read at 450 or 405 nm, respectively. Assay #2 is an ELISA that uses a competitive binding approach in which cell culture supernatant is incubated in the presence of either 300 ng of competitor or non-competitor (soluble monomer or tetramer peptide-HLA-A2 complexes) in wells on 96-well plates that have been coated with 100 ng of specific peptide-HLA-A2 tetramer and blocked with 5% milk. After 1 hr incubation, the plate is washed, probed with goat anti-mouse HRP and developed using TMB or ABTS.

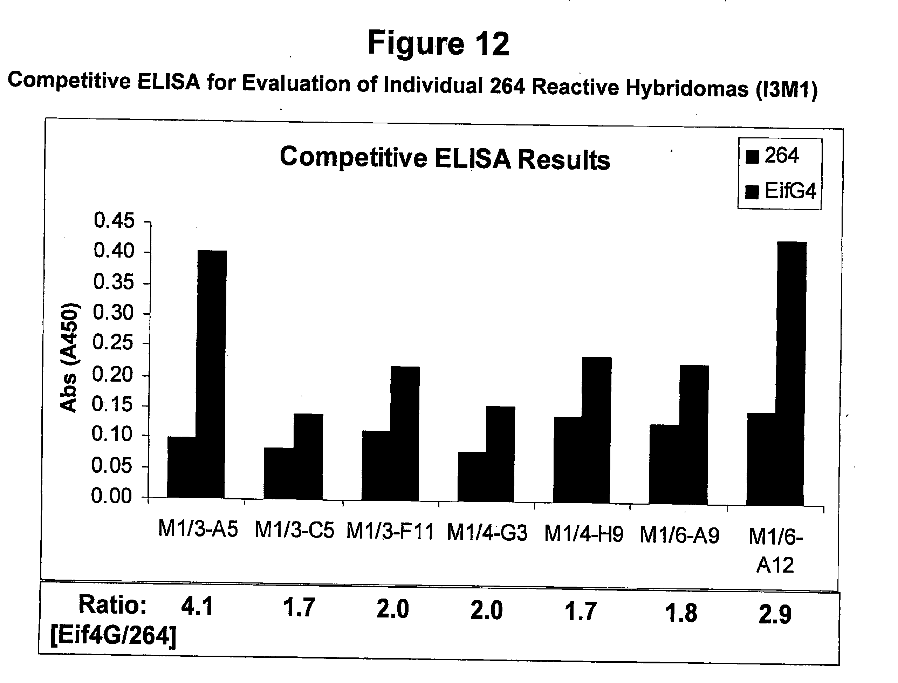

Figure 12 illustrates a competitive ELISA assay for evaluation of individual hybridomas (I3M1) reactive against 264p-HLA-A2 complexes. Redbar= addition of 264p-HLA-A2 tetramer (competitor, 0.3 µg); Blue bar = addition of eIF4Gp-HLA-A2 tetramer (non-competitor, 0.3 µg). Hybridoma cell culture supernatant (50 µL) was incubated in the presence of 300 ng of competitor (264 peptide-HLA-A2 tetramer) or non-competitor (eIF4G peptide-HLA-A2 tetramer) in wells on a 96-well plate coated previously with 100 ng of 264 peptide-HLA-A2 tetramer. After 1 hr incubation, the plate was washed, probed with goat anti-mouse HRP, developed using TMB or ABTS and read at 450 or 405 nm, respectively. Results were calculated by dividing the absorbance read in the presence of non-competitor by the absorbance read in the presence of competitor [eIF4G/264]. Ratios of 2 or greater were considered to be positive, and hybridoma clones with this desired ratio were selected for further analysis. Figure 12 shows 4 different hybridoma supernatants (M1/3-A5, M1/3-F11, M1/4-G3, and M1/6-A12) with a specific binding ratio [eIF4G/264] of 2 or greater.

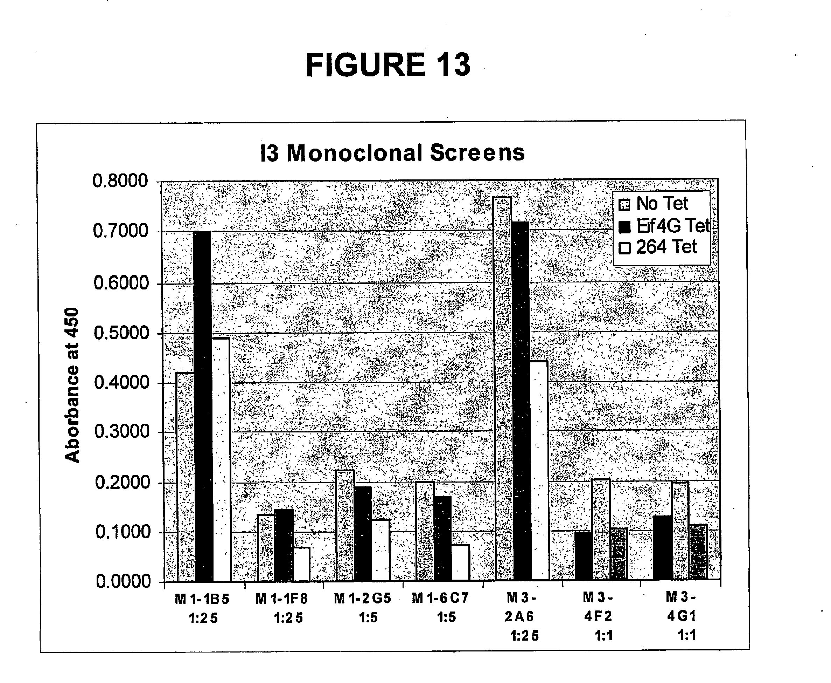

Figure 13 illustrates the results of a competitive ELISA assay for evaluation of individual hybridomas to determine if the hybridoma produced from mouse bleed I3M1 expresses anti-264-HLA-A2 antibodies. Hybridoma cell culture supernatant (50 µL) was incubated without any tetramer addition or in the presence of 300 ng of competitor (264 peptide-HLA-A2 tetramer) or non-competitor (eIF4G peptide-HLA-A2 tetramer) in wells on a 96-well plate coated previously with 100ng of 264 peptide-HLA-A2 tetramer. After 1 hr incubation, the plate was washed, probed with goat anti-mouse HRP, developed using TMB or ABTS and read at 450 or 405 nm, respectively Figure 13 illustrates three different hybridoma supernatants with favorable eIF4G/264 ratios. These include M1-1F8, M1-2G5, M1-6C7 and M3-2A6, which were selected for further analysis.

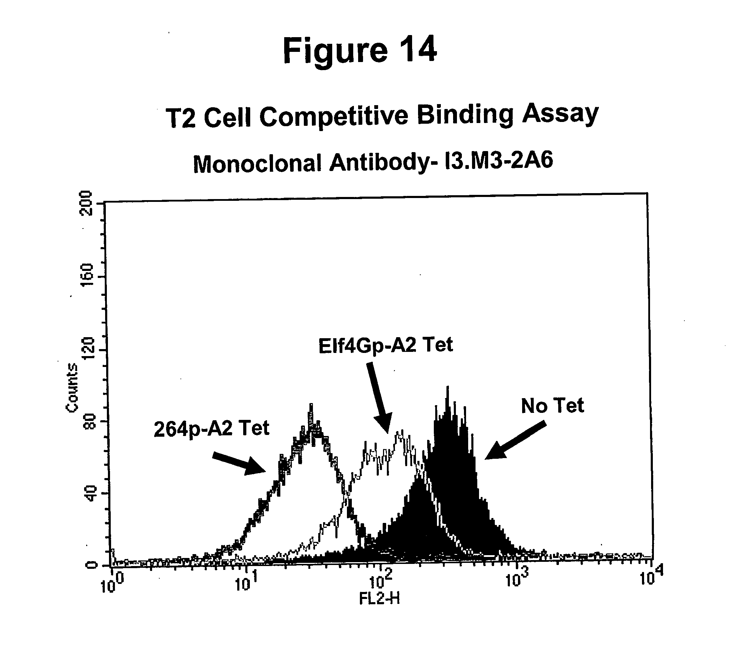

Figure 14 illustrates the characterization of monoclonal antibody 13.M3-2A6 by the cell-based competitive binding assay. T2 cells (HLA-A2⁺, TAP deficient) were stained with cell supernatant from hybridoma I3.M3-2A6 (immunogen = 264 tetramers) in the presence of (1) tetramer complex that would compete with specific binding to 264p-HLA-A2; (2) tetramer complex that would not compete with specific binding (eIF4Gp); or (3) no tetramer, to demonstrate that the antibody specifically recognizes the 264p-HLA-A2 complex on the cell surface. Cell supernatant was pre-absorbed against 20 µg of soluble Her2/neu-peptide-HLA-A2 complexes, diluted 1:200 and added (100 µl) to tube containing 1 µg of either 264p-HLA-A2 tetramer (competitor) or eIF4Gp-HLA-A2 tetramer (non competitor) for 15 minutes at room temperature. 5x10⁵ T2 cells were incubated in 100 µl of buffer containing 100 µg of 264 peptide for 6 hours at 37°C, washed, resuspended in 100 µl, and added to the preabsorbed/tetramer treated supernatant for 20 minutes at room temperature. After staining, the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). 264 peptide-competition resulted in a significant shift of the T2 cell trace (thick line, open trace) to the left (towards the origin) while the eIF4G peptide competition (thin line, open trace) resulted in a much smaller shift away from T2s stained in the absence of tetramer, indicating the presence of a monoclonal antibody with a high degree of specificity for the 264p-HLA-A2 complex.

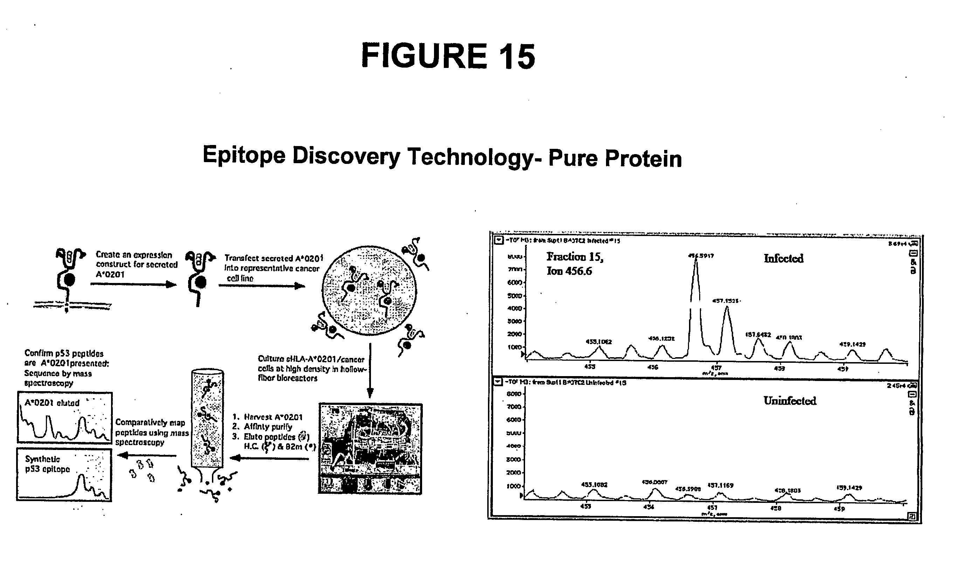

Figure 15 illustrates a broad outline of the epitope discovery technology described in detail in Hildebrand et al. (US Serial No. 09/974,366, filed October 10, 2001. Soluble HLA-secreting transfectants are created in a cancerous or diseased cell line of interest. In a separate experiment, a normal (i.e., noncancerous or non-diseased) cell line also transfected with a construct encoding the soluble HLA is grown and cultured. Soluble HLA molecules are collected from both cell lines, and the peptides are eluted. Mass spectrometric maps are generated comparing cancerous (or diseased) peptides to normal peptides. Differences in the maps are sequenced to identify their precise amino acid sequence, and such sequence is utilized to determine the protein from which the peptide was derived (i.e., its "source protein"). This method was utilized to identify the peptide elF4G, which has a higher frequency of peptide binding to soluble HLA-A2 in HIV infected cells compared to uninfected cells. This protein is known to be degraded in HIV infected T cells, and elevated levels of the elF4G peptide presented by HLA-A2 molecules was determined using this technology.

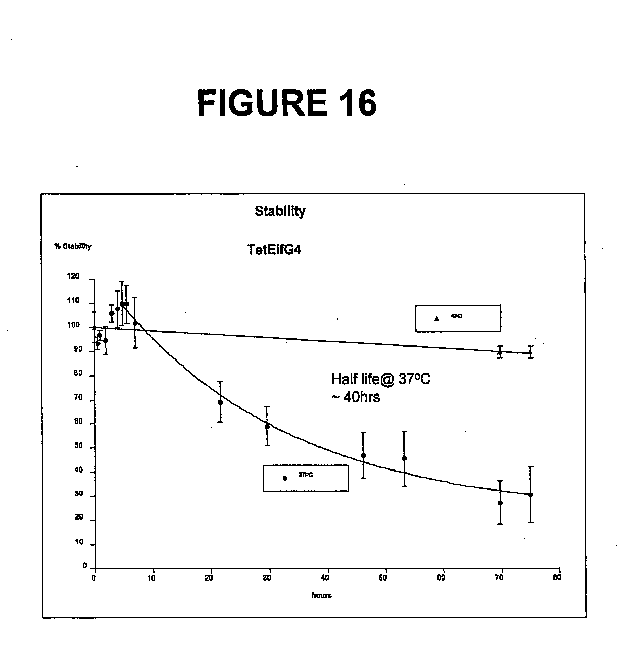

Figure 16 illustrates the stability of the eIF4Gp-HLA-A2 tetramers. Tetramer stability was assessed in mouse serum at 37°C (•) and at 4°C (▲) using the conformational antibodies BB7.2 and W6/32. 25 µg of elF4G peptide-tetramer complex was added to 5 mL of 100% mouse serum and incubated at 4oC and 37°C for 75hr. At designated times, 50 µL aliquots of sample were removed and stored at -20°C and remained frozen until completion of the experiment. To determine the integrity of the peptide-HLA tetramer, samples were evaluated using a sandwich ELISA and two antibodies, BB7.2 and W6/32 that bind only conformationally intact peptide-HLA tetramers. An ELISA protocol was developed using 96-well plates (Nunc maxisorb plates) that were coated O/N at 4°C with 0.5 µg of BB7.2, washed with buffer (PBS/0.05% Tween-20) and then blocked with 200 µl of 5% milk for 1 hr at room temperature. Sample (50mL) from each time point was added in duplicate wells, incubated for 1 hr at room temperature, washed, and then 50 µL of at 1:1000 dilution of biotin conjugated W6/32 antibody was added to each well and incubated for 1 hr at room temperature. To detect bound antibody the streptavidin-HRP (horseradish peroxidase) conjugate was added to wells at 1:500 dilution, incubated for 15 minutes, washed and then assay was developed using ABTS substrate. All sample signals were plotted as % of control. Control tetramer was added to serum, mixed, and immediately removed for assaying by ELISA. The half-life of stability for the eIF4G-peptide-HLA-A2 tetramer at 4°C was greater than 72hrs while at 37°C the half-life was approximately 40 hrs.

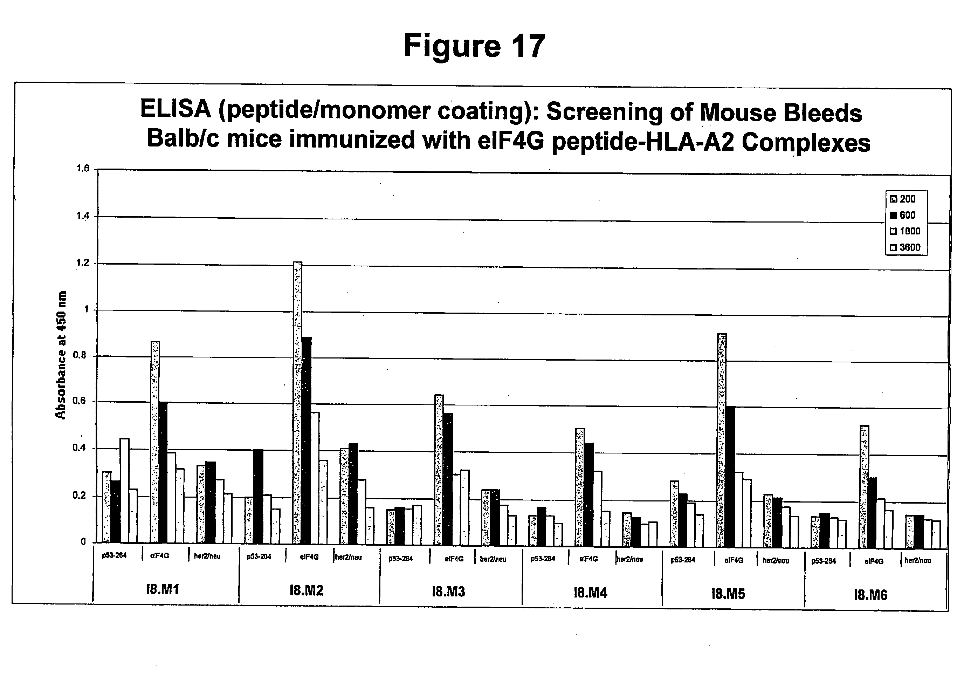

Figure 17 illustrates the results from an ELISA of bleeds from 6 individual Balb/c mice immunized with tetramers of eIF4Gp-HLA-A2. Mouse samples from left to right are I8.M1, I8.M2, I8.M3, 18.M4, I8.M5, 18.M6. P53-264 = 264p-HLA-A2 monomer (0.5 µg/well), Eif4G = eIF4Gp-HLA-A2 monomer (0.5 µg/well), and Her2/neu = Her2/neu peptide-HLA-A2 monomer (0.5 µg/well). The dilutions of sample bleeds start at 1:200 (blue bar) and titrate down to 1:3600 (light blue bar). Mice (female Balb/c) were immunized 4 times every 2 weeks by subcutaneous injection in the region behind the head or in the side flanks with 100 µl containing 50 µg of elF4G peptide-HLA-A2 tetramer and 25 µg of QuilA (adjuvant). Bleeds were taken at 3 weeks, 5 weeks and just prior to sacrificing mice. Figure 17 shows results from mice sera after 3 immunizations (week 5). Detection of polyclonal antibodies reactive for eIF4G peptide-HLA-A2 tetramer was carried out by ELISA (assay #2 described in Figure 5). The ELISA results demonstrate that a 264 peptide-HLA-A2 antibody response can be elicited in female Balb/c (18.M1-M6) mice using the immunization protocol and screening assay of the presently disclosed and claimed invention.

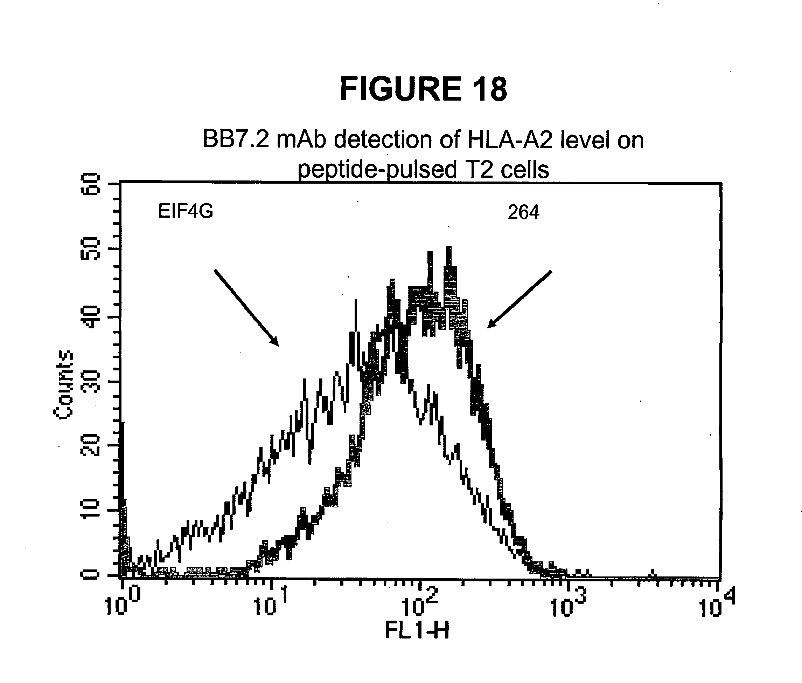

Figure 18 illustrates T2 cell direct binding assay performed according to the method of Figure 7. T2 cells (HLA-A2⁺, TAP deficient) were stained with BB7.2 antibody (specific for HLA-A2) to demonstrate that HLA-A2 was present on the surface on these cells. T2 cells were incubated in 100 µl of buffer containing 100 µg of either 264 or elF4G peptide for 6 hours at 37°C, washed and stained with 0.5 µg BB7.2 for 20 min. Negative control cells were not pulsed with peptide. After staining, the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, Califomia) and analyzed using Cell Quest software (version 3.3, BD Biosciences). BB7.2 binding was slightly stronger with T2 cells loaded with 264 peptide as indicated by the slightly greater rightward shift with 264 pulsed-T2 cells compared to elF4G pulsed cells.

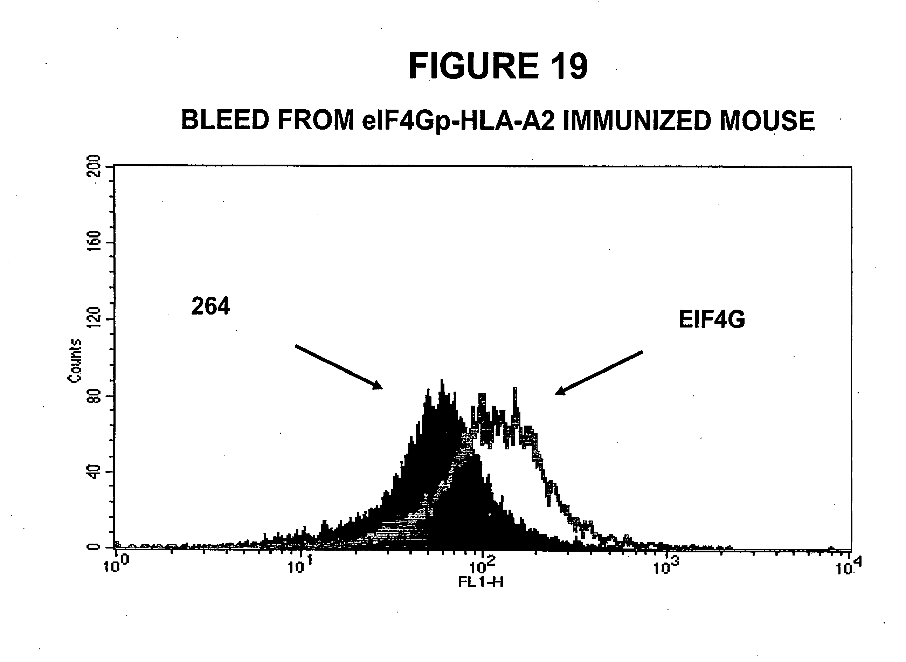

Figure 19 illustrates the results of staining of elF4Gp-loaded T2 cells with a bleed from an eIF4Gp-HLA-A2 immunized mouse. T2 cells (HLA-A2⁺, TAP deficient) were stained with preabsorbed, diluted serum from mouse I8M2 (immunized with eIF4G tetramers) to demonstrate that antibodies exist in the serum which are specific for the eIF4Gp-HLA-A2 complex. 5x10⁵ T2 cells were incubated in 100 µl of buffer containing 100 µg of either eIF4G or 264 peptide for 6 hours at 37°C, washed and stained with 100 µl of a 1:200 dilution of preabsorbed sera for 20 min. After staining, the reaction was washed once with 3-4 ml wash buffer and resuspended in approximately 100 µl of wash buffer containing 0.5 µg of FITC-conjugated goat anti-mouse IgG (Caltag, Burlingame, CA). Samples were collected on a FACscan (BD biosciences, San Diego, California) and analyzed using Cell Quest software (version 3.3, BD Biosciences). eIF4G peptide-pulsed T2 cells (open trace) shifted significantly to the right of the 264 peptide pulsed T2s when stained, indicating the presence of eIF4Gp-HLA-A2 specific antibodies from immunized mice.

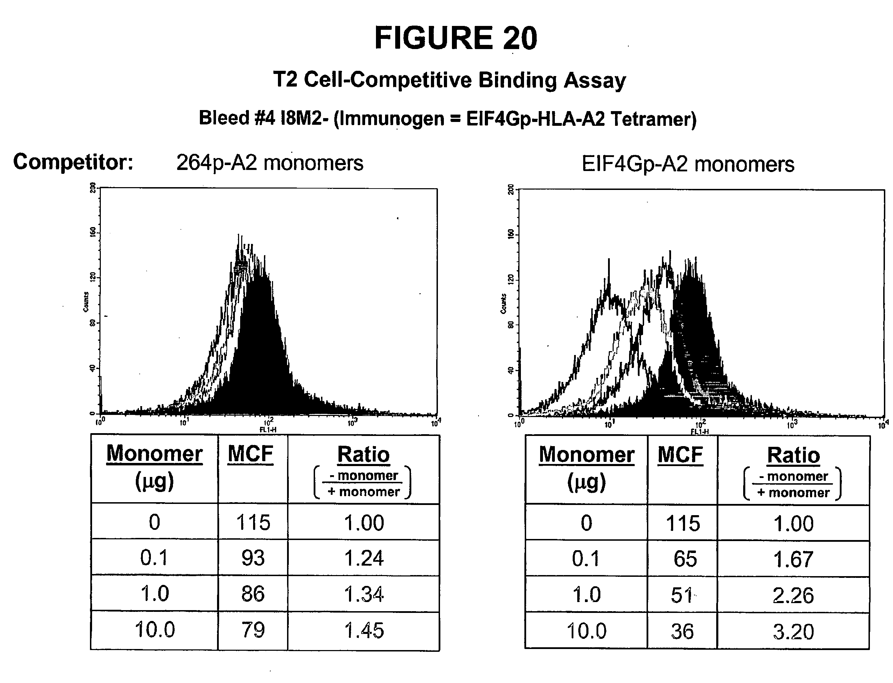

Figure 20 illustrates the results of a T2 cell-competitive binding assay, the method of which is outlined in Figure 7. T2 cells (HLA-A2⁺, TAP deficient) were stained with pre-absorbed, diluted serum from mouse 18M2 (immunized with elF4Gp tetramers) in the presence of (1) monomer complex that would compete with specific binding to eIF4Gp-HLA-A2; (2) monomer complex that would not compete with specific binding (264p); or (3) no monomer, to demonstrate that the antibody specifically recognizes the eIF4Gp-HLA-A2 complex on the cell surface. Cell supernatant was pre-absorbed against 20 µg of soluble Her2/neu-peptide-HLA-A2 complexes, diluted 1:200 and added (100 µl) to tube containing 1 µg of either eIF4Gp-HLA-A2 monomer (competitor) or 264p-HLA-A2 monomer (non competitor) for 15 minutes at room temperature. 5x10⁵ T2 cells were incubated in 100 µl of buffer containing 100 µg of elF4G peptide for 6 hours at 37°C, washed, resuspended in 100 µl, and added to the preabsorbed/monomertreated supernatant for 20 minutes at room temperature. After staining, the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). eIF4G peptide-competition resulted in a significant shift of the T2 cell trace (thick line, open trace) to the left (towards the origin) while the 264 peptide competition (thin line, open trace) resulted in a much smaller shift away from T2s stained in the absence of monomer, indicating the presence of polyclonal antibodies with a high degree of specificity for the eIF4Gp-HLA-A2 complex.

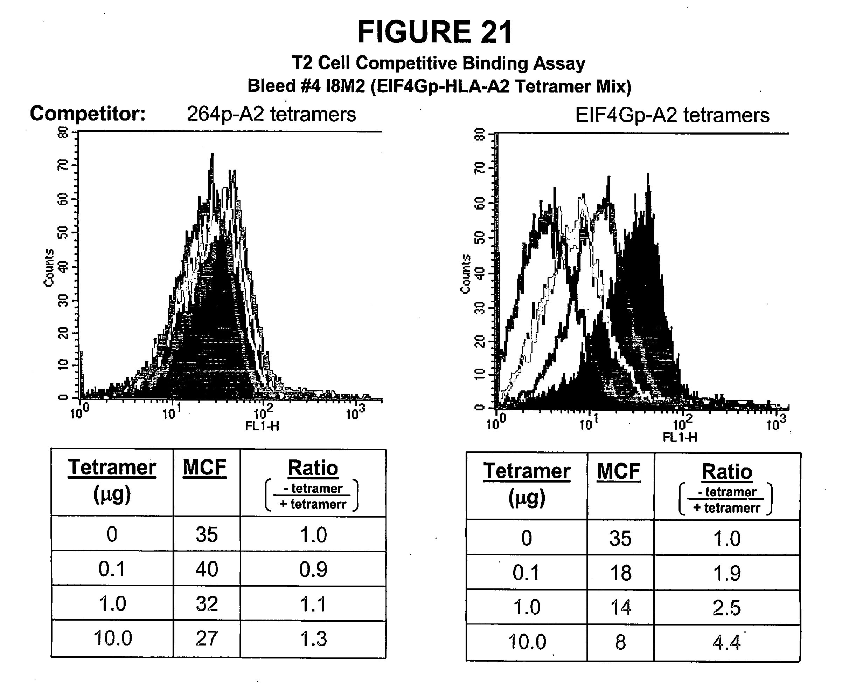

Figure 21 illustrates the results of another T2 cell-competitive binding assay similar to the one described in Figure 20, except that the competitor mixed with the mouse bleed prior to reacting with the T2 cells was in the form of a tetramer rather than a monomer. T2 cells (HLA-A2⁺, TAP deficient) were stained with pre-absorbed, diluted serum from mouse I8M2 (immunized with eIF4Gptetramers) in the presence of (1) tetramer complex that would compete with specific binding to eIF4Gp-HLA-A2; (2) tetramer complex that would not compete with specific binding (264p); or (3) no tetramer, to demonstrate that the antibody specifically recognizes the eIF4Gp-HLA-A2 complex on the cell surface. Cell supernatant was pre-absorbed against 20 µg of soluble Her2/neu-peptide-HLA-A2 complexes, diluted 1:200 and added (100 µl) to tube containing 1 µg of either eIF4Gp-HLA-A2 tetramer (competitor) or 264p-HLA-A2 tetramer (non competitor) for 15 minutes at room temperature. 5x10⁵ T2 cells were incubated in 100 µl of buffer containing 100 µg of elF4G peptide for 6 hours at 37°C, washed, resuspended in 100 µl, and added to the preabsorbed/tetramer treated supernatant for 20 minutes at room temperature. After staining, the reaction was washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). eIF4G peptide-competition resulted in a significant shift of the T2 cell trace (thick line, open trace) to the left (towards the origin), while the 264 peptide competition (thin line, open trace) resulted in a much smaller shift away from T2s stained in the absence of tetramer, indicating the presence of polyclonal antibodies with a high degree of specificity for the eIF4Gp-HLA-A2 complex.

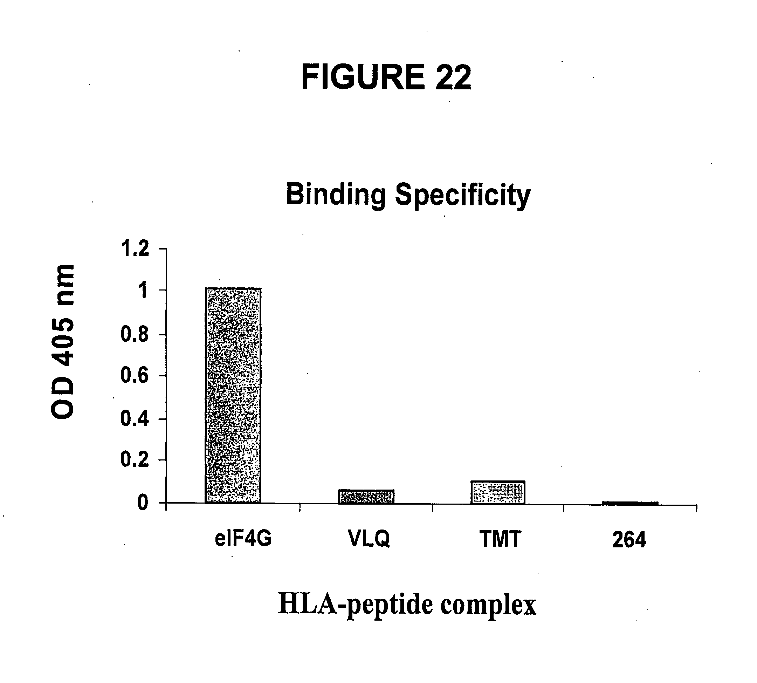

Figure 22 illustrates the binding specificity of mAb 4F7, as determined by ELISA. To assess the binding specificity of 4F7 TCR mimic, a 96-well plate was coated with 0.5 µg of specific (elF4G-peptide-HLA-A2 monomer) and non-specific (264, VLQ and TMT peptide-HLA-A2 monomers). The VLQ and TMT peptides are derived from the human beta-chorionic gonadotropin protein. After blocking wells with 5% milk, 100 ng of 4F7 antibody was added to each well and incubated for 1 hr at room temperature. Plates were washed, probed with 500 ng/well of goat anti-mouse IgG-HRP and developed using ABTS. These results show specific binding of 4F7 to eIF4G peptide-HLA-A2 tetramer coated wells but no binding to wells coated with non-relevant peptide-loaded HLA-A2 complexes.

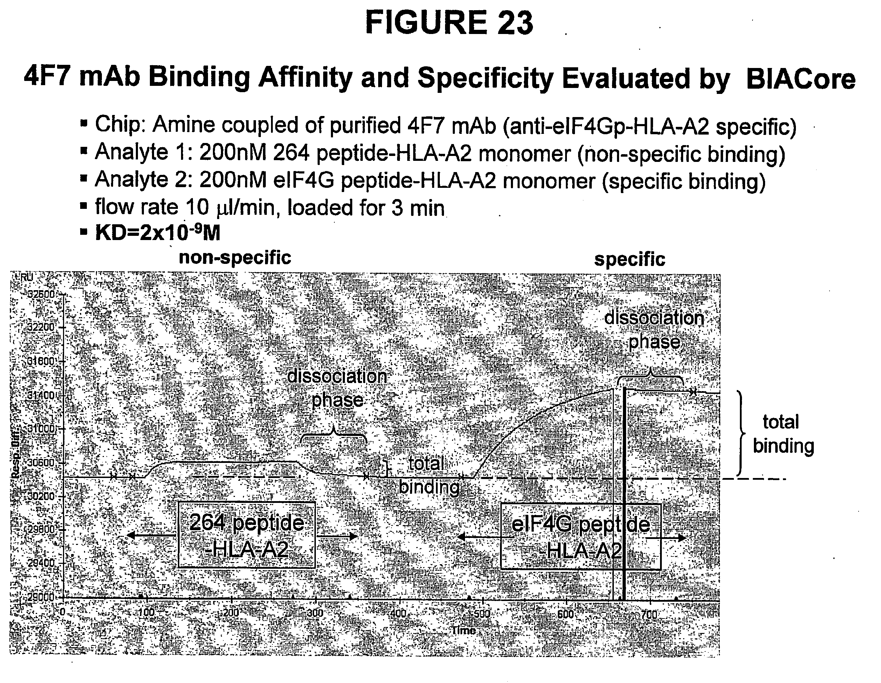

Figure 23 illustrates 4F7 TCR mimic binding affinity and specificity evaluated by surface plasmon resonance (BIACore). SPR (BIACore) was used to determine the binding affinity constant for 4F7 TCR mimic. Various concentrations of soluble monomer peptide-HLA-A2 (10, 20, 50, and 100 nM) were run over a 4F7 coated chip (4F7 coupled to a biosensor chip via amine chemistry), and then BIACore software was used to best fit the binding curves generated. The affinity constant of 4F7 mAb for its specific ligand was determined at 2 x10⁻⁹M.

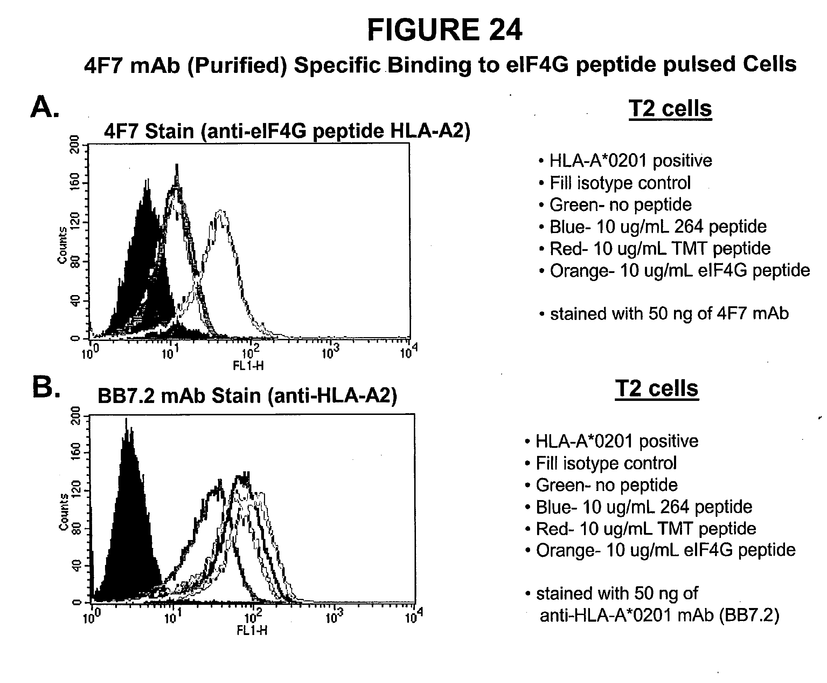

Figure 24 illustrates the specific binding of purified 4F7 mAb to eIF4G peptide pulsed cells. T2 cells (HLA-A2⁺, TAP deficient) were stained with cell supernatant from hybridoma 4F7 (immunogen = eIF4Gp tetramers) to demonstrate binding specificity for this monoclonal antibody for the eIF4Gp-HLA-A2 complex. 5x10⁵ T2 cells were incubated in 100 µl of buffer containing 100 µg of eIF4G, 264, or TMT peptide for 6 hours at 37°C, washed and stained with 100 µl of 4F7 culture supernatant for 20 min. In addition, cells that were not peptide pulsed were stained in an identical manner with 4F7 to determine the level of background or endogenous elF4Gp presented by HLA-A2 on T2 cells. After staining, the reactions were washed once with 3-4 ml wash buffer and resuspended in approximately 100 µl of wash buffer containing 0.5 µg of FITC-conjugated goat anti-mouse IgG (Caltag, Burlingame, CA). Cells were washed as above and resuspended in 0.5 ml wash buffer for analysis. As shown in Figure 24-A, samples were collected on a FACscan (BD biosciences, San Diego, California) and analyzed using Cell Quest software (version 3.3, BD Biosciences). eIF4G peptide-pulsed T2 cells shifted most significantly to the right of the IgG1 isotype stain. Both 264 and TMT peptide pulsed cells overlaid exactly with the 4F7 monoclonal stain of T2 cells that were not peptide pulsed, indicating that 4F7 recognizes a low level of endogenous elF4G peptide on T2 cells. These data also demonstrate specific binding of the 4F7 monoclonal antibody for elF4G peptide-pulsed T2 cells. Because peptide pulsed T2 cells showed a greater staining intensity with BB7.2 monoclonal antibody compared to cells that were not pulsed (Figure 24-B), it is concluded that the 4F7 monoclonal antibody does not react non-specifically against HLA-A2.

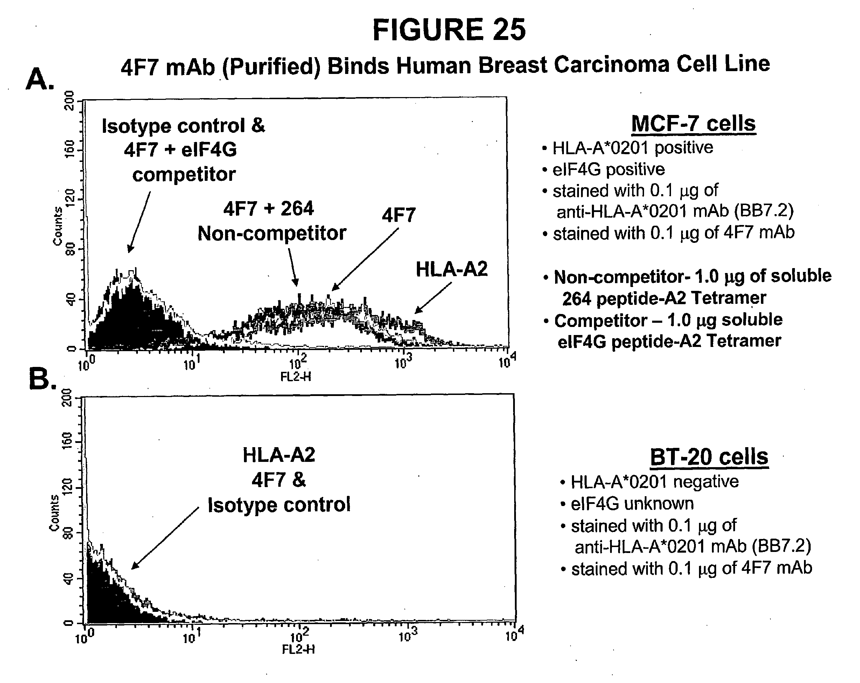

Figure 25 illustrates that purified 4F7 mAb binds eIF4Gp-HLA-A2 complexes on human breast carcinoma cell line MCF-7. MCF-7 cells (HLA-A2⁺) were stained with cell supernatant from hybridoma 4F7 (immunogen = eIF4Gp tetramers) in the presence of (1) tetramer complex that would compete with specific binding to eIF4Gp-HLA-A2; (2) tetramer complex that would not compete with specific binding (264p); or (3) no tetramer, to demonstrate that the antibody specifically recognizes the endogenous eIF4Gp-HLA-A2 complex on the cell surface. 5x10⁵ MCF-7 cells were incubated in 100 µl of buffer containing 100 µl of 4F7 culture supernatant plus 1 µg of either elF4Gp-HLA-A2 tetramer (competitor) or 264p-HLA-A2 tetramer (non competitor) or no addition for 15 minutes at room temperature. After staining, the reactions were washed once with 3-4 ml wash buffer and resuspended in approximately 100 µl of wash buffer containing 0.5 µg of PE-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). The data shown in Figure 25-A demonstrate 4F7 binding specificity for endogenous peptide eIF4Gp-HLA-A2 complexes on MCF-7 tumor cells. In panel B, it is shown that 4F7 and BB7.2 do not bind to HLA-A2 negative BT-20 breast cancer cells, further supporting the claim for 4F7 monoclonal antibody binding specificity for eIF4G peptide presented in the context of HLA-A2.

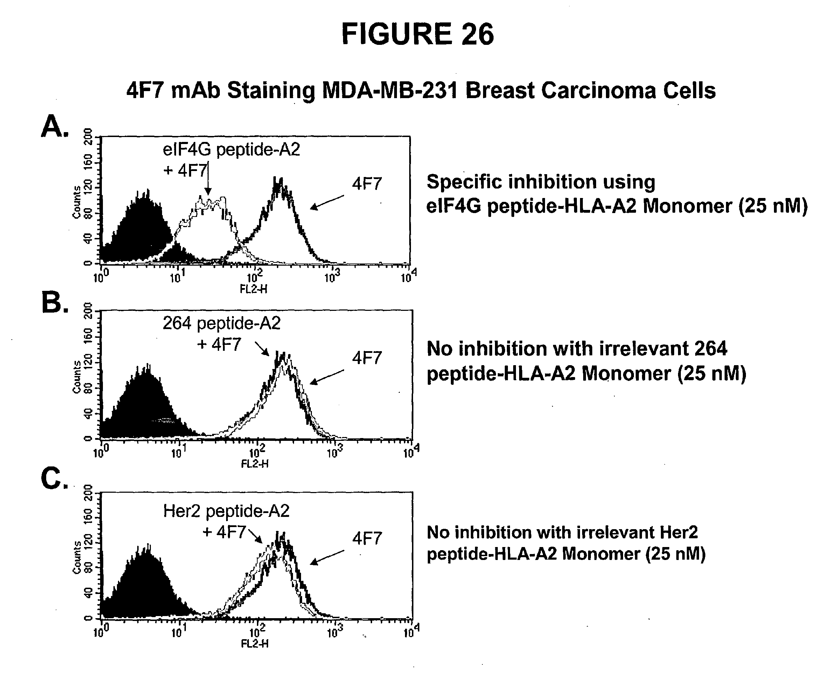

Figure 26 illustrates staining of MDA-MB-231 cells with 4F7 mAb (50 ng) in the absence or presence of soluble peptide-HLA-A2 monomers including elF4Gp (competitor; 25 nM), 264p (non-competitor; 25 nM) or Her2/neu peptide (non-competitor; 25 nM). MDA-MB-231 cells (HLA-A2⁺) were stained with cell supernatant from hybridoma 4F7 (immunogen = elF4Gp tetramers) in the presence of (1) monomer complex that would compete with specific binding to eIF4Gp-HLA-A2; (2) monomer complex that would not compete with specific binding to elF4Gp-HLA-A2 (264p and Her-2/neu); or (3) no monomer, to demonstrate that the antibody specifically recognizes endogenous eIF4Gp-HLA-A2 complex on the cell surface. 5x10⁵ MDA-MB-231 cells were incubated in 100 µl of buffer containing 100 µl of 4F7 culture supernatant plus 25 nM of eIF4Gp-HLA-A2 tetramer (competitor), 264p-HLA-A2 tetramer or Her-2/neu-HLA-A2 (non competitors) or no addition for 15 minutes at room temperature. After staining, the reactions were washed once with 3-4 ml wash buffer and resuspended in approximately 100 µl of wash buffer containing 0.5 µg of PE-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). Figure 26-A demonstrates 4F7 binding specificity for endogenous eIF4Gp-HLA-A2 complexes on MDA-231 tumor cells. Binding of the 4F7 TCR mimic to MDA-MB-231 cells is significantly reduced (see leftward shift with peak) in the presence of 25nM of competitor (eIF4Gp-HLA-A2 monomer). In panels B and C, it is shown that 4F7 binding is not blocked when non-relevant (264 and Her-2/neu) peptide-HLA-A2 monomers are used to compete with 4F7 binding to MDA-231 cells. These findings support previous binding specificity data and indicate eIF4Gp-HLA A2 as a novel tumor antigen.

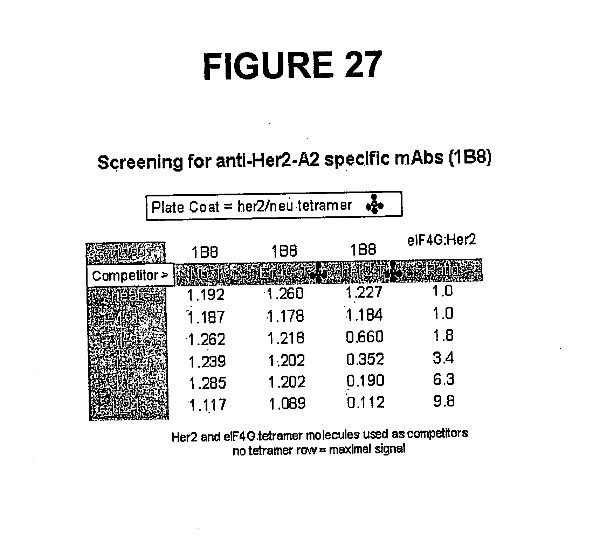

Figure 27 illustrates specific 1B8 mAb binding demonstrated by competitive ELISA. A competitive ELISA was used to screen for Her-2/neu-HLA-A2 reactive antibodies. The assay identified 36 candidates. The screening profile described here is for 1 B8 and is representative of many of the other anti-Her-2/neu-HLA-A2 reactive monoclonal antibodies identified. 1 B8 hybridoma cell culture supernatant was evaluated neat and at 3-fold decreasing amounts in a competitive ELISA. 50 µL samples from each dilution was added in duplicate to wells on a 96-well plate, and incubated without any tetramer addition or in the presence of 300 ng of competitor (Her-2/neu peptide-HLA-A2 tetramer) or non-competitor (elF4G peptide-HLA-A2 tetramer) in wells coated previously with 100 ng of Her-2/neu peptide-HLA-A2 tetramer. After 1hr incubation, the plate was washed, probed with goat anti-mouse HRP, developed using ABTS and read at 405 nm. The 1B8 TCR mimic supernatant at 1:243 dilution showed a specific binding ratio [eIF4G:Her2] of almost 10, demonstrating binding specificity for the Her-2/neu peptide-HLA-A2 complex.

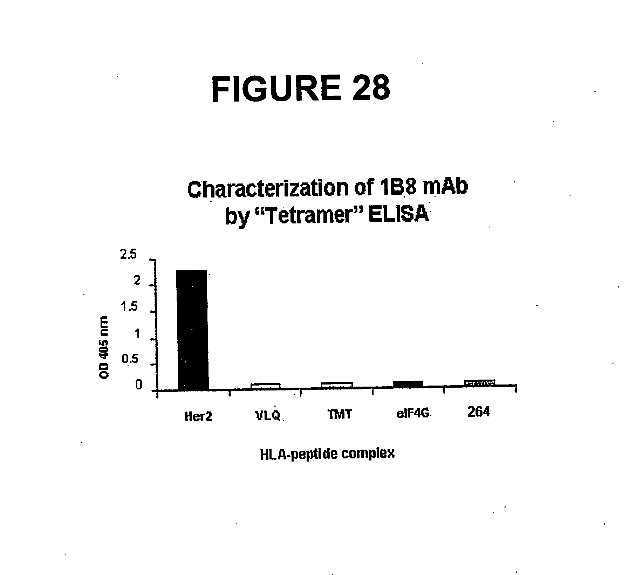

Figure 28 demonstrates the specificity of the 1 B8 mAb by tetramer ELISA. To assess the binding specificity of 1 B8 TCR mimic, a 96-well plate was coated with 0.5 µg of specific (Her-2/neu) and non-specific (264, eIF4G, VLQ and TMT) peptide-HLA-A2 monomers. After blocking wells with 5% milk, 100 ng of Protein-A purified 1B8 antibody was added to each well and incubated for 1 hr at room temperature. Plates were washed, probed with 500 ng/well of goat anti-mouse IgG-HRP and developed using ABTS. These results show specific binding of 1 B8 to Her-2/neu peptide-HLA-A2 tetramer coated wells but does not show any binding to wells coated with non-relevant peptide-loaded HLA-A2 complexes.

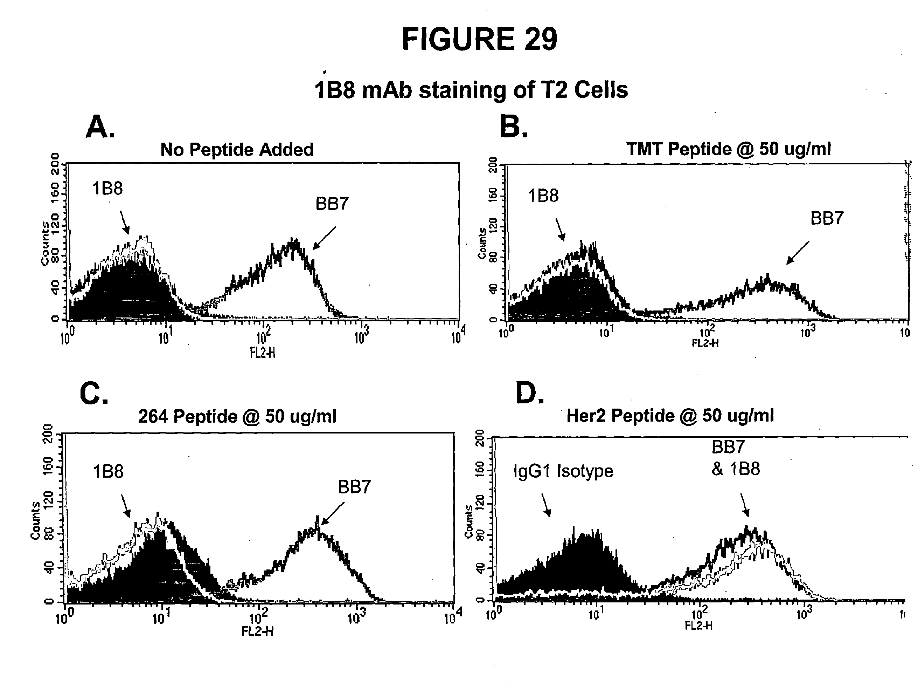

Figure 29 illustrates 1 B8 mAb staining of T2 cells. T2 cells (HLA-A2⁺, TAP deficient) were stained with cell supernatant from hybridoma 1B8 (immunogen = Her-2/neu tetramers) to demonstrate binding specificity for this monoclonal antibody for the Her2/neu-peptide-HLA-A2 complex. 5x10⁵ T2 cells were incubated in 100 µl of buffer containing 100 µg of Her-2/neu, 264, or TMT peptide for 6 hours at 37°C, washed and stained with 2 µl of 1 B8 culture supernatant for 20 min. In addition, cells that were not peptide pulsed were stained in an identical manner with 1B8 to determine background staining. In addition, cells were also stained with 0.5 µg of BB7.2 antibody to detect the level of HLA-A2 on the T2 cells and to determine the effectiveness of peptide loading by staining for HLA-A2 levels 6 hours after pulsing with peptide. After staining the reactions were washed once with 3-4 ml wash buffer and resuspended in approximately 100 µl of wash buffer containing 0.5 µg of PE-conjugated goat anti-mouse IgG (Caltag, Burlingame, CA). Cells were washed as above and resuspended in 0.5 ml wash buffer for analysis. As shown in Figure 29, samples were collected on a FACscan (BD biosciences, San Diego, California) and analyzed using Cell Quest software (version 3.3, BD Biosciences). Panel A shows no binding by 1 B8 TCR mimic to T2 cells without exogenous peptide. Panels B and C show no binding with 1B8 to T2 cells pulsed with either 264 or TMT peptide. Panel D shows specific binding of the 1 B8 TCR mimic to T2 cells loaded with the Her-2/neu peptide as indicated by the strong rightward shift of the open peak. In addition, all cells were stained with the BB7.2 antibody with a greater than 2-fold shift in staining intensity seen with peptide loaded T2 cells (see Panels B thru D). In all Panels the IgG1 and IgG2b isotype controls did not stain T2 cells (see filled peaks in all Panels). Collectively, these data demonstrate specific binding of the 1B8 monoclonal antibody for Her-2/neu peptide-pulsed T2 cells.

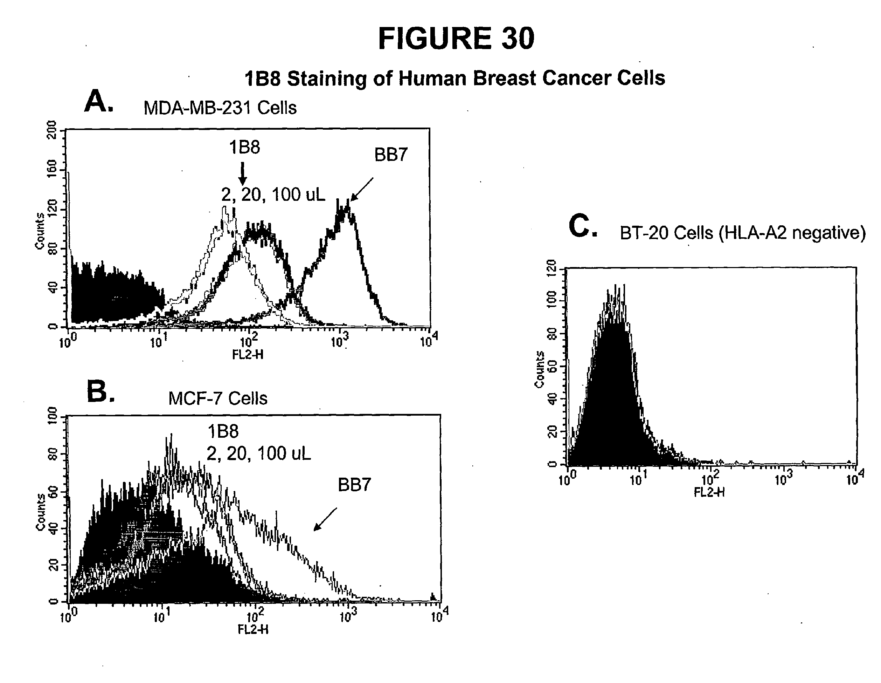

Figure 30 illustrates 1B8 staining of MDA-MB-231 and MCF-7 human breast carcinoma cells. MDA-MB-231 cells (HLA-A2⁺) were stained with cell supernatant from hybridoma 1B8 (immunogen = Her-2/neu tetramers) to demonstrate that the antibody specifically recognizes endogenous Her-2/neu peptide-HLA-A2 complex on the cell surface. 5x10⁵ MDA-MB-231 cells were incubated in 100 µl of buffer containing 2, 20, or 100 µl of 1 B8 culture supernatant for 15 minutes at room temperature. After staining the reactions were washed once with 3-4 ml wash buffer and resuspended in approximately 100 µl of wash buffer containing 0.5µg of PE-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). Figure 30 demonstrates a 1B8 titration effect for binding to endogenous Her-2/neu peptide-HLA-A2 complexes on (A) MDA-231 and (B) MCF-7 human breast cancer cells. In addition, both cell lines stained positive for HLA-A2 using 0.5 µg BB7.2 antibody followed by detection with 0. 5 µg of goat anti-mouse-PE conjugate. In Figure 30C, neither 1B8 nor BB7.2 antibodies could stain the HLA-A2 negative human breast cancer cell line, BT-20. These data indicate the 1 B8 TCR mimic binding is specific for Her-2/neu peptide (369-377)-HLA-A2 and that the 1 B8 can detect this epitope on the surface of human breast cancer cells.

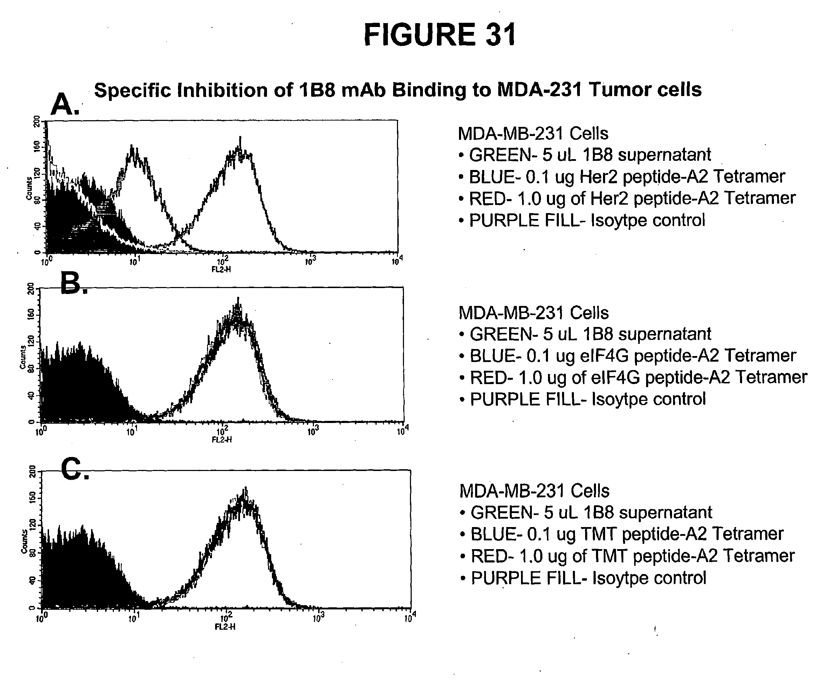

Figure 31 illustrates the specific inhibition of 1 B8 mAb binding to MDA-231 tumor cells. MDA-MB-231 cells (HLA-A2+) were stained with cell supernatant from hybridoma 1 B8 (immunogen = Her-2/neu tetramers) in the presence of (1) tetramer complex that would compete with specific binding to Her2/neu-HLA-A2; (2) tetramer complex that would not compete with specific binding to Her2/neu-HLA-A2 (264p and eIF4Gp); or (3) no tetramer, to demonstrate that the antibody specifically recognizes endogenous Her-2/neu peptide-HLA-A2 complex on the cell surface. 5x10⁵ MDA-MB-231 cells were incubated in 100 µl of buffer containing 100 µl of 1B8 culture supernatant alone or in the presence of 0.1 or 1.0 µg of Her-2/neu-HLA-A2 tetramer (competitor), 264p-HLA-A2 tetramer or eIF4Gp-HLA-A2 (non competitors) or without tetramer addition for 15 minutes at room temperature. After staining the reactions were washed once with 3-4 ml wash buffer and resuspended in approximately 100 µl of wash buffer containing 0.5 µg of PE-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). Figure 31 A demonstrates 1 B8 binding specificity for endogenous Her-2/neu peptide-HLA-A2 complexes on MDA-231 tumor cells. Binding of the 1B8 TCR mimic to MDA-MB-231 cells is significantly reduced in a dose-dependent manner (see leftward shift with peak) in the presence of competitor (Her-2/neu-HLA-A2 monomer). In panels B and C, it is shown that 1 B8 binding is not blocked when non-relevant (264 and Her-2/neu) peptide-HLA-A2 monomers are used to compete with 1 B8 binding to MDA-231 cells. These findings support previous binding specificity data and indicate Her-2/neu-HLA-A2 as a prevalent epitope on breast cancer cells.

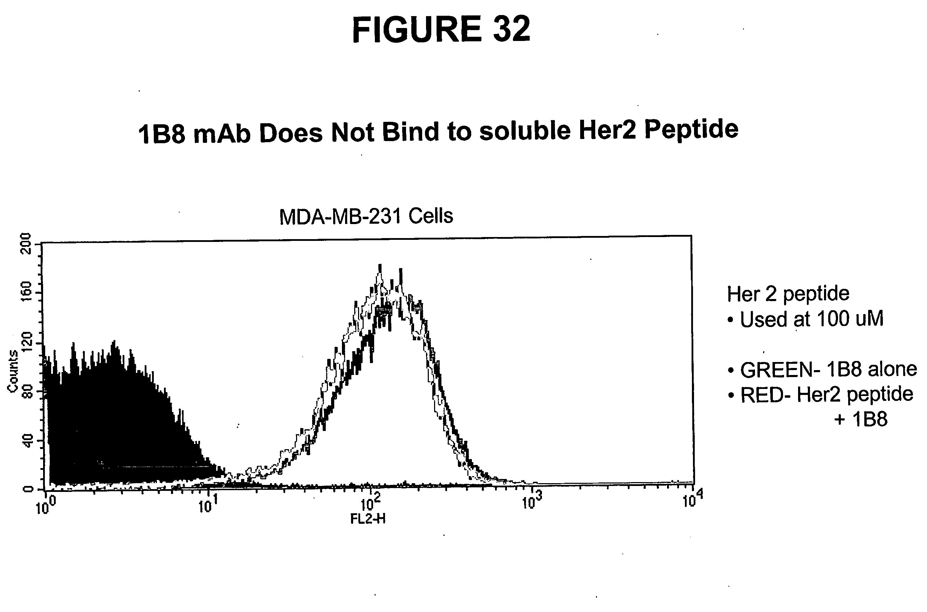

Figure 32 illustrates that 1 B8 mAb does not bind to soluble Her2/neu peptide. MDA-MB-231 cells (HLA-A2⁺) were stained with cell supernatant from hybridoma 1B8 (immunogen = Her-2/neu tetramers) in the presence or absence of 100 µM of exogenously added Her-2/neu peptide. 5x10⁵ MDA-MB-231 cells were incubated in 100 µl of buffer containing 100µl of 1 B8 culture supernatant for 15 minutes at room temperature. After staining the reactions were washed once with 3-4 ml wash buffer and resuspended in approximately 100 µl of wash buffer containing 0.5 µg of PE-conjugated goat anti-mouse IgG (Caltag, Burlingame, CA). Cells were washed as above and resuspended in 0.5 ml wash buffer for analysis. Samples were collected on a FACscan (BD biosciences, San Diego, California) and analyzed using Cell Quest software (version 3.3, BD Biosciences). Figure 32 demonstrates that 1B8 TCR mimic has dual specificity and does not bind to Her-2/neu peptide alone.

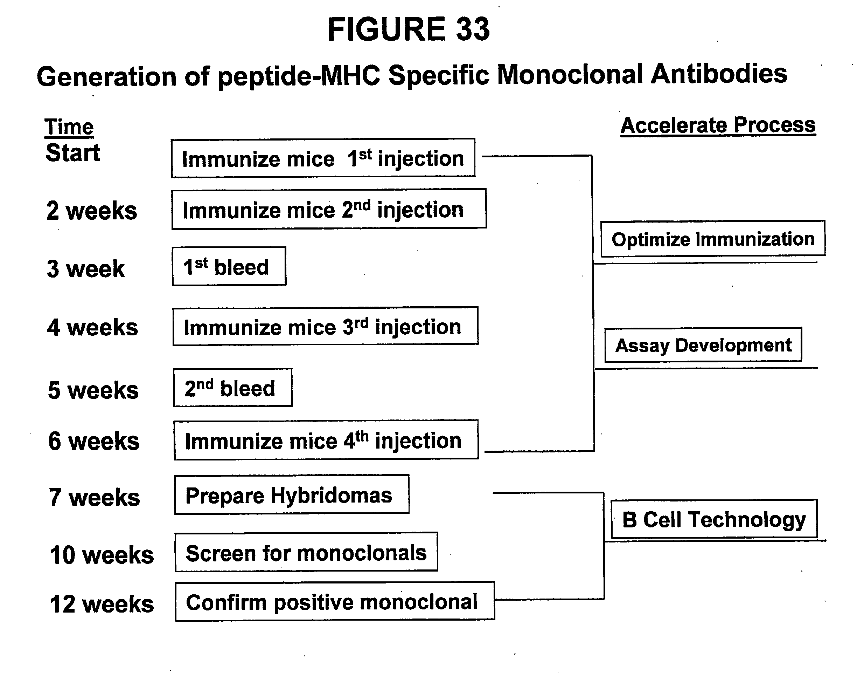

Figure 33 illustrates a protocol for the generation of peptide-MHC Class I specific TCR mimics of the present invention.

### DETAILED DESCRIPTION OF THE INVENTION

Before explaining at least one embodiment of the invention in detail by way of exemplary drawings, experimentation, results, and laboratory procedures, it is to be understood that the invention is not limited in its application to the details of construction and the arrangement of the components set forth in the following description or illustrated in the drawings, experimentation and/or results. The invention is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (2nd ed., Cold Spring Harbor Laboratory Press, Cold Spring Harbor, N.Y. (1989) and Coligan et al. Current Protocols in Immunology (Current Protocols, Wiley Interscience (1994)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The terms "isolated polynucleotide" and "isolated nucleic acid segment" as used herein shall mean a polynucleotide of genomic, cDNA, or synthetic origin or some combination thereof, which by virtue of its origin the "isolated polynucleotide" or "isolated nucleic acid segment" (1) is not associated with all or a portion of a polynucleotide in which the "isolated polynucleotide" or "isolated nucleic acid segment" is found in nature, (2) is operably linked to a polynucleotide which it is not linked to in nature, or (3) does not occur in nature as part of a larger sequence.

The term "isolated protein" referred to herein means a protein of cDNA, recombinant RNA, or synthetic origin or some combination thereof, which by virtue of its origin, or source of derivation, the "isolated protein" (1) is not associated with proteins found in nature, (2) is free of other proteins from the same source, e.g., free of murine proteins, (3) is expressed by a cell from a different species, or, (4) does not occur in nature.

The term "polypeptide" as used herein is a generic term to refer to native protein, fragments, or analogs of a polypeptide sequence. Hence, native protein, fragments, and analogs are species of the polypeptide genus.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "operably linked" as used herein refers to positions of components so described are in a relationship permitting them to function in their intended manner. A control sequence "operably linked" to a coding sequence is ligated in such a way that expression of the coding sequence is achieved under conditions compatible with the control sequences.

The term "control sequence" as used herein refers to polynucleotide sequences which are necessary to effect the expression and processing of coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is essential for expression and processing, and can also include additional components whose presence is advantageous, for example, leader sequences and fusion partner sequences.

The term "polynucleotide" as referred to herein means a polymeric form of nucleotides of at least 10 bases in length, either ribonucleotides or deoxynucleotides or a modified form of either type of nucleotide. The term includes single and double stranded forms of DNA.

The term "oligonucleotide" referred to herein includes naturally occurring, and modified nucleotides linked together by naturally occurring, and non-naturally occurring oligonucleotide linkages. Oligonucleotides are a polynucleotide subset generally comprising a length of 200 bases or fewer. Preferably oligonucleotides are 10 to 60 bases in length and most preferably 12, 13, 14, 15, 16, 17, 18, 19, or 20 to 40 bases in length. Oligonucleotides are usually single stranded, e.g., for probes; although oligonucleotides may be double stranded, e.g., for use in the construction of a gene mutant. Oligonucleotides of the invention can be either sense or antisense oligonucleotides.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phosphoroselenoate, phosphorodiselenoate, phosphoroanilothioate, phoshoraniladate, phosphoroamidate, and the like. See e.g., LaPlanche et al. Nucl. Acids Res. 14:9081 (1986); Stec et al. J. Am. Chem. Soc. 106:6077 (1984); Stein et al. Nucl. Acids Res. 16:3209 (1988); Zon et al. Anti-Cancer Drug Design 6:539 (1991); Zon et al. Oligonucleotides and Analogues: A Practical Approach, pp. 87-108 (F. Eckstein, Ed., Oxford University Press, Oxford England (1991)); Stec et al. U.S. Pat. No. 5,151,510; Uhlmann and Peyman Chemical Reviews 90:543 (1990), An oligonucleotide can include a label for detection, if desired.

The term "selectively hybridize" referred to herein means to detectably and specifically bind. Polynucleotides, oligonucleotides and fragments thereof in accordance with the invention selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments of the invention and a nucleic acid sequence of interest will be at least 80%, and more typically with preferably increasing homologies of at least 85%, 90%, 95%, 99%, and 100%. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. See Dayhoff, M. O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program. The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotide or amino acid sequences: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence. Generally, a reference sequence is at least 18 nucleotides or 6 amino acids in length, frequently at least 24 nucleotides or 8 amino acids in length, and often at least 48 nucleotides or 16 amino acids in length. Since two polynucleotides or amino acid sequences may each (1) comprise a sequence (i.e., a portion of the complete polynucleotide or amino acid sequence) that is similar between the two molecules, and (2) may further comprise a sequence that is divergent between the two polynucleotides or amino acid sequences, sequence comparisons between two (or more) molecules are typically performed by comparing sequences of the two molecules over a "comparison window" to identify and compare local regions of sequence similarity. A "comparison window", as used herein, refers to a conceptual segment of at least 18 contiguous nucleotide positions or 6 amino acids wherein a polynucleotide sequence or amino acid sequence may be compared to a reference sequence of at least 18 contiguous nucleotides or 6 amino acid sequences and wherein the portion of the polynucleotide sequence in the comparison window may comprise additions, deletions, substitutions, and the like (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman Adv. Appl. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85:2444 (1988), by computerized implementations of these algorithms (GAP, BESTFIT, FASTA, and TFASTA in the Wisconsin Genetics Software Package Release 7.0, (Genetics Computer Group, 575 Science Dr., Madison, Wis.), Geneworks, or MacVector software packages), or by inspection, and the best alignment (i.e., resulting in the highest percentage of homology over the comparison window) generated by the various methods is selected.

The term "sequence identity" means that two polynucleotide or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. See Immunology-A Synthesis (2nd Edition, E. S. Golub and D. R. Gren, Eds., Sinauer Associates, Sunderland, Mass. (1991)), by Stereoisomers (e.g., D-amino acids) of the twenty conventional amino acids, unnatural amino acids such as α-,α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the lefthand direction is the amino terminal direction and the righthand direction is the carboxy-terminal direction, in accordance with standard usage and convention.

Similarly, unless specified otherwise, the lefthand end of single-stranded polynucleotide sequences is the 5' end; the lefthand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity, and most preferably at least 99 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

As discussed herein, minor variations in the amino acid sequences of antibodies or immunoglobulin molecules are contemplated as being encompassed by the present invention, providing that the variations in the amino acid sequence maintain at least 75%, more preferably at least 80%, 90%, 95%, and most preferably 99%. In particular, conservative amino acid replacements are contemplated. Conservative replacements are those that take place within a family of amino acids that are related in their side chains. Genetically encoded amino acids are generally divided into families: (1) acidic=aspartate, glutamate; (2) basic=lysine, arginine, histidine; (3) nonpolar=alanine, valine, leucine, isoleucine, proline, phenylalanine, methionine, tryptophan; and (4) uncharged polar=glycine, asparagine, glutamine, cysteine, serine, threonine, tyrosine. More preferred families are: serine and threonine are aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid within a framework site. Whether an amino acid change results in a functional peptide can readily be determined by assaying the specific activity of the polypeptide derivative. Fragments or analogs of antibodies or immunoglobulin molecules can be readily prepared by those of ordinary skill in the art. Preferred amino- and carboxy-termini of fragments or analogs occur near boundaries of functional domains. Structural and functional domains can be identified by comparison of the nucleotide and/or amino acid sequence data to public or proprietary sequence databases. Preferably, computerized comparison methods are used to identify sequence motifs or predicted protein conformation domains that occur in other proteins of known structure and/or function. Methods to identify protein sequences that fold into a known three-dimensional structure are known. Bowie et al. Science 253:164 (1991). Thus, the foregoing examples demonstrate that those of skill in the art can recognize sequence motifs and structural conformations that may be used to define structural and functional domains in accordance with the invention.

Preferred amino acid substitutions are those which: (1) reduce susceptibility to proteolysis, (2) reduce susceptibility to oxidation, (3) alter binding affinity for forming protein complexes, (4) alter binding affinities, and (5) confer or modify other physicochemical or functional properties of such analogs. Analogs can include various mutations of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts. A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure ©. Branden and J. Tooze, eds., Garland Publishing, New York, N.Y. (1991)); and Thornton et at. Nature 354:105 (1991).

The term "polypeptide fragment" as used herein refers to a polypeptide that has an amino-terminal and/or carboxy-terminal deletion, but where the remaining amino acid sequence is identical to the corresponding positions in the naturally-occurring sequence deduced, for example, from a full-length cDNA sequence. Fragments typically are at least 5, 6, 8 or 10 amino acids long, preferably at least 14 amino acids long, more preferably at least 20 amino acids long, usually at least 50 amino acids long, and even more preferably at least 70 amino acids long.

"Antibody" or "antibody peptide(s)" refer to an intact antibody, or a binding fragment thereof that competes with the intact antibody for specific binding. Binding fragments are produced by recombinant DNA techniques, or by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody substantially inhibits adhesion of a receptor to a counterreceptor when an excess of antibody reduces the quantity of receptor bound to counterreceptor by at least about 20%, 40%, 60% or 80%, and more usually greater than about 85% (as measured in an in vitro competitive binding assay).

The term "MHC as used herein will be understood to refer to the Major Histocompability Complex, which is defined as a set of gene loci specifying major histocompatibility antigens. The term "HLA" as used herein will be understood to refer to Human Leukocyte Antigens, which is defined as the histocompatibility antigens found in humans. As used herein, "HLA" is the human form of "MHC".

The terms "MHC light chain" and "MHC heavy chain" as used herein will be understood to refer to portions of the MHC molecule. Structurally, class I molecules are heterodimers comprised of two noncovalently bound polypeptide chains, a larger "heavy" chain (α) and a smaller "light" chain (β-2-microglobulin or β2m). The polymorphic, polygenic heavy chain (45 kDa), encoded within the MHC on chromosome six, is subdivided into three extracellular domains (designated 1, 2, and 3), one intracellular domain, and one transmembrane domain. The two outermost extracellular domains, 1 and 2, together form the groove that binds antigenic peptide. Thus, interaction with the TCR occurs at this region of the protein. The 3 domain of the molecule contains the recognition site for the CD8 protein on the CTL; this interaction serves to stabilize the contact between the T cell and the APC. The invariant light chain (12 kDa), encoded outside the MHC on chromosome 15, consists of a single, extracellular polypeptide. The terms "MHC light chain", "β-2-microglobulin", and "β2m" may be used interchangeably herein.

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is <1 µM, preferably <100 nM and most preferably <10 nM.

The term "antibody" is used in the broadest sense, and specifically covers monoclonal antibodies (including full length monoclonal antibodies), polyclonal antibodies, multispecific antibodies (e.g., bispecific antibodies), and antibody fragments (e.g., Fab, F(ab')₂ and Fv) so long as they exhibit the desired biological activity. Antibodies (Abs) and immunoglobulins (lgs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond. While the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light chain variable domain is aligned with the variable domain of the heavy chain. Particular amino acid residues are believed to form an interface between the light and heavy chain variable domains (Clothia et al., J. Mol. Biol. 186, 651-66, 1985); Novotny and Haber, Proc. Natl. Acad. Sci. USA 82 4592-4596 (1985).

An "isolated" antibody is one which has been identified and separated and/or recovered from a component of the environment in which is was produced. Contaminant components of its production environment are materials which would interfere with diagnostic or therapeutic uses for the antibody, and may include enzymes, hormones, and other proteinaceous or nonproteinaceous solutes. In preferred embodiments, the antibody will be purified as measurable by at least three different methods: 1) to greater than 50% by weight of antibody as determined by the Lowry method, and more preferably more than 75% by weight, and more preferably more than 85% by weight, and more preferably more than 95% by weight, and most preferably more than 99% by weight; 2) to a degree sufficient to obtain at least 10 residues of N-terminal or internal amino acid sequence by use of a spinning cup sequentator, and more preferably at least 15 residues of sequence; or 3) to homogeneity by SDS-PAGE under reducing or non-reducing conditions using Coomasie blue or, preferably, silver stain. Isolated antibody includes the antibody in situ within recombinant cells since at least one component of the antibody's natural environment will not be present. Ordinarily, however, isolated antibody will be prepared by at least one purification step.

The term "antibody mutant" refers to an amino acid sequence variant of an antibody wherein one or more of the amino acid residues have been modified. Such mutants necessarily have less than 100% sequence identity or similarity with the amino acid sequence having at least 75% amino acid sequence identity or similarity with the amino acid sequence of either the heavy or light chain variable domain of the antibody, more preferably at least 80%, more preferably at least 85%, more preferably at least 90%, and most preferably at least 95%.

The term "variable" in the context of variable domain of antibodies, refers to the fact that certain portions of the variable domains differ extensively in sequence among antibodies and are used in the binding and specificity of each particular antibody for its particular antigen. However, the variability is not evenly distributed through the variable domains of antibodies. It is concentrated in three segments called complementarity determining regions (CDRs) also known as hypervariable regions both in the light chain and the heavy chain variable domains. There are at least two techniques for determining CDRs: (1) an approach based on cross-species sequence variability (i.e., Kabat et al., Sequences of Proteins of Immunological Interest (National Institute of Health, Bethesda, Md. 1987); and (2) an approach based on crystallographic studies of antigen-antibody complexes (Chothia, C. et al. (1989), Nature 342: 877). The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies (see Kabat et al.) The constant domains are not involved directly in binding an antibody to an antigen, but exhibit various effector function, such as participation of the antibody in antibody-dependent cellular toxicity.

The term "antibody fragment" refers to a portion of a full-length antibody, generally the antigen binding or variable region. Examples of antibody fragments include Fab, Fab', F(ab')₂ and Fv fragments. Papain digestion of antibodies produces two identical antigen binding fragments, called the Fab fragment, each with a single antigen binding site, and a residual "Fc" fragment, so-called for its ability to crystallize readily. Pepsin treatment yields an F(ab')₂ fragment that has two antigen binding fragments which are capable of cross-linking antigen, and a residual other fragment (which is termed pFc'). As used herein, "functional fragment" with respect to antibodies, refers to Fv, F(ab) and F(ab')₂ fragments.

An "Fv" fragment is the minimum antibody fragment which contains a complete antigen recognition and binding site. This region consists of a dimer of one heavy and one light chain variable domain in a tight, non-covalent association (V_{H} -V_{L} dimer). It is in this configuration that the three CDRs of each variable domain interact to define an antigen binding site on the surface of the V_{H} -V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

The Fab fragment [also designated as F(ab)] also contains the constant domain of the light chain and the first constant domain (CH1) of the heavy chain. Fab' fragments differ from Fab fragments by the addition of a few residues at the carboxyl terminus of the heavy chain CH1 domain including one or more cysteines from the antibody hinge region. Fab'-SH is the designation herein for Fab' in which the cysteine residue(s) of the constant domains have a free thiol group. F(ab') fragments are produced by cleavage of the disulfide bond at the hinge cysteines of the F(ab')₂ pepsin digestion product. Additional chemical couplings of antibody fragments are known to those of ordinary skill in the art.

The light chains of antibodies (immunoglobulin) from any vertebrate species can be assigned to one of two clearly distinct types, called kappa (.kappa.) and lambda (.lambda.), based on the amino sequences of their constant domain.

Depending on the amino acid sequences of the constant domain of their heavy chains, "immunoglobulins" can be assigned to different classes. There are at least five (5) major classes of immunoglobulins: IgA, IgD, IgE, IgG and IgM, and several of these may be further divided into subclasses (isotypes), e.g., IgG-1, IgG-2, IgG-3 and IgG4; IgA-1 and IgA-2. The heavy chains constant domains that correspond to the different classes of immunoglobulins are called α, Δ, ε, Y and µ, respectively. The subunit structures and three-dimensional configurations of different classes of immunoglobulins are well known.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In additional to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, Nature 256, 495 (1975), or may be made by recombinant methods, e.g., as described in U.S. Pat. No. 4,816,567. The monoclonal antibodies for use with the present invention may also be isolated from phage antibody libraries using the techniques described in Clackson et al. Nature 352: 624-628 (1991), as well as in Marks et al., J. Mol. Biol. 222: 581-597 (1991).

Utilization of the monoclonal antibodies of the present invention may require administration of such or similar monoclonal antibody to a subject, such as a human. However, when the monoclonal antibodies are produced in a non-human animal, such as a rodent, administration of such antibodies to a human patient will normally elicit an immune response, wherein the immune response is directed towards the antibodies themselves. Such reactions limit the duration and effectiveness of such a therapy. In order to overcome such problem, the monoclonal antibodies of the present invention can be "humanized", that is, the antibodies are engineered such that antigenic portions thereof are removed and like portions of a human antibody are substituted therefor, while the antibodies' affinity for specific peptide/MHC complexes is retained. This engineering may only involve a few amino acids, or may include entire framework regions of the antibody, leaving only the complementarity determining regions of the antibody intact. Several methods of humanizing antibodies are known in the art and are disclosed in US Patent Nos. 6,180,370, issued to Queen et al on January 30, 2001; 6,054,927, issued to Brickell on April 25, 2000; 5,869,619, issued to Studnicka on February 9, 1999; 5,861,155, issued to Lin on January 19, 1999; 5,712,120, issued to Rodriquez et al on January 27, 1998; and 4,816,567, issued to Cabilly et al on March 28, 1989

Humanized forms of antibodies are chimeric immunoglobulins, immunoglobulin chains or fragments thereof (such as Fv, Fab, Fab', F(ab')2 or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., 1986; Riechmann et al., 1988; Verhoeyen et al., 1988), by substituting rodent CDRs or CDR sequences for the corresponding sequences of a human antibody. (See also U.S. Patent No.5,225,539.) In some instances, Fᵥ framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, 1992).

97 published articles relating to the generation or use of humanized antibodies were identified by a PubMed search of the database as of April 25, 2002. Many of these studies teach useful examples of protocols that can be utilized with the present invention, such as Sandbom et al., Gatroenterology,120:1330 (2001); Mihara et al., Clin. Immunol. 98:319 (2001); Yenari et al., Neurol. Res. 23:72 (2001); Morales et al., Nucl. Med. Biol. 27:199 (2000); Richards et al., Cancer Res. 59:2096 (1999); Yenari et al., Exp. Neurol. 153:223 (1998); and Shinkura et al., Anticancer Res. 18:1217 (1998) For example, a treatment protocol that can be utilized in such a method includes a single dose, generally administered intravenously, of 10-20 mg of humanized mAb per kg (Sandborn, et al. 2001). In some cases, alternative dosing patterns may be appropriate, such as the use of three infusions, administered once every two weeks, of 800 to 1600 mg or even higher amounts of humanized mAb (Richards et al., 1999). However, it is to be understood that the invention is not limited to the treatment protocols described above, and other treatment protocols which are known to a person of ordinary skill in the art may be utilized in the methods of the present invention.

The presently disclosed and claimed invention further includes fully human monoclonal antibodies against specific peptide/MHC complexes. Fully human antibodies essentially relate to antibody molecules in which the entire sequence of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., Hybridoma, 2:7 (1983)) and the EBV hybridoma technique to produce human monoclonal antibodies (see Cole, et al., PNAS 82:859 (1985)). Human monoclonal antibodies may be utilized in the practice of the present invention and may be produced by using human hybridomas (see Cote, et al., PNAS 80:2026 (1983)) or by transforming human B-cells with Epstein Barr Virus in vitro (see Cole, et al., 1985).

In addition, human antibodies can also be produced using additional techniques, including phage display libraries (Hoogenboom et al., NucleicAcids Res.19:4133 (1991); Marks et al., J Mol Biol. 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in Marks et al., J Biol. Chem. 267:16007 (1992); Lonberg et al., Nature, 368:856 (1994); Morrison, 1994; Fishwild et al., Nature Biotechnol. 14:845 (1996); Neuberger, Nat. Biotechnol. 14:826 (1996); and Lonberg and Huszar, Int Rev Immunol. 13:65 (1995).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the XENOMOUSE™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Pat. No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Pat. No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

The term "agent" is used herein to denote a chemical compound, a mixture of chemical compounds, a biological macromolecule, or an extract made from biological materials.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or calorimetric methods). In certain situations, the label or marker can also be therapeutic. Various methods of labeling polypeptides and glycoproteins are known in the art and may be used. Examples of labels for polypeptides include, but are not limited to, the following: radioisotopes or radionuclides (e_{.}g_{.}, ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, β-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985))

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

The term patient includes human and veterinary subjects.

A "liposome" is a small vesicle composed of various types of lipids, phospholipids and/or surfactant. The components of the liposome are commonly arranged in a bilayer formation, similar to the lipid arrangement of biological membranes.

"Treatment" refers to both therapeutic treatment and prophylactic or preventative measures. Those in need of treatment include those already with the disorder as well as those in which the disorder is to be prevented.

A "disorder" is any condition that would benefit from treatment with the polypeptide. This includes chronic and acute disorders or diseases including those pathological conditions which predispose the mammal to the disorder in question.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastrointestinal cancer, pancreatic cancer, glioblastoma, cervical cancer, ovarian cancer, liver cancer, bladder cancer, hopatoma, breast cancer, colon cancer, colorectal cancer, endometrial carcinoma, salivary gland carcinoma, kidney cancer, renal cancer, prostate cancer, vulval cancer, thyroid cancer, hepatic carcinoma and various types of head and neck cancer.

"Mammal" for purposes of treatment refers to any animal classified as a mammal, including human, domestic and farm animals, nonhuman primates, and zoo, sports, or pet animals, such as dogs, horses, cats, cows, etc.

As mentioned hereinabove, depending on the application and purpose, the T cell receptor mimic of the presently disclosed and claimed invention may be attached to any of various functional moieties. A T cell receptor mimic of the present invention attached to a functional moiety may be referred to herein as an "immunoconjugate". Preferably, the functional moiety is a detectable moiety or a therapeutic moiety.

As is described and demonstrated in further detail hereinbelow, a detectable moiety or a therapeutic moiety may be particularly advantageously employed in applications of the present invention involving use of the T cell receptor mimic to detect the specific peptide/MHC complex, or to kill target cells and/or damage target tissues.

The present invention include the T cell receptor mimics described herein attached to any of numerous types of detectable moieties, depending on the application and purpose. For applications involving detection of the specific peptide/MHC complex, the detectable moiety attached to the T cell receptor mimic is preferably a reporter moiety that enables specific detection of the specific peptide/MHC complex bound by the T cell receptor mimic of the presently disclosed and claimed invention.

While various types of reporter moieties may be utilized to detect the specific peptide/MHC complex, depending on the application and purpose, the reporter moiety is preferably a fluorophore, an enzyme or a radioisotope. Specific reporter moieties that may utilized in accordance with the present invention include, but are not limited to, green fluorescent protein (GFP), alkaline phosphatase (AP), peroxidase, orange fluorescent protein (OFP), β-galactosidase, fluorescein isothiocyanate (FITC), phycoerythrin, Cy-chrome, rhodamine, blue fluorescent protein (BFP), Texas red, horseradish peroxidase (HPR), and the like.

A fluorophore may be advantageously employed as a detection moiety enabling detection of the specific peptide/MHC complex via any of numerous fluorescence detection methods. Depending on the application and purpose, such fluorescence detection methods include, but are not limited to, fluorescence activated flow cytometry (FACS), immunofluorescence confocal microscopy, fluorescence in-situ hybridization (FISH), fluorescence resonance energy transfer (FRET), and the like.

Various types of fluorophores, depending on the application and purpose, may be employed to detect the specific peptide/MHC complex. Examples of suitable fluorophores include, but are not limited to, phycoerythrin, fluorescein isothiocyanate (FITC), Cy-chrome, rhodamine, green fluorescent protein (GFP), blue fluorescent protein (BFP), Texas red, and the like.

Ample guidance regarding fluorophore selection, methods of linking fluorophores to various types of molecules, such as a T cell receptor mimic of the present invention, and methods of using such conjugates to detect molecules which are capable of being specifically bound by antibodies or antibody fragments comprised in such immunoconjugates is available in the literature of the art [for example, refer to: Richard P. Haugland, "Molecular Probes: Handbook of Fluorescent Probes and Research Chemicals 1992-1994", 5th ed., Molecular Probes, Inc. (1994); U.S. Pat. No. 6,037,137 to Oncoimmunin Inc.; Hermanson, "Bioconjugate Techniques", Academic Press New York, N.Y. (1995); Kay M. et al.,1995. Biochemistry 34:293; Stubbs et al., 1996. Biochemistry 35:937; Gakamsky D. et al., "Evaluating Receptor Stoichiometry by Fluorescence Resonance Energy Transfer," in "Receptors: A Practical Approach," 2nd ed., Stanford C. and Horton R. (eds.), Oxford University Press, UK. (2001); U.S. Pat. No. 6,350,466 to Targesome, Inc.]. Therefore, no further description is considered necessary.

Alternately, an enzyme may be advantageously utilized as the detectable moiety to enable detection of the specific peptide/MHC complex via any of various enzyme-based detection methods. Examples of such methods include, but are not limited to, enzyme linked immunosorbent assay (ELISA; for example, to detect the specific peptide/MHC complex in a solution), enzyme-linked chemiluminescence assay (for example, to detect the complex on solubilized cells), and enzyme-linked immunohistochemical assay (for example, to detect the complex in a fixed tissue).

Numerous types of enzymes may be employed to detect the specific peptide/MHC complex, depending on the application and purpose. Examples of suitable enzymes include, but are not limited to, horseradish peroxidase (HPR), β-galactosidase, and alkaline phosphatase (AP). Ample guidance for practicing such enzyme-based detection methods is provided in the literature of the art (for example, refer to: Khatkhatay M I. and Desai M., 1999. J Immunoassay 20:151-83; Wisdom G B., 1994. Methods Mol Biol. 32:433-40; Ishikawa E. et al., 1983. J Immunoassay 4:209-327; Oellerich M., 1980. J Clin Chem Clin Biochem. 18:197-208; Schuurs A H. and van Weemen B K., 1980. J Immunoassay 1:229-49).

The present invention include the T cell receptor mimics described herein attached to any of numerous types of therapeutic moieties, depending on the application and purpose. Various types of therapeutic moieties that may be utilized in accordance with the present invention include, but are not limited to, a cytotoxic moiety, a toxic moiety, a cytokine moiety, a bi-specific antibody moiety, and the like. Specific examples of therapeutic moieties that may be utilized in accordance with the present invention include, but are not limited to, *Pseudomonas* exotoxin, Diptheria toxin, interleukin 2, CD3, CD16, interleukin 4, interleukin 10, Ricin A toxin, and the like.

The functional moiety may be attached to the T cell receptor mimic of the present invention in various ways, depending on the context, application and purpose. A polypeptidic functional moiety, in particular a polypeptidic toxin, may be advantageously attached to the antibody or antibody fragment via standard recombinant techniques broadly practiced in the art (for Example, refer to Sambrook et al., infra, and associated references, listed in the Examples section which follows). A functional moiety may also be attached to the T cell receptor mimic of the presently disclosed and claimed invention using standard chemical synthesis techniques widely practiced in the art [for example, refer to the extensive guidelines provided by The American Chemical Society (for example at: http://www.chemistry.org/portal/Chemistry)]. One of ordinary skill in the art, such as a chemist, will possess the required expertise for suitably practicing such such chemical synthesis techniques.

Alternatively, a functional moiety may be attached to the T cell receptor mimic by attaching an affinity tag-coupled T cell receptor mimic of the present invention to the functional moiety conjugated to a specific ligand of the affinity tag. Various types of affinity tags may be employed to attach the T cell receptor mimic to the functional moiety. In one embodiment, the affinity tag is a biotin molecule or a streptavidin molecule. A biotin or streptavidin affinity tag can be used to optimally enable attachment of a streptavidin-conjugated or a biotin-conjugated functional moiety, respectively, to the T cell receptor mimic due to the capability of streptavidin and biotin to bind to each other with the highest non covalent binding affinity known to man (i.e., with a Kd of about 10⁻¹⁴ to 10⁻¹⁵).

A pharmaceutical composition of the present invention includes a T cell receptor mimic of the present invention and a therapeutic moiety conjugated thereto. The pharmaceutical composition of the present invention may be an antineoplastic agent. A diagnostic composition of the present invention includes a T cell receptor mimic of the present invention and a detectable moiety conjugated thereto.

The present invention relates to methodologies for producing antibodies that function as T-cell receptor mimics (TCRₘs) and recognize peptides displayed in the context of HLA molecules, wherein the peptide is associated with a tumorigenic, infectious or disease state. These antibodies will mimic the specificity of a T cell receptor (TCR) such that the molecules may be used as therapeutic and diagnostic reagents. In one embodiment, the T cell receptor mimics of the presently disclosed and claimed invention will have a higher binding affinity than a T cell receptor. In a preferred embodiment, the T cell receptor mimic produced by the method of the presently disclosed and claimed invention has a binding affinity of about 10 nanomolar or greater.

The methods of the presently claimed and disclosed invention begin with the production of an immunogen. The immunogen comprises a peptide/MHC complex, wherein the 3-dimensional presentation of the peptide in the binding groove is the epitope recognized with high specificity by the antibody. The immunogen may be any form of a stable peptide/MHC complex that may be utilized for immunization of a host capable of producing antibodies to the immunogen, and the immunogen may be produced by any methods known to those skilled in the art. The immunogen is used in the construction of an agent that will activate a clinically relevant cellular immune response against the tumor cell which displays the particular peptide/MHC complex.

The peptide epitopes of the peptide/MHC complex of the immunogen are antigens that have been discovered as being novel to cancer cells, and such peptide epitopes are present one the surface of cells associated with a tumorigenic, infectious or disease state such as cancer cells and displayed in the context of MHC molecules.

The immunogen may be produced in a manner so that it is table, or it may be modified by various means to make it more stable.

According to the present invention, the immunogen is produced by a cell-based approach through genetic engineering and recombinant expression, thus significantly increasing the half-life of the complex. The genetically-engineered and recombinantly expressed peptide class 1 MHC complex may be genetically engineered such that the complex is produced in the form of a single-chain trimer. In this method, the MHC heavy chain, β-2 microglobulin and peptide are all produced as a single-chain trimer that is linked together. Methods of producing single-chain trimers are known in the art and are disclosed particularly in Yu et al. (2002). Other methods involve forming a single-chain dimmer in which the peptide-β2m molecules are linked together, and in the single-chain dimmer, the β2m molecule may or may not be membrane bound.

In a second embodiment, the immunogen of the presently claimed and disclosed invention is produced by multimerizing two or more peptide/MHC complexes. The term "multimer" as used herein will be understood to include two or more copies of the peptide/MHC complex which are covalently or non-covalently attached together, either directly or indirectly. The MHC molecules of the complexes may be produced by any methods known in the art. Examples of MHC production include but are not limited to endogenous production and purification, or recombinant production and expression in host cells. In a preferred embodiment, the MHC heavy chain and β2m molecules are expressed in *E*. *coli* and folded together with a synthesized peptide. In another embodiment, the peptide/MHC complex may be produced as the genetically-engineered single-chain trimer (or the single-chain dimer plus MHC heavy chain) described hereinabove.

For multimerizing the two or more copies of the peptide/MHC complex to form the immunogen, each of the peptide/MHC complexes may be modified in some manner known in the art to enable attachment of the peptide/MHC complexes to each other, or the multimer may be formed around a substrate to which each copy of the peptide/MHC complex is attached. The multimer can contain any desired number of peptide/MHC, complexes and thus form any multimer desired, such as but not limited to, a dimer, a trimer, a tetramer, a pentamer, a hexamer, and the like. Specific examples of multimers which may be utilized in accordance with the present invention are described hereinbelow; however, these examples are not to be regarded as limiting, and other methods of multimerization known to those of skill in the art are also within the scope of the present invention. Streptavidin has four binding sites for biotin, so a BSP (biotinylation signal peptide) tail may be attached to the MHC molecule during production thereof, and a tetramer of the desired peptide/MHC complex could be formed by combining the peptide/MHC complexes with the BSP tails with biotin added enzymatically *in vitro.* An immunoglobulin heavy chain tail may be utilized as a substrate for forming a dimer, while a TNF tail may be utilized as a substrate for forming a trimer. An IgM tail could be utilized as a substrate for forming various combinations, such as tetramers, hexamers and pentamers. In addition, the peptide/MHC complexes may be multimerized through liposome encapsulation or artificial antigen presenting cell technology (see U.S. Serial No. 10/050,231, filed by Hildebrand et al. on January 16, 2002, Further, the peptide/MHC complexes may be multimerized through the use of polymerized streptavidin and would produce what is termed a "streptamer" (see http://www.streptamer.com/streptamer/,

The immunogen of the present invention may further be modified for providing better performance or for aiding in stabilization of the immunogen. Examples of modifications which may be utilized in accordance with the present invention include but are not limited to, modifying anchor/tail or modifying amino acids in peptide/MHC complex, PEGalation, chemical cross-linking, changes in pH or salt depending on the specific peptide of the peptide/MHC complex, addition of one or more chaperone proteins that stabilize certain peptide/MHC complexes, addition of one or more adjuvants that enhance immunogenicity (such as but not limited to the addition of a T cell epitope on a multimer), and the like.

Once the immunogen is produced and stabilized, it is delivered to a host for eliciting an immune response. The host may be any animal known in the art that is useful in biotechnological screening assays and is capable of producing recoverable antibodies when administered an immunogen, such as but not limited to, rabbits, mice and rats. Preferably, the host is a mouse, such as a Balb/c mouse or a transgenic mouse. In another embodiment, the mouse is transgenic for the particular MHC molecule of the immunogen so as to minimize the antigenicity of the immunogen, thereby ensuring that the 3-dimensional domain of the peptide sitting in the binding pocket of the MHC molecule is the focus of the antibodies generated thereto and thus is preferentially recognized with high specificity. In yet another embodiment, the mouse is transgenic and produces human antibodies, thereby greatly easing the development work for creating a human therapeutic.

After the host is immunized and allowed to elicit an immune response to the immunogen, a screening assay is performed to determine if the desired antibodies are being produced. In a preferred embodiment, the assay requires four components plus the sera of the mouse to be screened. The four components include: (A) a binding/capture material (such as but not limited to, streptavidin, avidin, biotin, etc.) coated on wells of a solid support, such as a microtiter plate; (B) properly folded HLA trimer (HLA heavy chain plus β2m plus peptide) molecule containing an irrelevant peptide; (C) properly folded HLA tetramer or trimer containing the peptide of interest; and (D) at least one antibody which recognizes mouse IgG and IgA constant regions and is covalently linked to a disclosing agent, such as but not limited to, peroxidase or alkaline phosphatase.

The solid support of (A) must be able to bind the HLA molecule of interest in such a way as to present the peptide and the HLA to an antibody without stearic or other hindrance. The preferred configuration of the properly folded HLA trimers in (B) and (C) above is a single-site biotinylation. If single-site biotinylation cannot be achieved, then other methods of capture, such as antibody may be used. If antibody is used to capture the HLA molecule onto the solid support, it cannot cross-react with the anti-mouse IgG and IgA in (D) above.

Prior to assaying the serum from immunized mice, it is preferred that the bleeds from the immunized mice be preabsorbed to remove antibodies that are not peptide specific. The preabsorption step should remove antibodies that are reactive to epitopes present on any component of the immunogen other than the peptide, including but not limited to, β2m, HLA heavy chain, a substrate utilized for multimerization, an immunogen stabilizer, and the like.

One preferred embodiment of methods of assaying serum from immunized mice is described in the attached disclosures and figures (see for example Figure 5), as well as in the Example provided hereinafter. Once it is determined that the desired antibodies are being produced, a standard hybridoma fusion protocol can be employed to generate cells producing monoclonal antibodies. These cells are plated such that individual clones can be identified, selected as individuals, and grown up in individual wells in plates. The supernatants from these cells can then be screened for production of antibodies of the desired specificity. These hybridoma cells can also be grown as individual clones and mixed and sorted or grown in bulk and sorted as described below for cells expressing surface immunoglobulin of the desired reactivity.

In another embodiment of the present invention, cell sorting is utilized to isolate desired B cells, such as B memory cells, prior to hybridoma formation. One method of sorting which may be utilized in accordance with the present invention is FACS sorting, as B memory cells have immunoglobulin on their surface, and this specificity may be utilized to identify and capture these cells. FACS sorting is a preferred method as it involves two color staining. Optionally, beads can be coated with peptide/HLA complex (with FITC or PE) and attached to a column, and B cells with immunoglobulin on their surface can be identified by FACS as well as by binding to the complex. In yet another alternative, a sorting method using magnetic beads, such as those produced by Dynal or Miltenyi, may be utilized.

In another embodiment of the present invention, the sorted B cells may further be differentiated and expanded into plasma cells, which secrete antibodies, screened for specificity and then used to create hybridomas or have their antibody genes cloned for expression in recombinant form.

Once the antibodies are sorted, they are assayed to confirm that they are specific for one peptide/MHC complex and to determine if they exhibit any cross reactivity with other HLA molecules. However, other methods of assaying for quality control are within the skill of a person of ordinary skill in the art and therefore are also within the scope of the present invention.

The present invention also includes a predictive screen to determine if a particular peptide can be utilized in an immunogen of the present invention for producing the desired antibodies which act as T-cell receptor mimics. These screens include but are not limited to, stability, refolding, IC₅₀, K_{d}, and the like. The present invention may provide a threshold of binding affinity of peptide so that a predictive threshold can be created for examining entire proteins of interest for potential peptides. This threshold can also be used as a predictor of yield that can be obtained in the refolding process of producing the peptide/MHC complex. In addition, if a potential peptide is shown to be low to medium in the predictive screens, methods of modifying the immunogen can be attempted at the onset of the production of immunogen.

The TCR mimics of the present invention have a variety of uses. The TCR mimic reagents could be utilized in a variety of vaccine-related uses. In one embodiment, the TCR mimics could be utilized as direct therapeutic agents, either as an antibody or bispecific molecule. In another embodiment, the TCR mimics of the present invention could be utilized for carcinogenic profiling, to provide an individualized approach to cancer detection and treatment. The term "carcinogenic profiling" as used herein refers to the screening of cancer cells with TCRm's of various specificities to define a set of peptide/MHC complexes on the tumor. In another embodiment, the TCR mimics of the present invention could be utilized for vaccine validation, as a useful tool to determine whether desired T cell epitopes are displayed on cells such as but not limited to, tumor cells, viral infected cells, parasite infected cells, and the like. The TCR mimics of the present invention could also be used as research reagents to understand the fate of antigen processing and presentation *in vivo* and *in vitro,* and these processes could be evaluated between solid tumor cells, metastatic tumor cells, cells exposed to chemo-agents, tumor cells after exposure to a vaccine, and the like. The TCR mimics of the present invention could also be utilized as vehicles for drug transport to transport payloads of toxic substances to tumor cells or viral infected cells. Further, the TCR mimics of the present invention could also be utilized as diagnostic reagents for identifying tumor cells, viral infected cells, and the like. In addition, the TCR mimic reagents of the present invention could also be utilized in metabolic typing, such as but not limited to, to identify disease-induced modifications to antigen processing and presentation as well as peptide-HLA presentation and tumor sensitivity to drugs.

**Table I - Peptides Utilized in the Methods of the Present Invention**

| Name | Sequence | SEQ ID NO: | Origin | Position | IC₅₀ | Tetramer Yield (mg) |
|---|---|---|---|---|---|---|
| p 5 3 (264) | LLGRNSFEV | 1 | Tumor suppressor p53 | (264-272) | 1273 | 1.99 +/- 0.76 |
| elF4G | VLMTEDIKL | 2 | eukaryotic transcription initiation factor 4 gamma | (720-728) | 690.3 | 2.77 +/- 1.09 |
| Her2/neu | KIFGSLAFL | 3 | tyrosine kinase-type cell surface receptor Her2 (EC 2.7.1.112) (C-erbB-2) | (369-377) | 881.9 | 0.89 +/- 0.69 |

### EXAMPLE 1

The human p53 protein is an intracellular tumor suppressor protein. Point mutations in the p53 gene inactivate or reduce the effectiveness of the p53 protein and leave cells vulnerable to transformation during progression towards malignancy. As cells attempt to compensate for a lack of active p53, over production of the p53 protein is common to many human cancers including breast cancer, resulting in cytoplasmic increases in p53 peptide fragments such as the peptide 264-272. There are many reports demonstrating that surface HLA-A2 presents the 264-peptide epitope from wild-type p53 (Theobald et al., 1995; and Theobald et al.,1998). Cytotoxic T lymphocytes have been generated against the 264-peptide-HLA-A2 complexes (referred to herein as 264p-HLA-A2) on breast cancer cells from peripheral blood monolayer cells (PBMC) of healthy donors and individuals with breast cancer (Nikitina et al., 2001; Barfoed et al., 2000; and Gnjatic et al., 1998). Further, several studies have reported successful immunization with the 264 peptide in HLA-A2 transgenic mice (Yu et al., 1997; and Hoffmann et al., 2005). The studies were successful in generated murine CTL lines reactive against the 264p-HLA-A2 complex and showed that these murine CTL lines could detect and destroy human breast cancer cells. Because the 264-peptide presented by HLA-A2 on the surface of malignant cells is recognized by the immune system and it has relatively high affinity (IC₅₀ <1 nM) (Chikamatsu et al., 1999), the 264 peptide was utilized in Example 1 to construct 264p-HLA-A2 tetramers for use in immunizing mice for production of T cell receptor mimics in accordance with the present invention.

Preparation of 264p-HLA-A2 peptide tetramers: The heavy and light (β2m) chains of the HLA-A2 Class I molecule were expressed and prepared separately in *E. coli* as insoluble inclusion bodies according to established protocols. The inclusion bodies were dissolved in 10 M urea, and the heavy and light chains were mixed at a molar ratio of 1:2 at a concentration of 1 and 2 mM respectively with 10 mg of a synthetic peptide (LLGRNSFEV; SEQ ID NO:1) from the human p53 tumor suppressor protein (amino acids 264-272) in a protein refolding buffer and were allowed to refold over 60 hr at 4°C with stirring. The filtrate of this mix was concentrated, and the buffer was exchanged with 10 mM Tris pH 8.0. The mix was biotinylated using a recombinant birA ligase for two hours at room temperature and then subjected to size exclusion chromatography on a Sephadex S-75 column (Superdex S-75, Amersham GE Health Sciences) (Figure 1). Alternatively, a monomer HLA-A2-peptide can be purified from a Sephadex S-75 column, concentrated and then biotinylated using birA ligase for 2 hours at room temperature. The refolded biotinylated monomer peak was reisolated on the S-75 column and then multimerized with streptavidin (SA) at a 4:1 molar ratio. The multimerized sample was subjected to size exclusion chromatography on a Sephadex S-200 column (Figure 2).

The stability of the 264p-HLA-A2 tetramers was assessed in mouse serum at different temperatures using the conformational antibodies BB7.2 and W6/32 (Figure 3). The results suggest that 50% of the 264p-HLA-A2 tetramers maintain a conformational integrity after 10 h incubation at 37°C. Only 10% of tetramers remain stable after 40 h incubation. However, the multimerization of 264p-HLA-A2 greatly increased the half life of the molecules; normally monomers only have a few hours half life in mouse serum. It was not clear *a priori* that these tetramers would be stable long enough to elicit a robust immune response in mouse, but recent results indicated that at least a fraction of the injected tetramers were stable long enough in mice to elicit a specific antibody response.

Immunization of Balb/c mice (female and male) with peptide-HLA-A2: The complete structure of the peptide-HLA-A2 tetramer immunogen is shown in Figure 4. Balb/c mice (female and male) were immunized with the 264p-HLA-A2 tetramers. Each mouse was injected subcutaneously every 2 weeks (up to 5 times) with immunogen (50 µg) in PBS which also contained 25 µg of Quil A (adjuvant) in 100 µl.

Blood samples from mice were collected into 1.5 ml eppendorf microcentrifuge tubes containing heparin, and plasma was clarified by centrifugation at 6,000 x g for 10 minutes. The recovered plasma samples were then frozen at -20°C and later used in screening assays. Samples were diluted 1:200 into 0.5% milk in Phosphate Buffered Saline solution (PBS) and pre-absorbed with refolded monomer HLA-A2 containing an irrelevant peptide (Her2/neu) before screening.

Effective assays were needed to analyze anti-peptide-HLA antibodies in the serum of immunized mice, and several factors complicate this analysis. One of these factors is predicated on the fact that a specific antibody response against a complex epitope represented by both the peptide and the binding site of the HLA molecule is being sought, and this epitope may represent only a minor target to B cells. A significant portion of the antibodies raised against peptide-HLA tetramers are generated against HLA as well as streptavidin (SA) utilized to tetramerize the peptide-HLA complexes; consequently, an assay protocol had to be developed that allowed for detection of a low concentration of specific antibodies in a milieu of non-specific ones. To resolve this problem, a pre-absorption step was incorporated into an ELISA assay format. This step was designed to remove antibodies against HLA and β2-microglobulin from the reaction. In a variation of this assay, biotinylated non-relevant monomers were used to pre-absorb and then remove the formed complexes from the reaction on a sold surface-bound SA. In the ELISA format, sera from immunized mice are first reacted with HLA-A2 monomers containing another irrelevant peptide before reacting them with HLA-A2 complexes of the relevant peptide. The specifics of these assays are described in more detail herein below.

Pre-Absorption assay: Serum from the immunized mice was used in an ELISA format to identify "peptide-specific" antibody responses. Remember that TCR mimics are antibodies having dual specificity for both peptide and HLA. In addition, the immunized mice will produce antibody specificities against HLA epitopes. It is these antibodies that the pre-absorption protocol substantially removes from the serum samples. In order to substantially remove antibodies that were not peptide specific, a pre-absorption step was included in the protocol. It was assumed that 12 µg of IgG is present in 1 ml of mouse serum, and that 10% of the IgG in immunized mouse serum is specific for an epitope on the peptide-HLA-A2 immunogen. Based on these assumptions, 1.2 µg of IgG in 1 ml of serum from an immunized mouse is potentially specific for some position on the peptide-A2 molecules and is not "peptide specific". In order to remove these non-specific antibodies, 20 µg of biotinylated Her2/neupeptide-HLA-A2 (which differs from 264p-HLA-A2 only in the peptide) was added to 1 ml of a 1:200 dilution of each mouse bleed. Samples were incubated overnight at 4°C with agitation. The next morning 0.5 ml of sample was added to a well in a 12 well plate (which had been coated the previous night with 10 mg of streptavidin and blocked in 5% milk protein) and incubated for 1 hour. The pre-absorbed samples were then transferred to a second streptavidin coated well on the plate. This process was repeated one more time (a total of 3) to ensure efficient removal of antibody-HLA complexes and antibodies reactive to streptavidin and/or biotin. After completing the pre-absorption steps, samples were ready for use in the screening ELISA.

Screening ELISA: Figure 5 demonstrates the development of an ELISA assay for screening mouse bleeds to determine if there are antibodies specific to the peptide-of-interest-HLA-molecule complex present. Pre-absorbed serum samples from six Balb/c mice were individually tested in the ELISA screening assay of Figure 5 (see Figure 6). Briefly, 96 well plates (maxisorb; Nunc) were coated the night before with 0.5 µg of either biotinylated 264p-HLA-A2 monomer or biotinylated elF4Gp-HLA-A2 monomer at 4°C. (Subsequence interactions used non-biotinylated forms of the relevant and irrelevant HLAs.) The following day, wells were blocked with 1% milk for 1 h at room temperature and rinsed 1x in PBS. The pre-absorbed serum samples (50 µl/well) were then added to wells starting at 1:200 dilution, and titrating down to a final dilution equivalent to either 1:1600 or 1:3200. After 2 hr incubation at room temperature, the plate was washed 2x in PBS followed by the addition of antibody conjugate (goat anti-mouse-HRP, 1:500 dilution) and incubated for 1 h at room temperature. The plate was then washed 3x in PBS and developed after addition of 50 µl of tetramethylbenzidine (TMB) substrate. Development time was 5 to 10 minutes, and the reaction was stopped with the addition of 50 µl quench buffer (2 M sulfuric acid). The results were read at 450 nm absorbance (Figure 6).

For a positive control in the assay, BB7.2 mAb was used at 50 to 200 ng/well. This mAb recognizes only conformationally correct forms of the refolded peptide-HLA-A2 molecule. For a negative control in the assay, a peptide-HLA-A2 complex containing an irrelevant peptide was coated on the plate. In this particular assay, the negative control was elF4G peptide-loaded HLA-A2 monomer.

In addition, the mice used for the production of the antibodies were pre-bled in order to ensure that Balb/c mice do not harbor antibodies specific for the desired antigens before immunization. Assay background was determined using pre-bleed samples at 1:200 and 1:400 dilution. The highest absorbance reading recorded for pre-bleeds was less than OD 0.06 at 450 nm.

Figure 6 shows the results from an ELISA of six individual bleeds from Balb/c mice immunized with tetramers of 264p-HLA-A2. The data shown in Figure 6 demonstrates that both male and female mice immunized with 264p-HLA-A2 tetramers make specific antibody to 264p-HLA-A2 monomers. Bleeds incubated in wells containing elF4Gp-HLA-A2 monomers (irrelevant peptide) were used to evaluate non-specific reactivity of bleeds. The findings shown in Figure 6 demonstrate minimal reactivity to elF4Gp/A2 with signal to noise ratios ranging from 3 to 6 fold, indicating that immunization of mice with peptide-A2 tetramers leads to the generation of specific antibody responses to the immunogen.

The results presented in Figure 6 demonstrate that antibodies in the serum reacted twice as strongly or stronger with 264p-HLA-A2 as compared to elF4Gp-HLA-A2, suggesting that some specific antibodies against the p53-264p epitope are present. The larger the difference in the response between reactivity with HLA-A2 complexes with a relevant or irrelevant peptide, the higher the titer for specific antibodies in the sera. The results in Figure 6 clearly demonstrate that serially diluted sera from all six mice generated a signal with 264p-HLA-A2 monomers that was 2-5 times stronger than the signal with eIF4Gp-HLA-A2 monomers, clearly demonstrating the effectiveness of the methods of the present invention.

T2 binding assay: To confirm the ELISA findings, the binding of the different mouse bleed samples to T2 cells pulsed with either the 264 peptide (peptide of interest) or the elF4G peptide (irrelevant peptide) was investigated, as shown in Figure 7. T2 cells are a human B lymphoblastoid cell line (ATCC CRL-1999) that has been well characterized by Peter Creswell (Wei et al., 1992). T2 cells are useful for studying recognition of HLA-A2 antigens because they are deficient in peptide loading. These cells have been found to be deficient in TAP1/2 proteins, which are necessary proteins for transporting peptides from the cytosol into the endoplasmic reticulum for loading HLA class I molecules. Because of the TAP1/2 deficiency, these cells express a low level of empty HLA-A2 molecules on the surface. Thus, these cells can be primed (loaded) with peptides of choice, and the cells will display them appropriately in the context of HLA-A2 molecules on their surface. Addition of peptide to these cells leads to peptide binding to the HLA-A2 molecules which are constantly cycling to the surface and stabilization of the HLA-A2 structure. The more stable structure increases the density of surface displayed HLA-A2 molecules that are loaded with the particular peptide of interest. T2 cells can be loaded with relevant or irrelevant peptide, and the reactivity of immune sera from immunized mice against them can be measured. The larger the difference in the response between T2 cells loaded with relevant or irrelevant peptide, the higher the titer for specific antibodies in the sera.

T2 cells were loaded with either the 264 or the elF4G peptide, and then the cells were stained with the BB7.2 antibody to detect the level of HLA-A2 molecules present on the surface of T2 cells. Figure 8 shows that both 264 and elF4G peptides have been successfully loaded by comparing the BB7.2 staining profile of cells that received peptide versus the cells that did not receive peptide (negative controls). These findings demonstrate that elF4G peptide may be more efficient at loading and stabilizing HLA-A2 on T2 cells than the 264 peptide.

Figure 9 illustrates the results of staining of 264 peptide-loaded T2 cells with the I3M2 mouse bleed. The pre-absorbed mouse sample preferentially binds cells pulsed with 264 peptide. In contrast, Figure 10 demonstrates that the pre-bleed samples (mice bleeds taken prior to immunization) show no sign of reactivity to T2 cells pulsed with either the 264- or elF4G peptide. In combination, these results clearly demonstrate that a polyclonal peptide-HLA specific antibody response can be generated to the specific three-dimensional , and that these antibodies are specific for the immunogen that was used. They confirm that the antibodies produced also recognize a "native" or natural form of the peptide-HLA-A2 complex and are not restricted in reactivity to the refolded form used to prepare the immunogen.

Hybridomas were generated by submitting 12. mice immunized with 264p-HLA-A2 to the Hybridoma Center, Oklahoma State University, Stillwater, Oklahoma, for hybridoma generation using standard technology. In total, the center returned 1440 supernatants from p53-264 hybridoma isolates for screening. Figure 11 depicts development of assays to screen hybridomas to determine if they are producing anti-peptide-HLA specific antibodies. In a primary ELISA screen, 40 positives were identified, and in a secondary screen, 7 positives against 264p-HLA-A2 were identified. The results from screening hybridoma supernatants by a competitive binding ELISA are shown in Figure 12. Supernatants that had ratios of eIF4G/264 greater than 1.7 were considered positive, and after expanding hybridoma numbers, the supernatant was re-screened. Approximately 1500 wells were screened, and approximately 50 positives were identified after the primary screen.

Hybridomas determined positive after a first screening were expanded, and the supernatant was diluted and rescreened by competitive ELISA two weeks after cell growth. Figure 13 represents data obtained from a competitive ELISA of these positive hybridoma clones. TCRm's specific for 264p-HLA A2 were determined by showing a reduction in absorbance (read at 450 nm) after addition of competitor (no tetramer versus 264p tetramer), while no change in absorbance was observed after addition of non-competitor (no tetramer versus elF4Gp tetramer). These findings confirm anti-264p-HLA-A2 specificity of TCRm's and validate the protocols of the presently disclosed and claimed invention for generating monoclonal antibodies specific for peptide-HLA complexes.

Supernatant from 13.M3-2A6 was characterized further by a cell-based competitive binding assay, as shown in Figure 14. These findings demonstrate that 13.M3.2A6 TCRm has specificity for the authentic 264p-HLA A2 epitope. This is illustrated by the significant reduction of TCRm binding to 264p pulsed T2 cells in the presence of the competitor versus the non-competitor. The competitor reduces binding by greater than 3.5 fold (as measured by mean channel fluorescence) compared to the effect of an equivalent amount of non-competitor.

Therefore, the results presented herein in Example 1 clearly demonstrate that the immunogen of the present invention is capable of eliciting an immune response in a host that is specific for an epitope formed by a desired peptide presented in the context of an HLA molecule.

These results also indicate there is a significant component of the antibody reactivity in most of the immunized mice that recognizes epitopes that are not specific to the peptide in the context of the HLA binding groove. Rather, these antibodies probably recognize other epitopes common to properly folded HLA-A2 molecules (independent of the peptide region) or epitopes which form as the immunogen is processed, unfolded and denatured in the body.

Appropriate measures must be taken to remove these "non-peptide-specific" antibodies from the serum prior to evaluating it for the presence of a true TCR mimic antibody. The ability to discover an antibody which recognizes the peptide of interest in its authentic three-dimensional configuration when the HLA-binding groove is dependent upon (1) the creation of an immunogen capable of presenting the peptide in this context, and (2) the ability to prepare the serum from the immunized animal in such a way that the peptide specific reactivity is revealed.

### EXAMPLE 2

The eukaryotic translation initiation factor 4 gamma (elF4G) is a protein which is part of a complex of molecules that are critical in regulating translation. When breast carcinoma cell lines (MCF-7 and MDA-MB-231) were stressed with serum starvation, the elF4G protein degrades into smaller peptide fragments (Morley et al., 2000; Morley et al., 2005; Bushell et al., 2000; and Clemens, 2004). A peptide of elF4G has been identified as being presented by HLA molecules on HIV infected cells at a higher frequency than in uninfected cells by the epitope discovery method of Hildebrand et al. The epitope discovery methodology is shown in Figure 15. Briefly, an expression construct encoding a secreted HLA molecule is transfected into a normal cell line and an infected, diseased or cancerous cell line (in this case, an HIV infected cell line), and the cell lines are cultured at high density in hollow-fiber bioreactors. Then, the secreted HLA molecules are harvested and affinity purified, and the peptides bound therein are eluted. The peptides from the uninfected cell line and the HIV infected cell line are then comparatively mapped using mass spectroscopy to identify peptides. that are presented by HLA at a higher frequency in the HIV infected than in the uninfected cells. Using this method, the peptide VLMTEDIKL (SEQ ID NO:2), was identified, and determined to be a peptide fragment of eukaryotic translation initiation factor 4 gamma (elF4G). The peptide of SEQ ID NO:2 is referred to herein as the "elF4G peptide", or "elF4Gp".

Monomers and tetramers of elF4Gp-HLA-A2 complexes were produced in a similar manner as described in Example 1 for the 264p-HLA-A2 complexes. Briefly, 10 mg (10 µM) of peptide were refolded with 46 mg (1 µM) of HLA-A2 heavy chain and 28 mg (2 µM) of HLA light chain under appropriate redox conditions over approximately 60 hours at 4°C. The monomers were biotinylated and multimerized with streptavidin to form tetramers, and the tetramers were purified on a Superdex S200 column. Under the abovementioned conditions, typically 10-20 mg properly folded monomer, 8-12 mg of biotinylated monomer, and 2-3 mg of tetramers were produced.

Tetramer stability was assessed as described in Example 1 for the 264p-HLA-A2 tetramers. In contrast to the 264p-HLA-A2 tetramers, which have a half life of 10 hours at 37°C, elF4Gp-HLA-A2 tetramers have a half life of 20 hours, and 40% of tetramers remain stable after 40 hours of incubation.

The elF4Gp-HLA-A2 tetramers were utilized to immunize Balb/c mice as described in Example 1, and the mice were bled and sera assayed using the ELISA method described above in Example 1 and in Figure 5. Sera from a mouse immunized with elF4Gp-HLA-A2 tetramers was pre-absorbed with biotinylated 264p-HLA-A2 monomers. The serum was reacted with SA on a solid surface and then used in an ELISA format. Serum was reacted with solid surface bound (1) 264p-HLA-A2 monomers; (2) elF4Gp-HLA-A2 monomers; or (3) Her2/neu-peptide-HLA-A2 monomers, and the bound antibody was detected with a goat anti mouse (GAM)-HRP conjugate antibody. The ELISA reactions were then developed with TMB (an HRP chromogenic substrate), and the absorbance read at 450 nm. The results shown in Figure 17 illustrate that antibodies in the serum generated a signal that was twice as strong or stronger with elF4Gp-HLA-A2 than with either 264p-HLA-A2 or Her2/neu-peptide-HLA-A2, suggesting that some specific antibodies against the elF4Gp epitope are present.

To confirm the ELISA findings, cell based assays were performed. T2 cell direct binding assays, as described in Example 1 and in Figure 7, were performed, and the results shown in Figures 18 and 19. In these assays, T2 cells were loaded with a relevant (elF4Gp) or irrelevant (264p) peptide, and the reactivity of immune sera from immunized mice against them were measured. Figure 18 demonstrates the detection of HLA-A2 levels on peptide-pulsed T2 cells using BB7.2 mAb. This figure demonstrates the successful and relatively equivalent loading of both the 264 and elF4G peptides on the surface of HLA-A2 T2 cells.

Figure 19 demonstrates the results of staining elF4G and 264 peptide-loaded T2 cells with a bleed from a mouse immunized with eIF4Gp-HLA-A2. 264 peptide loaded cells are shown in the solid peak. The pre-absorbed serum sample was used at a dilution of 1:400 for staining and preferentially binds cells pulsed with the elF4G peptide (as shown by the rightward shift). The pre-bleed sample shows no sign of reactivity to T2 cells pulsed with either peptide (not shown).

Next, T2 cell-based competitive assays, as described in Example 1 and in Figure 7, were used to further evaluate the specificity of the polyclonal antibody to elF4Gp-HLA-A2, and the results are shown in Figures 20 and 21. In these assays, sera from mice immunized with elF4Gp-HLA-A2 tetramers were diluted 1:200 in PBS and pre-absorbed against Her2/neupeptide-HLA-A2. The sera was then mixed with elF4Gp-HLA-A2 or with 264p-HLA-A2, either in the form of monomers (Figure 20) or tetramers (Figure 21) and before being reacted with T2 cells loaded with elF4G peptide (100 µg/ml).

In the Figures, the maximum staining signal (filled peak) is shown for the anti-serum. To assess the specificity of antibody binding, a competitor (elF4Gp-HLA-A2) or a non-competitor (264p-HLA-A2) was included in the cell staining reaction mix at three different concentrations (0.1, 1.0 and 10 µg). The results shown in Figures 20 and 21 reveal that the addition of the 264p-HLA-A2 monomer or tetramer had little inhibitory activity on anti-serum binding to elF4G peptide-loaded T2 cells. In contrast, a dose-response effect of specific binding to T2 cells was observed in the presence of the competitor elF4Gp-HLA-A2 monomer or tetramer. These findings provide additional evidence that the immunization strategy of the presently disclosed and claimed invention can elicit a specific anti-peptide-HLA-A2 IgG antibody response.

Mouse hybridomas were generated as described in Example 1 using standard technology, and immunogen specific monoclonals were identified using a competitive binding ELISA (as described herein before). From over 800 clones, 27 mAb candidates were identified, and 4F7 mAb (IgG1 isotype) was selected for further characterization. After expanding the 4F7 hybridoma cell line by known methods in the art, the mAb was purified from 300 ml of culture supernatant on a Protein-A column that yielded 30 mg of 4F7 mAb. The specificity of antibody binding to relevant peptide-HLA-A2 tetramers and 3 irrelevant peptide-HLA-A2 tetramers was determined by ELISA, as shown in Figure 22. The 4F7 mAb showed specific binding only to elF4Gp-HLA-A2 tetramers; no signal was detected using irrelevant peptide-A2 controls, which included peptide VLQ and TMT, both derived from the human beta chorionic gonadotropin protein, and 264 peptide derived from the human p53 tumor suppressor protein.

Next, the binding affinity and specificity of the 4F7 mAb was determined by plasmon surface resonance (BIACore). 4F7 mAb was coupled to a biosensor chip via amine chemistry, and soluble monomers of HLA-A2 loaded with 264 or elF4G peptide were passed over the antibody coated chip. In Figure 23, specific binding of soluble elF4Gp-HLA-A2 monomer to 4F7 mAb was observed, while no binding to 264p-HLA-A2 complexes containing the irrelevant peptide p53-264 was observed. The affinity constant of 4F7 mAb for its specific ligand was determined at 2 x10⁻⁹M.

In Figures 22 and 23, 4F7 binding to recombinant elF4Gp-HLA-A2 molecules was demonstrated. In Figure 24, 4F7 binding to elF4Gp-HLA-A2 complexes on the surface of T2 cells was demonstrated. In this experiment cells were pulsed at 10 µg/ml with the following peptides: elF4G, 264, and TMT. Unpulsed T2 cells were also used as a control. In Figure 24A, T2 cells pulsed with irrelevant peptides or no peptide and stained with 4F7 (50 ng) displayed minimal signal. In contrast, 4F7 staining of elF4G peptide loaded T2 cells resulted in a significant rightward shift, indicating specific binding of 4F7. In Panel B, T2 cells were stained with BB7.2 mAb (specific for HLA-A2). T2 cells loaded with any of the peptides resulted in a rightward shift of the peak, indicating that each of the peptides efficiently loads the HLA on the cell surface. These data also indicate that the 4F7 binding to T2 cells is dependent on the antibody recognizing both peptide and HLA-A2.

The next goal was to use the 4F7 mAb to detect elF4Gp-HLA-A2 complexes on human breast carcinoma cell lines MCF-7 and MDA-MB-231. In Figure 25A, MCF-7 cells were stained with 100 ng of 4F7 mAb and showed a significant rightward shift compared to the isotype control. To determine if binding was indeed specific for the elF4G peptide, soluble tetramers (competitor and non-competitor) were used to block 4F7 binding. As expected, elF4Gp-HLA-A2 tetramer completely blocked 4F7 staining, while the non-competitor, 264p-HLA-A2, failed to block 4F7 mAb from binding to cells. In Figure 25B, the HLA-A2 negative breast carcinoma cell line BT-20 was not stained with 4F7 mAb. These findings support the specific binding of 4F7 antibody to elF4Gp-HLA-A2 complex.

In Figure 26, three panels are shown in which MDA-231 cells were stained with 4F7 mAb (50 ng) in the absence or presence of soluble peptide-HLA-A2 monomers. The three peptide-HLA-A2 monomers selected were elF4Gp (competitor) and 264p and Her2/neu peptide (non-competitors). As shown in Figure 26A, 4F7 binds to MDA-231 cells, and its binding is significantly inhibited using competitor. In contrast, no reduction in binding signal strength was seen with either non-competitor, indicating that 4F7 binds to tumor cells in a specific manner.

These data confirm the isolation of a novel TCRm monoclonal antibody with specificity for a peptide derived from the elF4G protein that is presented by HLA-A2 on the surface of breast cancer cells.

### EXAMPLE 3

Her-2(9₃₆₉) represents a common epitope expressed by various tumor types including breast carcinomas (Brossart et al., 1999). Approximately 20-30% of primary breast cancers express Her-2. The Her-2/neu receptor protein is a member of the tyrosine kinase family of growth factor receptors (Coussens et al., 1985) that is frequently amplified and overexpressed in breast cancer (Slamon et al., 2001). The Her-2/neu protein is generally displayed on the surface of cells and, during malignancy, is detected at high levels on tumor cells. Although its precise anti-tumor mechanism(s) remain unknown, Herceptin, an anti-Her-2/neu antibody, is used in breast cancer treatment to target the receptor on the surface of tumor cells. In addition to using antibodies to attack tumors expressing Her-2/neu receptor on their surface, Her-2/neu oncoprotein contains several HLA-A2-restricted epitopes that are recognized by CTL on *autologous* tumors. The most extensively studied Her-2 epitope (and the one utilized herein in Example 3) spans amino acids 369-377 (Her-2(9₃₆₉)) (KIFGSLAFL; SEQ ID NO:3) (Fisk et al., 1995) and is recognized by tumor associated lymphocytes as well as reactive T cell clones as an immunodominant HLA-A2-restricted epitope. The peptide has been shown to bind with high affinity to HLA-A2 alleles (Fisk et al., 1995; and Seliger et al., 2000). The Her-2(9₃₆₉) epitope binds to HLA-A2 with intermediate affinity (IC₅₀ ∼ 50 nM) (Rongcun et al., 1999), and because it is grossly overexpressed on malignant cells, it has been used as a vaccine candidate in several clinical trials. For instance, Knutson et al. (2002) demonstrated that patients immunized with Her-2(9₃₆₉) could develop interferon-gamma (IFN-γ) responses to the peptide and exhibited increased Her-2(9₃₆₉)-specific precursor frequencies.

Her2/neu-peptide-HLA-A2 monomers and tetramers were generated as described above in Example 1. However, Her2/neu-peptide-HLA-A2 tetramers were generated at a lower efficiency than for either 264p-HLA-A2 tetramers (Example 1) or elF4Gp-HLA-A2 tetramers (Example 2), as shown in Table 1. The relatively low tetramer yields with Her2/neu peptide do not correlate well with the high affinity of this peptide to HLA-A2. The IC₅₀ of Her2/neu peptide is lower than p53-264, yet tetramer yield with Her2/neu peptide is two to three fold less than tetramer yield with p53-264.

To solve this yield problem, it was determined that the peptide needed to be solubilized in a solvent, such as but not limited to, DMSO or DMF, prior to refolding with the heavy and light chains. Once the Her2/neu peptide was solubilized in DMSO, sufficient amounts of Her2/neu peptide monomer and tetramer were produced.

The Her2/neu-peptide-HLA-A2 tetramers were utilized for immunization of Balb/c mice and generation of monoclonal antibodies as described in detail in Examples 1 and 2. A monoclonal antibody reactive for Her2/neu-peptide-HLA-A2 was isolated and designated 1 B8. Screening of hybridoma supernatant of 1B8 is shown in Figure 27, in which specific binding of 1B8 mAb is demonstrated by competitive ELISA. Her2/neu-peptide-HLA-A2 tetramer (100 ng/well) was used to coat a 96-well plate. In Figure 27, 3-fold dilutions of the 1B8 culture supernatant were made in the presence of competitor (500 ng of Her2/neu-peptide-HLA-A2 tetramer), non-competitor (500 ng of eIF4Gp-HLA-A2 tetramer), or no tetramer. No difference was observed between no tetramer and the non-competitive control. In contrast, a significant reduction was observed in the presence of competitor at dilutions of 1:27, 1:81 and 1:243, indicating specificity for the Her2/neu-peptide-HLA-A2 complex.

Figure 28 demonstrates the specificity of the 1B8 mAb by tetramer ELISA. In this experiment a 96-well plate was coated with one of the peptide-tetramers including the following peptides: Her2/neu peptide, VLQ, TMT, eIF4Gp, and 264p. A strong absorbance signal was detected only in wells containing Her2/neu-peptide-HLA-A2, indicating 1 B8 has specificity for binding to this target.

In Figure 29, T2 cells were stained with cell supernatant from hybridoma 1B8 to demonstrate binding specificity for this monoclonal antibody for the Her2/neu-peptide-HLA-A2 complex. T2 cells were incubated with Her-2/neu, 264, or TMT peptide and then stained with 1B8 or BB7.2 antibody. Panel A shows no binding by 1B8 TCR mimic to T2 cells without exogenous peptide. Panels B and C show no binding with 1 B8 to T2 cells pulsed with either 264 or TMT peptide. Panel D shows specific binding of the 1 B8 TCR mimic to T2 cells loaded with the Her-2/neu peptide as indicated by the strong rightward shift of the open peak. In addition, all cells were stained with the BB7.2 antibody with a greater than 2-fold shift in staining intensity seen with peptide loaded T2 cells (see Panels B thru D). In all Panels the IgGl and IgG2b isotype controls did not stain T2 cells (see filled peaks in all Panels). Collectively, these data demonstrate specific binding of the 1 B8 monoclonal antibody for Her-2/neu peptide-pulsed T2 cells.

Figure 30 illustrates 1B8 staining of MDA-MB-231 and MCF-7 human breast carcinoma cells. MDA-MB-231 cells were stained with 1B8 to demonstrate that the antibody specifically recognizes endogenous Her-2/neu peptide-HLA-A2 complex on the cell surface. Figure 30 demonstrates a 1 B8 titration effect for binding to endogenous Her-2/neu peptide-HLA-A2 complexes on (A) MDA-231 and (B) MCF-7 human breast cancer cells. In addition, both cell lines stained positive for HLA-A2 using BB7.2 antibody. In Figure 30C, neither 1B8 nor BB7.2 antibodies could stain the HLA-A2 negative human breast cancer cell line, BT-20. These data indicate the 1B8 TCR mimic binding is specific for Her-2/neu peptide-HLA-A2 and that the 1B8 can detect this epitope on the surface of human breast cancer cells.

Literature indicates that there is low Her2 expression on the surface of MDA-MB-231 cells and moderate Her2 expression on the surface of MCF-7 cells (Menendez et al., 2004a; and Menendez et al., 2004b). High surface levels of Her2 expression may correlate with low levels of peptide epitope being presented.

Figure 31 illustrates the specific inhibition of 1B8 mAb binding to MDA-231 tumor cells. MDA-MB-231 cells were stained with 1 B8 in the presence of (1) tetramer complex that would compete with specific binding to Her2/neu-HLA-A2; (2) tetramer complex that would not compete with specific binding to Her2/neu-HLA-A2 (264p and eIF4Gp); or (3) no tetramer, to demonstrate that the antibody specifically recognizes endogenous Her-2/neu peptide-HLA-A2 complex on the cell surface. MDA-MB-231 cells were incubated with 1B8 alone or in the presence of (1) Her-2/neu-HLA-A2 tetramer (competitor); (2) 264p-HLA-A2 tetramer (non competitor); (3) eIF4Gp-HLA-A2 tetramer (non competitor); or (4) without tetramer addition. Figure 31A demonstrates 1B8 binding specificity for endogenous Her-2/neu peptide-HLA-A2 complexes on MDA-231 tumor cells. Binding of the 1B8 TCR mimic to MDA-MB-231 cells is significantly reduced in a dose-dependent manner (see leftward shift with peak) in the presence of competitor (Her-2/neu-HLA-A2 monomer). In panels B and C, it is shown that 1B8 binding is not blocked when non-relevant (264 and Her-2/neu) peptide-HLA-A2 monomers are used to compete with 1 B8 binding to MDA-231 cells. These findings support previous binding specificity data and indicate Her-2/neu-HLA-A2 as a prevalent epitope on breast cancer cells.

Figure 32 illustrates that 1B8 mAb does not bind to soluble Her2/neu peptide. MDA-MB-231 cells were stained with 1 B8 in the presence or absence of exogenously added Her-2/neu peptide. Figure 32 demonstrates that 1 B8 TCR mimic has dual specificity and does not bind to Her-2/neu peptide alone.

Thus, a new angle of attack on a proven anti-cancer target has been reported herein. The reported levels of Her2/neu peptide on the surface of MDA cells, which are reported as being low or non-existent, contrasts sharply to the staining reaction seen with the antibody of the present invention, which recognizes peptides from the protein. This may indicate that a much higher percentage of cancer cells express the receptor, but that the receptor does not traffic effectively to the surface of the cell; however, it is still a good target based on the expression level of the Her2/neu peptide associated with HLA-A2.

### SUMMARY

Shown in Figure 33 is a timeline of the protocol of generating peptide-MHC specific monoclonal antibodies of the presently disclosed and claimed invention. As evidenced by the figure and the examples provided herein above, a rapid method of generating peptide-MHC specific monoclonal antibodies has been demonstrated, wherein the peptide-MHC specific monoclonal antibodies can be generated in 8-12 weeks.

The value of monoclonal antibodies which recognized peptide-MHC complexes has been recognized for some time, as described in the Background of the Prior Art section, and several groups have generated antibodies of this type for use in investigating the characteristics of the complexes (Murphy et al., 1992; Eastman et al., 1996; Dadaglio et al., 1997; Messaoudi et al., 1999; Porgador et al., 1997; Rognan et al., 2000; Polakova et al., 2000; Denkberg et al., 2003; Denkberg et al., 2002; Biddison et al., 2003; Cohen et al., 2003; and Steenbakkers et al., 2003). There are several aspects of the presently disclosed and claimed invention that are novel over the prior art methods, and which overcome the disadvantages and defects of the prior art. First, the method of the presently disclosed and claimed invention results in hybridoma cells producing high affinity, full-length antibodies to specific peptide-HLA complexes. An example of the affinity range achieved is shown by the 4F7 monoclonal antibody (see for example, Figure 23 and Example 2), which has a K_{D} of approximately 1 nM. Affinity measurements for the 1 B8 monoclonal antibody indicate that it is in the same affinity range. The affinity of these two antibodies is high enough that they can distinctly stain breast cancer cell lines, and this aspect of the presently disclosed and claimed invention contrasts sharply with the weak staining reported for antibodies from a phage display library (Denkberg et al., 2003).

Second, in contrast to the prior art methods that utilize phage display libraries, the product produced by the method of the presently disclosed and claimed invention is "ready to use"; it is a whole antibody which is easy to purify and characterize, and does not require any further manipulation to achieve expression of significant quantities of material.

Third, the method of the presently disclosed and claimed invention requires significantly less time to product when compared to the prior art methods. The method of the presently disclosed and claimed invention can complete the cycle from immunization to identification of candidate hybridomas in as few as eight weeks, as shown in Figure 33 and as achieved as described herein for monoclonal antibody 1 B8. The method of the presently disclosed and claimed invention is both rapid and reproducible.

Fourth, the immunogen employed in the method of the presently disclosed and claimed invention is novel. The immunogen consists of peptide-HLA complexes that are loaded solely with the peptide of interest. The immunogens are made in a form which allows production and characterization of miiligram quantities of highly purified material which correctly presents the three dimensional structure of the peptide-HLA complex. This complex can be easily manipulated to form higher order multimers. Preliminary data indicates that the use of tetrameric forms of the peptide-HLA immunogen is more efficient at generating a specific response than are monomeric or mixed multimeric forms of the immunogen.

Fifth, the screening processes described in the presently claimed and disclosed invention are unique and completely describe methods to discern the presence of anti-peptide/HLA antibodies in the serum of immunized mice, even in the presence of antibodies which react with other epitopes present on the complex. The screening processes also produce methods to identify and characterize monoclonal antibodies produced after hybridoma fusion.

The presently disclosed and claimed invention overcomes obstacles encountered in prior art methods, which reported low yields of specific monoclonal responses (Eastman et al., 1996; Dadaglio et al., 1997; and Andersen et al., 1996). The antibodies generated by the method of the presently disclosed and claimed invention are also clearly distinct from those reported from phage libraries. As an example, a phage-derived Fab which recognized hTERT-HLA-A2 complex would stain hTERT-peptide pulsed HLA-A2 positive cells (Lev et al., 2002), but would not stain tumor cells (Parkhurst et al., 2004), indicating that this prior art antibody had either low specificity, or low affinity, or both. Such an antibody would not be useful in applications described herein for the presently disclosed and claimed invention, such as but not limited to, epitope validation in vaccine development and other clinical applications.

Thus, in accordance with the present invention, there has been provided a method of producing antibodies that recognize peptides associated with a tumorigenic or disease state, wherein the antibodies will mimic the specificity of a T cell receptor, that fully satisfies the objectives and advantages set forth hereinabove. in conjunction language set forth

### REFERENCES

The following references, to the extent that they provide exemplary procedural or other details supplementary to those set forth herein, are:
Altman, J.D. et al. Phenotypic analysis of antigen-specific T lymphocytes. Science, 274:94-96 (1996).
Andersen, P.S. et al. A recombinant antibody with the antigen-specific, major histocompatibility complex-restricted specificity of T cells. Proc Natl Acad Sci U S A, 93:1820-1824 (1996).
Apostolopoulos, V., Haurum, J.S. & McKenzie, I.F. MUC1 peptide epitopes associated with five different H-2 class I molecules. Eur J Immunol, 27:2579-2587 (1997).
Barfoed, A. M., et al. Cytotoxic T-lymphocyte clones, established by stimulation with the HLA-A2 binding p5365-73 wild type peptide loaded on dendritic cells in vitro, specifically recognize and lyse HLA-A2 tumour cells overexpressing the p53 protein. Scand J Immunol, 51(2): 128-33 (2000).
Biddison, W.E., et al. Tax and M1 peptide/HLA-A2-specific Fabs and T cell receptors recognize nonidentical structural features on peptide/HLA-A2 complexes. J Immunol, 171:3064-3074 (2003).
Bohm, C.M. et al. Identification of HLA-A2-restricted epitopes of the tumor associated antigen MUC2 recognized by human cytotoxic T cells. Int J Cancer, 75:688-693 (1998).
Brooks, S.C., Locke, E.R. & Soule, H.D. Estrogen receptor in a human cell line (MCF-7) from breast carcinoma. J Biol Chem, 248:6251-6253 (1973).
Brossart, P. et al. Identification of HLA-A2-restricted T-cell epitopes derived from the MUC1 tumor antigen for broadly applicable vaccine therapies. Blood, 93:4309-4317 (1999).
Bushell, M., et al. Cleavage of polypeptide chain initiation factor eIF4GI during apoptosis in lymphoma cells: characterisation of an internal fragment generated by caspase-3-mediated cleavage." Cell Death Differ, 7(7): 628-36 (2003).
Chames, P. et al. TCR-like human antibodies expressed on human CTLs mediate antibody affinity-dependent cytolytic activity. J Immunol, 169:1110-1118 (2002).
Chikamatsu, K. et al. Generation of anti-p53 cytotoxic T lymphocytes from human peripheral blood using autologous dendritic cells. Clin Cancer Res, 5:1281-1288 (1999).
Clemens, M. J. Targets and mechanisms for the regulation of translation in malignant transformation." Oncogene, 23(18): 3180-8 (2004).
Clinchy, B. et al. The growth and metastasis of human, HER-2/neu-overexpressing tumor cell lines in male SCID mice. Breast Cancer Res Treat, 61:217-228 (2000).
Cohen, C.J., Denkberg, G., Lev, A., Epel, M. & Reiter, Y. Recombinant antibodies with MHC-restricted, peptide-specific, T-cell receptor-like specificity: new tools to study antigen presentation and TCR-peptide-MHC interactions. J Mol Recognit, 16:324-332 (2003).
Coussens, L. et al. Tyrosine kinase receptor with extensive homology to EGF receptor shares chromosomal location with neu oncogene. Science, 230:1132-1139 (1985).
Cox et al. identification of a peptide recognized by five melanoma-specific human cytotoxic T cell lines. Science, 264:716-719 (1994).
Dadaglio, G., et al., Characterization and quantitation of peptide-MHC complexes produced from hen egg lysozyme using a monoclonal antibody. Immunity, 6(6):727-38 (1997).
DeGroot et al. Rapid determination of HLA B*07 ligands from the West Nile virus NY99 genome. Emerging Infectious Diseases, 7:4 (2001).
DeLeo, A.B. p53-based immunotherapy of cancer. Crit Rev Immunol, 18:29-35 (1998).
Denkberg, G., E. Klechevsky, and Y. Reiter, Modification of a tumor-derived peptide at an H LA-A2 anchor residue can alter the conformation of the MHC-peptide complex: probing with TCR-like recombinant antibodies. J Immunol, 169(8):4399-407 (2002).
Denkberg, G. et al. Direct visualization of distinct T cell epitopes derived from a melanoma tumor-associated antigen by using human recombinant antibodies with MHC- restricted T cell receptor-like specificity. Proc Natl Acad Sci U S A, 99:9421-9426 (2002).
Denkberg, G. et al. Selective targeting of melanoma and APCs using a recombinant antibody with TCR-like specificity directed toward a melanoma differentiationo antigen. J. Immunol. 171:2197-2207 (2003).
Disis, M.L. etal. Existent T-cell and antibody immunity to HER-2/neu protein in patients with breast cancer. Cancer Res, 54:16-20 (1994).
Diwan, M. & Park, T.G. Stabilization of recombinant interferon-alpha by pegylation for encapsulation in PLGA microspheres. Int J Pharm, 252:111-122 (2003).
Dols, A. et al. Identification of tumor-specific antibodies in patients with breast cancer vaccinated with gene-modified allogeneic tumor cells. J Immunother, 26:163-170 (2003).
Eastman, S., et al., A study of complexes of class II invariant chain peptide: major histocompatibility complex class II molecules using a new complex-specific monoclonal antibody. Eur J Immunol, 26(2):385-93 (1996).
Fingeroth, J. D. et al. Epstein-Barr virus receptor of human B lymphocytes is the C3d receptor CR2. Proc Natl Acad Sci U S A, 81:4510-4514 (1984).
Fisk, B., Blevins, T.L., Wharton, J.T. & loannides, C.G. Identification of an immunodominant peptide of HER-2/neu protooncogene recognized by ovarian tumor-specific cytotoxic T lymphocyte lines. J Exp Med, 181:2109-2117 (1995).
Glennie, M.J. & van de Winkel, J.G. Renaissance of cancer therapeutic antibodies. Drug Discov Today, 8:503-510 (2003).
Gnjatic, S. et al. Accumulation of the p53 protein allows recognition by human CTL of a wild-type p53 epitope presented by breast carcinomas and melanomas. J Immunol, 160:328-333 (1998).
Hickman, H.D. et al. C-terminal epitope tagging facilitates comparative ligand mapping from MHC class I positive cells. Hum Immunol, 61:1339-1346 (2000).
Hickman, H.D. et al. Cutting Edge: Class I presentation of host peptides following HIV infection. J Immunol, 171:22-26 (2003).
Hickman, H.D. et al. Toward a definition of self: proteomic evaluation of the class I peptide repertoire. J Immunol, 172:2944-2952 (2004).
Hoffmann, T. K., H. Bier, et al. p53 as an immunotherapeutic target in head and neck cancer. Adv Otorhinolaryngol, 62:151-60 (2005).
Irsch, J. et al. Isolation and characterization of allergen-binding cells from normal and allergic donors. Immunotechnology, 1:115-125 (1995).
Jager, E., Jager, D. & Knuth, A. Antigen-specific immunotherapy and cancer vaccines. Int J Cancer, 106:817-820 (2003).
Joseph, A.M., Babcock, G.J. & Thorley-Lawson, D.A. Cells expressing the Epstein-Barr virus growth program are present in and restricted to the naive B-cell subset of healthy tonsils. J Virol, 74:9964-9971 (2000).
Keams-Jonker, M. et al. EBV binds to lymphocytes of transgenic mice that express the human CR2 gene. Virus Res, 50:85-94 (1997).
Knutson, K.L, Schiffman, K., Cheever, M.A. & Disis, M.L. Immunization of cancer patients with a HER-2/neu, HLA-A2 peptide, p369-377, results in short-lived peptide-specific immunity. Clin Cancer Res, 8:1014-1018 (2002).
Kodituwakku, A.P., Jessup, C., Zola, H. & Roberton, D.M. Isolation of antigen-specific B cells. Immunol Cell Biol, 81:163-170 (2003).
Kohler, G. & Milstein, C. Continuous cultures of fused cells secreting antibody of predefined specificity. Nature, 256:495-497 (1975).
Lev, A. et al. Isolation and characterization of human recombinant antibodies endowed with the antigen-specific, major histocompatibility complex-restricted specificity of T cells directed toward the widely expressed tumor T-cell epitopes of the telomerase catalytic subunit. Cancer Res, 62:3184-3194 (2002).
Liu, A. H., Creadon, G. & Wysocki, L.J. Sequencing heavy-and light-chain variable genes of single B-hybridoma cells by total enzymatic amplification. Proc Natl Acad Sci U S A, 89:7610-7614 (1992).
Luescher, I.F. et al. HLA photoaffinity labeling reveals overlapping binding of homologous melanoma-associated gene peptides by HLA-A1, HLA-A29, and HLA-B44. J Biol Chem, 271:12463-12471 (1996).
Maeurer, M.J., Martin, D., Elder, E., Storkus, W.J. & Lotze, M.T. Detection of naturally processed and HLA-A1-presented melanoma T-cell epitopes defined by CD8(+) T-cells' release of granulocyte-macrophage colony-stimulating factor but not by cytolysis. Clin Cancer Res, 2:87-95 (1996).
Menendez, J. A., R. Lupu, et al. Inhibition of tumor-associated fatty acid synthase hyperactivity induces synergistic chemosensitization of HER -2/ neu-overexpressing human breast cancer cells to docetaxel (taxotere). Breast Cancer Res Treat, 84(2): 183-95 (2004a).
Menendez, J. A., S. Ropero, et al. Omega-6 polyunsaturated fatty acid gamma-linolenic acid (18:3n-6) enhances docetaxel (Taxotere) cytotoxicity in human breast carcinoma cells: Relationship to lipid peroxidation and HER-2/neu expression. Oncol Rep, 11(6): 1241-52 (2004b).
Messaoudi, L, J. LeMaoult, and J. Nikolic-Zugic, The mode of ligand recognition by two peptide:MHC class I-specific monoclonal antibodies. J Immunol, 163(6):3286-94 (1999).
Miki, T., Yano, S., Hanibuchi, M. & Sone, S. Bone metastasis model with multiorgan dissemination of human small-cell lung cancer (SBC-5) cells in natural killer cell-depleted SCID mice. Oncol Res, 12:209-217(2000).
Morley, S. J., et al. Differential requirements for caspase-8 activity in the mechanism of phosphorylation of eIF2α, cleavage of eIF4GI and signaling events associated with the inhibition of protein synthesis in apoptotic Jurkat T cells. FEBS Lett, 477(3): 229-36 (2000).
Morley, S. J., et al. Initiation factor modifications in the preapoptotic phase. Cell Death Differ, 12(6): 571-84 (2005).
Murphy, D.B., et al., Monoclonal antibody detection of a major self peptide. MHC class II complex. J Immunol, 148(11): 3483-91 (1992).
Nahta, R., Hung, M.C. & Esteva, F.J. The HER-2-targeting antibodies trastuzumab and pertuzumab synergistically inhibit the survival of breast cancer cells. Cancer Res, 64:2343-2346 (2004).
Nikitina, E. Y., et al. Dendritic cells transduced with full-length wild-type p53 generate antitumor cytotoxic T lymphocytes from peripheral blood of cancer patients. Clin Cancer Res, 7(1): 127-35 (2001).
Oshiba, A., Renz, H., Yata, J. & Gelfand, E.W. Isolation and characterization of human antigen-specific B lymphocytes. Clin Immunol Immunopathol, 72:342-349 (1994).
Parkhurst, M.R., et al., Immunization of patients with the hTERT:540-548 peptide induces peptide-reactive T lymphocytes that do not recognize tumors endogenously expressing telomerase. Clin Cancer Res, 10(14): 4688-98 (2004).
Polakova, K., et al., Antibodies directed against the MHC-I molecule H-2Dd complexed with an antigenic peptide: similarities to a T cell receptor with the same specificity. J Immunol, 165(10): 5703-12 (2000).
Porgador, A., Yewdell, J.W., Deng, Y., Bennink, J.R. & Germain, R.N. Localization, quantitation, and in situ detection of specific peptide-MHC class I complexes using a monoclonal antibody. Immunity, 6:715-726 (1997).
Reisbach, G., Gebhart, E. & Cailleau, R. Sister chromatid exchanges and proliferation kinetics of human metastatic breast tumor cells lines. Anticancer Res, 2:257-260 (1982).
Rognan, D., et al., Modeling the interactions of a peptide-major histocompatibility class I ligand with its receptors. I. Recognition by two alpha beta T cell receptors. J Comput Aided Mol Des, 14(1): 53-69 (2000).
Rongcun, Y. et al. Identification of new HER2/neu-derived peptide epitopes that can elicit specific CTL against autologous and allogeneic carcinomas and melanomas. J Immunol, 163:1037-1044 (1999).
Schlichtholz, B. et al. The immune response to p53 in breast cancer patients is directed against immunodominant epitopes unrelated to the mutational hot spot. Cancer Res, 52:6380-6384 (1992).
Seliger, B., et al. HER-2/neu is expressed in human renal cell carcinoma at heterogeneous levels independently of tumor grading and staging and can be recognized by HLA-A2.1-restricted cytotoxic T lymphocytes. Int J Cancer, 87(3): 349-59 (2000).
Shastri, N., Schwab, S. & Serwold, T. Producing nature's gene-chips: the generation of peptides for display by MHC class I molecules. Annu Rev Immunol, 20:463-493 (2002).
Slamon, D.J. et al. Use of chemotherapy plus a monoclonal antibody against HER2 for metastatic breast cancer that overexpresses HER2. N Engl J Med, 344:783-792 (2001).
Steenbakkers, P.G., et al., Localization of MHC class II/human cartilage glycoprotein-39 complexes in synovia of rheumatoid arthritis patients using complex-specific monoclonal antibodies. J Immunol, 170(11): 5719-27 (2003).
Sun, Y. et al. [Result of phase II clinical trial of herceptin in advanced Chinese breast cancer patients]. Zhonghua Zhong Liu Za Zhi, 25:581-583 (2003).
Theobald, M., et al. Targeting p53 as a general tumor antigen. Proc Natl Acad Sci U S A, 92(26): 11993-7 (1995).
Theobald, M., et al. The sequence alteration associated with a mutational hotspot in p53 protects cells from lysis by cytotoxic T lymphocytes specific for a flanking peptide epitope. J Exp Med, 188(6):1017-28 (1998).
Townsend, S. E., Goodnow, C.C. & Cornall, R.J. Single epitope multiple staining to detect ultralow frequency B cells. J Immunol Methods, 249:137-146 (2001).
van der Burg, S.H., Visseren, M.J., Brandt, R.M., Kast, W.M. & Melief, C.J. Immunogenicity of peptides bound to MHC class I molecules depends on the MHC-peptide complex stability. J Immunol,156: 3308-3314 (1996).
Wei, M. L. and P. Cresswell. HLA-A2 molecules in an antigen-processing mutant cell contain signal sequence-derived peptides. Nature, 356(6368): 443-6 (1992).
Welch, W.R. et al. Antigenic heterogeneity in human ovarian cancer. Gynecol Oncol, 38:12-16 (1990).
Wilkinson, K.A., Hudecz, F., Vordermeier, H.M., Ivanyi, J. & Wilkinson, R.J. Enhancement of the T cell response to a mycobacterial peptide by conjugation to synthetic branched polypeptide. Eur J Immunol, 29: 2788-2796 (1999).
Yu, Z., et al. The use of transgenic mice to generate high affinity p53 specific cytolytic T cells. J Surg Res, 69(2): 337-43 (1997).
Yu, Y.Y., Netuschil, N., Lybarger, L., Connolly, J.M. & Hansen, T.H. Cutting edge: single-chain trimers of MHC class I molecules form stable structures that potently stimulate antigen-specific T cells and B cells. J Immunol, 168: 3145-3149 (2002).

## Claims

1. A method of producing a T-cell receptor mimic, wherein the T cell receptor mimic comprises an antibody reactive against a specific perptide/class I MHC complex, comprising the steps of:
identifying a peptide of interest, wherein the peptide of interest is capable of being presented by a Class I MHC molecule;
forming an immunogen comprising a tetramer of recombinantly expressed peptide/Class I MHC complexes, wherein the peptide of each of the peptide/Class I MHC complexes is the peptide of interest, and wherein the peptide/Class I MHC complexes are tetramerized through the use of polymerized streptavidin, and wherein each of the recombinantly expressed peptide/Class I MHC complexes is produced by one of:
a) recombinantly expressing the peptide/Class I MHC complex in the form of a single chain trimer; and
b) recombinantly expressing the Class I MHC heavy chain and the Class I MHC light chain separately in *E. coli,* and then refolding the Class I MHC heavy and light chains with peptide *in vitro*;
administering an effective amount of the immunogen to a non-human host for eliciting an immune response, wherein the immunogen retains a three-dimensional form thereof for a period of time sufficient to elicit an immune response against the three-dimensional presentation of the peptide in the binding groove of the Class I MHC molecule;
preabsorbing serum collected from the host to remove antibodies that are not peptide specific;
assaying the preabsorbed serum to determine if desired antibodies that recognize a three-dimensional presentation of the peptide in the binding groove of the Class I MHC molecule is being produced, wherein the desired antibodies can differentiate the peptide/Class I MHC complex from the Class I MHC molecule alone, the peptide alone, and a complex of Class I MHC and irrelevant peptide; and
isolating the desired antibodies.

2. The method of claim 1 wherein, in the step of forming an immunogen comprising a tetramer of recombinantly expressed peptide/Class I MHC complexes, a biotinylation signal peptide tail is attached to each of the four peptide/Class I MAC complexes for binding to one of the four binding sites on the polymerized streptavidin.

3. The method of claim 1 wherein the non-human host is selected from the group consisting of rabbits, mice and rats.

4. The method of claim 1 wherein the step of isolating the desired antibodies is further defined as isolating at least one of B cells expressing surface immunoglobulin, B memory cells, hybridomacells and plasma cells producing the desired antibodies.

5. The method of claim 4, wherein the step of isolating B memory cells is further defined as sorting the B memory cells using at least one of FACS sorting, beads coated with peptide/MHC complex, magnetic beads, and intracellular staining.

6. The method of claim 4, further comprising the step of differentiating and expanding the B memory cells into plasma cells.

7. The method of claim 1 further comprising the step of assaying the isolated desired antibodies to confirm their specificity and to determine if the isolated desired antibodies cross-react with other MHC molecules.

8. The method of claim 1 wherein the peptide of interest comprises at least one of SEQ ID NOS:1-3.

9. The method of claim 1 wherein the T cell receptor mimic produced by the method has a binding affinity of about 10 nanomolar or greater.

## Patentansprüche

1. Verfahren zur Herstellung eines T-Zell-Rezeptor-Mimetikums, wobei das T-Zell-Rezeptor-Mimetikum einen Antikörper umfasst, der gegen einen spezifischen Peptid/ MHC-Klasse-I-Komplex reaktiv ist, wobei das Verfahren die folgenden Schritte umfasst:
Identifizieren eines Peptids von Interesse, wobei das Peptid von Interesse dazu in der Lage ist, von einem MHC-Klasse-I-Molekül präsentiert zu werden;
Bilden eines Immunogens, das ein Tetramer von rekombinant exprimierten Peptid/MHC-Klasse-I-Komplexen umfasst, wobei das Peptid jedes der Peptid/MHC-Klasse-I-Komplexe das Peptid von Interesse ist und wobei die Peptid/MHC-Klasse-I-Komplexe durch die Verwendung von polymerisiertem Streptavidin tetramerisiert werden und wobei jeder der rekombinant exprimierten Peptid/MHC-Klasse-I-Komplexe durch eines der folgenden hergestellt wird:
a) rekombinantes Exprimieren des Peptid/MHC-Klasse-I-Komplexes in der Form eines einkettigen Trimers und
b) separates rekombinantes Exprimieren der schweren MHC-Klasse-I-Kette und der leichten MHC-Klasse-I-Kette in *E. coli* und dann Rückfalten der leichten und der schweren MHC-Klasse-I-Kette mit P*e*ptid *in vitro*;
Verabreichen einer wirksamen Menge des Immunogens an einen nichtmenschlichen Wirt zum Auslösen einer Immunantwort, wobei das Immunogen eine dreidimensionale Form davon für einen Zeitraum beibehält, der dazu ausreicht, eine Immunantwort gegen die dreidimensionale Präsentation des Peptids in der Bindungsfurche des MHC-Klasse-I-Moleküls auszulösen;
Vorabsorbieren von Serum, das vom Wirt gesammelt wurde, um Antikörper zu entfernen, die nicht peptidspezifisch sind;
Testen des vorabsorbierten Serums, um zu bestimmen, ob gewünschte Antikörper, die eine dreidimensionale Präsentation des Peptids in der Bindungsfurche des MHC-Klasse-I-Moleküls erkennen, produziert werden, wobei die gewünschten Antikörper den Peptid/MHC-Klasse-I-Komplex von dem MHC-Klasse-I-Molekül allein, dem Peptid allein und einem Komplex von MHC-Klasse-I und irrelevantem Peptid unterscheiden können; und
Isolieren der gewünschten Antikörper.

2. Verfahren nach Anspruch 1, wobei im Schritt des Bildens eines Immunogens, das ein Tetramer von rekombinant exprimierten Peptid/MHC-Klasse-I-Komplexen umfasst, ein Biotinylierungssignal-Peptidschwanz an jeden der vier Peptid/MHC-Klasse-I-Komplexe zum Binden an eine der vier Bindungsstellen an dem polymerisierten Streptavidin angelagert ist.

3. Verfahren nach Anspruch 1, wobei der nichtmenschliche Wirt aus der Gruppe bestehend aus Kaninchen, Mäusen und Ratten ausgewählt ist.

4. Verfahren nach Anspruch 1, wobei der Schritt des Isolierens der gewünschten Antikörper weiter als Isolieren von B-Zellen exprimierendem Oberflächen-Immunglobulin, B-Gedächtniszellen, Hybridomzellen und/oder Plasmazellen, die die gewünschten Antikörper produzieren, definiert ist.

5. Verfahren nach Anspruch 4, wobei der Schritt des Isolierens von B-Gedächtniszellen weiter als Sortieren der B-Speicherzellen unter Verwendung von FACS-Sortierung, mit Peptid/MHC-Komplex beschichteten Kügelchen, magnetischen Kügelchen und/oder intrazellulärer Färbung definiert ist.

6. Verfahren nach Anspruch 4, das weiterhin den Schritt des Differenzierens und Expandierens der B-Gedächtniszellen in Plasmazellen umfasst.

7. Verfahren nach Anspruch 1, das weiterhin den Schritt des Testens der isolierten gewünschten Antikörper umfasst, um deren Spezifität zu bestätigen und zu bestimmen, ob die isolierten gewünschten Antikörper mit anderen MHC-Molekülen kreuzreagieren.

8. Verfahren nach Anspruch 1, wobei das Peptid von Interesse mindestens eine von SEQ ID Nr. 1 - 3 umfasst.

9. Verfahren nach Anspruch 1, wobei das durch das Verfahren hergestellte T-Zell-Rezeptor-Mimetikum eine Bindungsaffinität von etwa 10 Nanomol oder höher hat.

## Revendications

1. Procédé de production d'un imitateur de récepteur de lymphocytes T, dans lequel l'imitateur de récepteur de lymphocytes T comprend un anticorps réactif contre un complexe peptide/CMH de classe I spécifique, comprenant les étapes consistant à :
identifier un peptide pertinent, dans lequel le peptide pertinent est capable d'être présenté par une molécule du CMH de classe I ;
former un immunogène comprenant un tétramère de complexes peptide/CMH de classe I exprimés de manière recombinée, dans lequel le peptide de chacun des complexes peptide/CMH de classe I est le peptide pertinent, et dans lequel les complexes peptide/CMH de classe I sont tétramérisés par l'utilisation de streptavidine polymérisée, et dans lequel chacun des complexes peptide/CMH de classe I exprimés de manière recombinée est produit par une expression parmi :
a) l'expression recombinée du complexe peptide/CMH de classe I sous la forme d'un trimère à chaîne unique ; et
b) l'expression recombinée de la chaîne lourde du CMH de classe I et de la chaîne légère du CMH de classe I séparément dans E. coli, puis le repliage des chaînes lourde et légère du CMH de classe I avec le peptide in vitro ;
administrer une quantité efficace de l'immunogène à un hôte non humain pour provoquer une réponse immunitaire, dans lequel l'immunogène converse une forme tridimensionnelle de celui-ci pendant une période de temps suffisante pour provoquer une réponse immunitaire contre la présentation tridimensionnelle du peptide dans la rainure de fixation de la molécule du CMH de classe I ;
préabsorber du sérum recueilli de l'hôte pour éliminer les anticorps qui ne sont pas spécifiques au peptide ;
doser le sérum préabsorbé pour déterminer si des anticorps souhaités qui reconnaissent une présentation tridimensionnelle du peptide dans la rainure de fixation de la molécule du CMH de classe I sont produits, dans lequel les anticorps souhaités peuvent différencier le complexe peptide/CMH de classe I de la molécule du CMH de classe I seule, du peptide seul et d'un complexe du CMH de classe I et d'un peptide non pertinent ; et
isoler les anticorps souhaités.

2. Procédé selon la revendication 1, dans lequel, dans l'étape consistant à former un immunogène comprenant un tétramère de complexes peptide/CMH de classe I exprimés de manière recombinée, une queue de peptide de signal de biotinylation est reliée à chacun des quatre complexes peptide/CMH de classe I pour la fixation à un des quatre sites de fixation sur la streptavidine polymérisée.

3. Procédé selon la revendication 1, dans lequel l'hôte non humain est sélectionné parmi le groupe constitué de lapins, souris et rats.

4. Procédé selon la revendication 1, dans lequel l'étape consistant à isoler les anticorps souhaités est en outre définie comme l'isolement d'au moins un élément parmi des lymphocytes B exprimant de l'immunoglobuline de surface, des lymphocytes B mémoires, des cellules d'hybridome et des cellules de plasma produisant les anticorps souhaités.

5. Procédé selon la revendication 4, dans lequel l'étape consistant à isoler des lymphocytes B mémoires est en outre définie comme le tri des lymphocytes B mémoires en utilisant au moins un procédé parmi le tri par trieur de cellules à fluorescence, des billes enduites de complexe peptide/CMH, des billes magnétiques et une coloration intracellulaire.

6. Procédé selon la revendication 4, comprenant en outre l'étape consistant à différencier et réaliser l'expansion des lymphocytes B mémoires dans des cellules de plasma.

7. Procédé selon la revendication 1, comprenant en outre l'étape consistant à doser les anticorps souhaités isolés pour confirmer leur spécificité et pour déterminer si les anticorps souhaités isolés réagissent de manière croisée avec d'autres molécules du CMH.

8. Procédé selon la revendication 1, dans lequel le peptide pertinent comprend au moins une de SEQ ID N° : 1 à 3.

9. Procédé selon la revendication 1, dans lequel l'imitateur de récepteur de lymphocytes T produit par le procédé a une affinité de fixation d'environ 10 nanomoles ou supérieure.


### SEQUENCE LISTING

<110> Weidanz, Jon Wittman, vaughan
<120> ANTIBODIES AS T CELL RECEPTOR MIMICS, METHODS OF PRODUCTION AND USES THEREOF
<130> 6703.007wo
<150> 60/374,857
   <151> 2004-05-27
<150> 60/640,020
   <151> 2004-12-28
<150> 60/646,338
   <151> 2005-01-24
<150> 60/673,296
   <151> 2005-04-20
<160> 3
<170> PatentIn version 3.3
<210> 1
   <211> 9
   <212> PRT
   <213> Homo sapiens
<400> 1
   Leu Leu Gly Arg Asn Ser Phe Glu Val
   1               5
<210> 2
   <211> 9
   <212> PRT
   <213> Homo sapiens
<400> 2
   Val Leu Met Thr Glu Asp Ile Lys Leu
   1               5
<210> 3
   <211> 9
   <212> PRT
   <213> Homo sapiens
<400> 3
   Lys Ile Phe Gly Ser Leu Ala Phe Leu
   1               5